# EUROPEAN PATENT APPLICATION

(11) **EP 4 292 611 A1**
(43) Date of publication of application: **20.12.2023**
(21) Application number: 22752241.4
(22) Date of filing: 08.02.2022
(51) Int. Cl.: A61K 39/395, A61K 35/00, G01N 33/574, C07K 16/28, C12N 15/13

(54) **ANTI-CD112R ANTIBODY AND USE THEREOF**

(30) Priority: 09.02.2021 CN 202110178859
(71) Applicant: Shanghai Junshi Biosciences Co., Ltd., Pilot Free Trade Zone Shanghai 201210 (CN); Suzhou Junmeng Biosciences Co., Ltd., Jiangsu 215002 (CN)
(72) Inventor: LIU, Dandan, Jiangsu 215002 (CN); ZHANG, Jing, Jiangsu 215002 (CN); ZHOU, Yuehua, Jiangsu 215002 (CN); YAO, Jian, Jiangsu 215002 (CN); ZHAO, Qiang, Jiangsu 215002 (CN); LIU, Hui, Jiangsu 215002 (CN); FENG, Hui, Jiangsu 215002 (CN)
(74) Representative: V.O.
(86) International application number: PCT/CN2022/075502
(87) International publication number: WO 2022/171080

(57) **Abstract**

An antibody specifically binding to CD112R or an antigen binding fragment thereof, a composition comprising same, a nucleic acid molecule encoding the antibody or the antigen binding fragment thereof, a vector and a host cell for expressing the antibody or the antigen binding fragment thereof, a treatment and diagnostic method using the antibody or the antigen binding fragment thereof, and use of the antibody or the antigen binding fragment thereof.

## Description

### TECHNICAL FIELD

The present invention provides an antibody or an antigen-binding fragment thereof specifically binding to CD112R and a composition comprising same. Also provided are a nucleic acid molecule encoding the antibody or the antigen-binding fragment thereof described herein, a vector and a host cell used for expressing the antibody or the antigen-binding fragment thereof described herein, and therapeutic and diagnostic methods and use of the antibody or the antigen-binding fragment thereof described herein.

### BACKGROUND

The immune system is a highly complex system composed of many cell types, including but not limited to T cells, B cells, natural killer cells, antigen-presenting cells, dendritic cells, monocytes, and macrophages. These cells possess complex and subtle systems to control their interactions and responses. Cells utilize activation and inhibition mechanisms and feedback loops to maintain control of the response and do not allow the negative consequences of an uncontrolled immune response (e.g., autoimmune disease).

CD112R (PVRIG), also known as poliovirus receptor-associated immunoglobulin domain-containing protein, Q6DKI7, or C7orf15, is a transmembrane domain protein of 326 amino acids, comprising a signal peptide (amino acids 1 to 40), an extracellular domain (amino acids 41 to 171), a transmembrane domain (amino acids 172 to 190), and a cytoplasmic domain (amino acids 191 to 326). CD112R binds to poliovirus receptor-related 2 (PVLR2, also known as nectin-2, CD112, herpesvirus entry mediator B (HVEB), or human plasma membrane glycoprotein), the binding partner of CD112R.

The administration of anti-CD112R immunotherapy offers the opportunity to increase, enhance, and maintain the immune response. CD112R is an inhibitory receptor mainly expressed by T cells and NK cells and competes with the activation receptor CD226 for binding to CD112. CD112 has a higher affinity for the interaction with CD112R than that with CD226, and can thus effectively regulate CD226-mediated cell activation. Anti-CD112R antibodies that block the interaction of CD112R with CD112 directly limit downstream inhibitory signaling of CD112R and also promote immune cell activation to a greater extent by increasing the interaction of CD226 with CD112.

Treatment with anti-CD112R antibodies provides an opportunity to down-regulate inhibitory signals that may occur when CD112R-expressing immune cells bind to CD112 on tumor cells and/or myeloid cells within the tumor microenvironment, and may enhance, increase, and maintain the anti-tumor immune response.

Currently, the only anti-CD112R monoclonal antibody therapy available is SRF813 (Surface Oncology), which is in the preclinical stage, and more novel anti-CD112R antibodies are still needed.

### SUMMARY

The present invention provides an anti-CD112R antibody or an antigen-binding fragment thereof, which has the advantage of having high affinity, high specificity, etc., for human CD 112R. The anti-CD112R antibody or the antigen-binding fragment thereof provided herein can be used as an independent therapy or in combination with other therapies and/or other anti-cancer pharmaceutical agents to treat, for example, cancers.

In one aspect, the present invention provides an anti-CD112R antibody or an antigen-binding fragment thereof, comprising a heavy chain variable region and/or a light chain variable region, wherein:
the heavy chain variable region comprises:
   (I) HCDR1, HCDR2, and HCDR3 having amino acid sequences set forth in SEQ ID NO: 1, SEQ ID NO: 2, and SEQ ID NO: 3, respectively; or HCDR1, HCDR2, and HCDR3 having 1, 2, or 3 amino acid alterations as compared with the amino acid sequences set forth in SEQ ID NO: 1, SEQ ID NO: 2, and SEQ ID NO: 3, respectively; or
   (II) HCDR1, HCDR2, and HCDR3 having amino acid sequences set forth in SEQ ID NO: 7, SEQ ID NO: 8, and SEQ ID NO: 9, respectively; or HCDR1, HCDR2, and HCDR3 having 1, 2, or 3 amino acid alterations as compared with the amino acid sequences set forth in SEQ ID NO: 7, SEQ ID NO: 8, and SEQ ID NO: 9, respectively; or
   (III) HCDR1, HCDR2, and HCDR3 having amino acid sequences set forth in SEQ ID NO: 13, SEQ ID NO: 14, and SEQ ID NO: 15, respectively; or HCDR1, HCDR2, and HCDR3 having 1, 2, or 3 amino acid alterations as compared with the amino acid sequences set forth in SEQ ID NO: 13, SEQ ID NO: 14, and SEQ ID NO: 15, respectively; or
   (IV) HCDR1, HCDR2, and HCDR3 having amino acid sequences set forth in SEQ ID NO: 16, SEQ ID NO: 17, and SEQ ID NO: 18, respectively; or HCDR1, HCDR2, and HCDR3 having 1, 2, or 3 amino acid alterations as compared with the amino acid sequences set forth in SEQ ID NO: 16, SEQ ID NO: 17, and SEQ ID NO: 18, respectively; or
   (V) HCDR1, HCDR2, and HCDR3 having amino acid sequences set forth in SEQ ID NO: 22, SEQ ID NO: 23, and SEQ ID NO: 24, respectively; or HCDR1, HCDR2, and HCDR3 having 1, 2, or 3 amino acid alterations as compared with the amino acid sequences set forth in SEQ ID NO: 22, SEQ ID NO: 23, and SEQ ID NO: 24, respectively; or
   (VI) HCDR1, HCDR2, and HCDR3 having amino acid sequences set forth in SEQ ID NO: 28, SEQ ID NO: 29, and SEQ ID NO: 30, respectively; or HCDR1, HCDR2, and HCDR3 having 1, 2, or 3 amino acid alterations as compared with the amino acid sequences set forth in SEQ ID NO: 28, SEQ ID NO: 29, and SEQ ID NO: 30, respectively; or
   (VII) HCDR1, HCDR2, and HCDR3 having amino acid sequences set forth in SEQ ID NO: 34, SEQ ID NO: 35, and SEQ ID NO: 36, respectively; or HCDR1, HCDR2, and HCDR3 having 1, 2, or 3 amino acid alterations as compared with the amino acid sequences set forth in SEQ ID NO: 34, SEQ ID NO: 35, and SEQ ID NO: 36, respectively; or
   (VIII) HCDR1, HCDR2, and HCDR3 having amino acid sequences set forth in SEQ ID NO: 40, SEQ ID NO: 41, and SEQ ID NO: 42, respectively; or HCDR1, HCDR2, and HCDR3 having 1, 2, or 3 amino acid alterations as compared with the amino acid sequences set forth in SEQ ID NO: 40, SEQ ID NO: 41, and SEQ ID NO: 42, respectively; or
   (IX) HCDR1, HCDR2, and HCDR3 having amino acid sequences set forth in SEQ ID NO: 49, SEQ ID NO: 50, and SEQ ID NO: 51, respectively; or HCDR1, HCDR2, and HCDR3 having 1, 2, or 3 amino acid alterations as compared with the amino acid sequences set forth in SEQ ID NO: 49, SEQ ID NO: 50, and SEQ ID NO: 51, respectively; or
   (X) HCDR1, HCDR2, and HCDR3 having amino acid sequences set forth in SEQ ID NO: 55, SEQ ID NO: 56, and SEQ ID NO: 57, respectively; or HCDR1, HCDR2, and HCDR3 having 1, 2, or 3 amino acid alterations as compared with the amino acid sequences set forth in SEQ ID NO: 55, SEQ ID NO: 56, and SEQ ID NO: 57, respectively; or
   (XI) HCDR1, HCDR2, and HCDR3 having amino acid sequences set forth in SEQ ID NO: 61, SEQ ID NO: 62, and SEQ ID NO: 63, respectively; or HCDR1, HCDR2, and HCDR3 having 1, 2, or 3 amino acid alterations as compared with the amino acid sequences set forth in SEQ ID NO: 61, SEQ ID NO: 62, and SEQ ID NO: 63, respectively; or
   (XII) HCDR1, HCDR2, and HCDR3 having amino acid sequences set forth in SEQ ID NO: 67, SEQ ID NO: 68, and SEQ ID NO: 69, respectively; or HCDR1, HCDR2, and HCDR3 having 1, 2, or 3 amino acid alterations as compared with the amino acid sequences set forth in SEQ ID NO: 67, SEQ ID NO: 68, and SEQ ID NO: 69, respectively; or
   (XIII) HCDR1, HCDR2, and HCDR3 having amino acid sequences set forth in SEQ ID NO: 73, SEQ ID NO: 74, and SEQ ID NO: 75, respectively; or HCDR1, HCDR2, and HCDR3 having 1, 2, or 3 amino acid alterations as compared with the amino acid sequences set forth in SEQ ID NO: 73, SEQ ID NO: 74, and SEQ ID NO: 75, respectively; or
   (XIV) HCDR1, HCDR2, and HCDR3 having amino acid sequences set forth in SEQ ID NO: 79, SEQ ID NO: 80, and SEQ ID NO: 81, respectively; or HCDR1, HCDR2, and HCDR3 having 1, 2, or 3 amino acid alterations as compared with the amino acid sequences set forth in SEQ ID NO: 79, SEQ ID NO: 80, and SEQ ID NO: 81, respectively; or
   (XV) HCDR1, HCDR2, and HCDR3 having amino acid sequences set forth in SEQ ID NO: 143, SEQ ID NO: 144, and SEQ ID NO: 145, respectively; or HCDR1, HCDR2, and HCDR3 having 1, 2, or 3 amino acid alterations as compared with the amino acid sequences set forth in SEQ ID NO: 143, SEQ ID NO: 144, and SEQ ID NO: 145, respectively;
the light chain variable region comprises:
   (I) LCDR1, LCDR2, and LCDR3 having amino acid sequences set forth in SEQ ID NO: 4, SEQ ID NO: 5, and SEQ ID NO: 6, respectively; or LCDR1, LCDR2, and LCDR3 having 1, 2, or 3 amino acid alterations as compared with the amino acid sequences set forth in SEQ ID NO: 4, SEQ ID NO: 5, and SEQ ID NO: 6, respectively; or
   (II) LCDR1, LCDR2, and LCDR3 having amino acid sequences set forth in SEQ ID NO: 10, SEQ ID NO: 11, and SEQ ID NO: 12, respectively; or LCDR1, LCDR2, and LCDR3 having 1, 2, or 3 amino acid alterations as compared with the amino acid sequences set forth in SEQ ID NO: 10, SEQ ID NO: 11, and SEQ ID NO: 12, respectively; or
   (III) LCDR1, LCDR2, and LCDR3 having amino acid sequences set forth in SEQ ID NO: 19, SEQ ID NO: 20, and SEQ ID NO: 21, respectively; or LCDR1, LCDR2, and LCDR3 having 1, 2, or 3 amino acid alterations as compared with the amino acid sequences set forth in SEQ ID NO: 19, SEQ ID NO: 20, and SEQ ID NO: 21, respectively; or
   (IV) LCDR1, LCDR2, and LCDR3 having amino acid sequences set forth in SEQ ID NO: 25, SEQ ID NO: 26, and SEQ ID NO: 27, respectively; or LCDR1, LCDR2, and LCDR3 having 1, 2, or 3 amino acid alterations as compared with the amino acid sequences set forth in SEQ ID NO: 25, SEQ ID NO: 26, and SEQ ID NO: 27, respectively; or
   (V) LCDR1, LCDR2, and LCDR3 having amino acid sequences set forth in SEQ ID NO: 31, SEQ ID NO: 32, and SEQ ID NO: 33, respectively; or LCDR1, LCDR2, and LCDR3 having 1, 2, or 3 amino acid alterations as compared with the amino acid sequences set forth in SEQ ID NO: 31, SEQ ID NO: 32, and SEQ ID NO: 33, respectively; or
   (VI) LCDR1, LCDR2, and LCDR3 having amino acid sequences set forth in SEQ ID NO: 37, SEQ ID NO: 38, and SEQ ID NO: 39, respectively; or LCDR1, LCDR2, and LCDR3 having 1, 2, or 3 amino acid alterations as compared with the amino acid sequences set forth in SEQ ID NO: 37, SEQ ID NO: 38, and SEQ ID NO: 39, respectively; or
   (VII) LCDR1, LCDR2, and LCDR3 having amino acid sequences set forth in SEQ ID NO: 43, SEQ ID NO: 44, and SEQ ID NO: 45, respectively; or LCDR1, LCDR2, and LCDR3 having 1, 2, or 3 amino acid alterations as compared with the amino acid sequences set forth in SEQ ID NO: 43, SEQ ID NO: 44, and SEQ ID NO: 45, respectively; or
   (VIII) LCDR1, LCDR2, and LCDR3 having amino acid sequences set forth in SEQ ID NO: 46, SEQ ID NO: 47, and SEQ ID NO: 48, respectively; or LCDR1, LCDR2, and LCDR3 having 1, 2, or 3 amino acid alterations as compared with the amino acid sequences set forth in SEQ ID NO: 46, SEQ ID NO: 47, and SEQ ID NO: 48, respectively; or
   (IX) LCDR1, LCDR2, and LCDR3 having amino acid sequences set forth in SEQ ID NO: 52, SEQ ID NO: 53, and SEQ ID NO: 54, respectively; or LCDR1, LCDR2, and LCDR3 having 1, 2, or 3 amino acid alterations as compared with the amino acid sequences set forth in SEQ ID NO: 52, SEQ ID NO: 53, and SEQ ID NO: 54, respectively; or
   (X) LCDR1, LCDR2, and LCDR3 having amino acid sequences set forth in SEQ ID NO: 58, SEQ ID NO: 59, and SEQ ID NO: 60, respectively; or LCDR1, LCDR2, and LCDR3 having 1, 2, or 3 amino acid alterations as compared with the amino acid sequences set forth in SEQ ID NO: 58, SEQ ID NO: 59, and SEQ ID NO: 60, respectively; or
   (XI) LCDR1, LCDR2, and LCDR3 having amino acid sequences set forth in SEQ ID NO: 64, SEQ ID NO: 65, and SEQ ID NO: 66, respectively; or LCDR1, LCDR2, and LCDR3 having 1, 2, or 3 amino acid alterations as compared with the amino acid sequences set forth in SEQ ID NO: 64, SEQ ID NO: 65, and SEQ ID NO: 66, respectively; or
   (XII) LCDR1, LCDR2, and LCDR3 having amino acid sequences set forth in SEQ ID NO: 70, SEQ ID NO: 71, and SEQ ID NO: 72, respectively; or LCDR1, LCDR2, and LCDR3 having 1, 2, or 3 amino acid alterations as compared with the amino acid sequences set forth in SEQ ID NO: 70, SEQ ID NO: 71, and SEQ ID NO: 72, respectively; or
   (XIII) LCDR1, LCDR2, and LCDR3 having amino acid sequences set forth in SEQ ID NO: 76, SEQ ID NO: 77, and SEQ ID NO: 78, respectively; or LCDR1, LCDR2, and LCDR3 having 1, 2, or 3 amino acid alterations as compared with the amino acid sequences set forth in SEQ ID NO: 76, SEQ ID NO: 77, and SEQ ID NO: 78, respectively; or
   (XIV) LCDR1, LCDR2, and LCDR3 having amino acid sequences set forth in SEQ ID NO: 82, SEQ ID NO: 83, and SEQ ID NO: 84, respectively; or LCDR1, LCDR2, and LCDR3 having 1, 2, or 3 amino acid alterations as compared with the amino acid sequences set forth in SEQ ID NO: 82, SEQ ID NO: 83, and SEQ ID NO: 84, respectively.

In some embodiments, the antibody or the antigen-binding fragment thereof described herein comprises:
(I) a heavy chain variable region, comprising HCDR1, HCDR2, and HCDR3 having amino acid sequences set forth in SEQ ID NO: 1, SEQ ID NO: 2, and SEQ ID NO: 3, respectively; and a light chain variable region, comprising LCDR1, LCDR2, and LCDR3 having amino acid sequences set forth in SEQ ID NO: 4, SEQ ID NO: 5, and SEQ ID NO: 6, respectively; or
(II) a heavy chain variable region, comprising HCDR1, HCDR2, and HCDR3 having amino acid sequences set forth in SEQ ID NO: 7, SEQ ID NO: 8, and SEQ ID NO: 9, respectively; and a light chain variable region, comprising LCDR1, LCDR2, and LCDR3 having amino acid sequences set forth in SEQ ID NO: 10, SEQ ID NO: 11, and SEQ ID NO: 12, respectively; or
(III) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 4, SEQ ID NO: 5, and SEQ ID NO: 6, respectively; or
(IV) a heavy chain variable region, comprising HCDR1, HCDR2, and HCDR3 having amino acid sequences set forth in SEQ ID NO: 16, SEQ ID NO: 17, and SEQ ID NO: 18, respectively; and a light chain variable region, comprising LCDR1, LCDR2, and LCDR3 having amino acid sequences set forth in SEQ ID NO: 19, SEQ ID NO: 20, and SEQ ID NO: 21, respectively; or
(V) a heavy chain variable region, comprising HCDR1, HCDR2, and HCDR3 having amino acid sequences set forth in SEQ ID NO: 22, SEQ ID NO: 23, and SEQ ID NO: 24, respectively; and a light chain variable region, comprising LCDR1, LCDR2, and LCDR3 having amino acid sequences set forth in SEQ ID NO: 25, SEQ ID NO: 26, and SEQ ID NO: 27, respectively; or
(VI) a heavy chain variable region, comprising HCDR1, HCDR2, and HCDR3 having amino acid sequences set forth in SEQ ID NO: 28, SEQ ID NO: 29, and SEQ ID NO: 30, respectively; and a light chain variable region, comprising LCDR1, LCDR2, and LCDR3 having amino acid sequences set forth in SEQ ID NO: 31, SEQ ID NO: 32, and SEQ ID NO: 33, respectively; or
(VII) a heavy chain variable region, comprising HCDR1, HCDR2, and HCDR3 having amino acid sequences set forth in SEQ ID NO: 34, SEQ ID NO: 35, and SEQ ID NO: 36, respectively; and a light chain variable region, comprising LCDR1, LCDR2, and LCDR3 having amino acid sequences set forth in SEQ ID NO: 37, SEQ ID NO: 38, and SEQ ID NO: 39, respectively; or
(VIII) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(IX) a heavy chain variable region, comprising HCDR1, HCDR2, and HCDR3 having amino acid sequences set forth in SEQ ID NO: 28, SEQ ID NO: 29, and SEQ ID NO: 30, respectively; and a light chain variable region, comprising LCDR1, LCDR2, and LCDR3 having amino acid sequences set forth in SEQ ID NO: 46, SEQ ID NO: 47, and SEQ ID NO: 48, respectively; or
(X) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XI) a heavy chain variable region, comprising HCDR1, HCDR2, and HCDR3 having amino acid sequences set forth in SEQ ID NO: 55, SEQ ID NO: 56, and SEQ ID NO: 57, respectively; and a light chain variable region, comprising LCDR1, LCDR2, and LCDR3 having amino acid sequences set forth in SEQ ID NO: 58, SEQ ID NO: 59, and SEQ ID NO: 60, respectively; or
(XII) a heavy chain variable region, comprising HCDR1, HCDR2, and HCDR3 having amino acid sequences set forth in SEQ ID NO: 61, SEQ ID NO: 62, and SEQ ID NO: 63, respectively; and a light chain variable region, comprising LCDR1, LCDR2, and LCDR3 having amino acid sequences set forth in SEQ ID NO: 64, SEQ ID NO: 65, and SEQ ID NO: 66, respectively; or
(XIII) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XIV) a heavy chain variable region, comprising HCDR1, HCDR2, and HCDR3 having amino acid sequences set forth in SEQ ID NO: 73, SEQ ID NO: 74, and SEQ ID NO: 75, respectively; and a light chain variable region, comprising LCDR1, LCDR2, and LCDR3 having amino acid sequences set forth in SEQ ID NO: 76, SEQ ID NO: 77, and SEQ ID NO: 78, respectively; or
(XV) a heavy chain variable region, comprising HCDR1, HCDR2, and HCDR3 having amino acid sequences set forth in SEQ ID NO: 79, SEQ ID NO: 80, and SEQ ID NO: 81, respectively; and a light chain variable region, comprising LCDR1, LCDR2, and LCDR3 having amino acid sequences set forth in SEQ ID NO: 82, SEQ ID NO: 83, and SEQ ID NO: 84, respectively; or
(XVI) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XVII) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XVIII) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XIX) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XX) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XXI) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XXII) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XXIII) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XXIV) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XXV) a heavy chain variable region, comprising HCDR1, HCDR2, and HCDR3 having amino acid sequences set forth in SEQ ID NO: 143, SEQ ID NO: 144, and SEQ ID NO: 145, respectively; and a light chain variable region, comprising LCDR1, LCDR2, and LCDR3 having amino acid sequences set forth in SEQ ID NO: 19, SEQ ID NO: 20, and SEQ ID NO: 21, respectively.

In some embodiments, the antibody described herein is a murine antibody or a chimeric antibody.

In some embodiments, the antibody or the antigen-binding fragment thereof described herein comprises a heavy chain variable region and a light chain variable region, wherein:
(I) the heavy chain variable region comprises an amino acid sequence set forth in SEQ ID NO: 85, or comprises an amino acid sequence having at least 95%, 96%, 97%, 98%, or 99% sequence identity to the amino acid sequence set forth in SEQ ID NO: 85; and the light chain variable region comprises an amino acid sequence set forth in SEQ ID NO: 86, or comprises an amino acid sequence having at least 95%, 96%, 97%, 98%, or 99% sequence identity to the amino acid sequence set forth in SEQ ID NO: 86; or
(II) the heavy chain variable region comprises an amino acid sequence set forth in SEQ ID NO: 87, or comprises an amino acid sequence having at least 95%, 96%, 97%, 98%, or 99% sequence identity to the amino acid sequence set forth in SEQ ID NO: 87; and the light chain variable region comprises an amino acid sequence set forth in SEQ ID NO: 88, or comprises an amino acid sequence having at least 95%, 96%, 97%, 98%, or 99% sequence identity to the amino acid sequence set forth in SEQ ID NO: 88; or
(III) the heavy chain variable region comprises an amino acid sequence set forth in SEQ ID NO: 89, or comprises an amino acid sequence having at least 95%, 96%, 97%, 98%, or 99% sequence identity to the amino acid sequence set forth in SEQ ID NO: 89; and the light chain variable region comprises an amino acid sequence set forth in SEQ ID NO: 90, or comprises an amino acid sequence having at least 95%, 96%, 97%, 98%, or 99% sequence identity to the amino acid sequence set forth in SEQ ID NO: 90; or
(IV) the heavy chain variable region comprises an amino acid sequence set forth in SEQ ID NO: 91, or comprises an amino acid sequence having at least 95%, 96%, 97%, 98%, or 99% sequence identity to the amino acid sequence set forth in SEQ ID NO: 91; and the light chain variable region comprises an amino acid sequence set forth in SEQ ID NO: 92, or comprises an amino acid sequence having at least 95%, 96%, 97%, 98%, or 99% sequence identity to the amino acid sequence set forth in SEQ ID NO: 92; or
(V) the heavy chain variable region comprises an amino acid sequence set forth in SEQ ID NO: 93, or comprises an amino acid sequence having at least 95%, 96%, 97%, 98%, or 99% sequence identity to the amino acid sequence set forth in SEQ ID NO: 93; and the light chain variable region comprises an amino acid sequence set forth in SEQ ID NO: 94, or comprises an amino acid sequence having at least 95%, 96%, 97%, 98%, or 99% sequence identity to the amino acid sequence set forth in SEQ ID NO: 94; or
(VI) the heavy chain variable region comprises an amino acid sequence set forth in SEQ ID NO: 95, or comprises an amino acid sequence having at least 95%, 96%, 97%, 98%, or 99% sequence identity to the amino acid sequence set forth in SEQ ID NO: 95; and the light chain variable region comprises an amino acid sequence set forth in SEQ ID NO: 96, or comprises an amino acid sequence having at least 95%, 96%, 97%, 98%, or 99% sequence identity to the amino acid sequence set forth in SEQ ID NO: 96; or
(VII) the heavy chain variable region comprises an amino acid sequence set forth in SEQ ID NO: 97, or comprises an amino acid sequence having at least 95%, 96%, 97%, 98%, or 99% sequence identity to the amino acid sequence set forth in SEQ ID NO: 97; and the light chain variable region comprises an amino acid sequence set forth in SEQ ID NO: 98, or comprises an amino acid sequence having at least 95%, 96%, 97%, 98%, or 99% sequence identity to the amino acid sequence set forth in SEQ ID NO: 98; or
(VIII) the heavy chain variable region comprises an amino acid sequence set forth in SEQ ID NO: 99, or comprises an amino acid sequence having at least 95%, 96%, 97%, 98%, or 99% sequence identity to the amino acid sequence set forth in SEQ ID NO: 99; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(IX) the heavy chain variable region comprises an amino acid sequence set forth in SEQ ID NO: 101 or comprises an amino acid sequence having at least 95%, 96%, 97%, 98%, or 99% sequence identity to the amino acid sequence set forth in SEQ ID NO: 101; and the light chain variable region comprises an amino acid sequence set forth in SEQ ID NO: 102, or comprises an amino acid sequence having at least 95%, 96%, 97%, 98%, or 99% sequence identity to the amino acid sequence set forth in SEQ ID NO: 102; or
(X) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 104, or comprises an amino acid sequence having at least 95%, 96%, 97%, 98%, or 99% sequence identity to the amino acid sequence set forth in SEQ ID NO: 104; or
(XI) the heavy chain variable region comprises an amino acid sequence set forth in SEQ ID NO: 105, or comprises an amino acid sequence having at least 95%, 96%, 97%, 98%, or 99% sequence identity to the amino acid sequence set forth in SEQ ID NO: 105; and the light chain variable region comprises an amino acid sequence set forth in SEQ ID NO: 106, or comprises an amino acid sequence having at least 95%, 96%, 97%, 98%, or 99% sequence identity to the amino acid sequence set forth in SEQ ID NO: 106; or
(XII) the heavy chain variable region comprises an amino acid sequence set forth in SEQ ID NO: 107 or comprises an amino acid sequence having at least 95%, 96%, 97%, 98%, or 99% sequence identity to the amino acid sequence set forth in SEQ ID NO: 107; and the light chain variable region comprises an amino acid sequence set forth in SEQ ID NO: 108, or comprises an amino acid sequence having at least 95%, 96%, 97%, 98%, or 99% sequence identity to the amino acid sequence set forth in SEQ ID NO: 108; or
(XIII) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XIV) the heavy chain variable region comprises an amino acid sequence set forth in SEQ ID NO: 111, or comprises an amino acid sequence having at least 95%, 96%, 97%, 98%, or 99% sequence identity to the amino acid sequence set forth in SEQ ID NO: 111; and the light chain variable region comprises an amino acid sequence set forth in SEQ ID NO: 112, or comprises an amino acid sequence having at least 95%, 96%, 97%, 98%, or 99% sequence identity to the amino acid sequence set forth in SEQ ID NO: 112; or
(XV) the heavy chain variable region comprises an amino acid sequence set forth in SEQ ID NO: 113 or comprises an amino acid sequence having at least 95%, 96%, 97%, 98%, or 99% sequence identity to the amino acid sequence set forth in SEQ ID NO: 113; and the light chain variable region comprises an amino acid sequence set forth in SEQ ID NO: 114, or comprises an amino acid sequence having at least 95%, 96%, 97%, 98%, or 99% sequence identity to the amino acid sequence set forth in SEQ ID NO: 114; or
(XVI) the heavy chain variable region comprises an amino acid sequence set forth in SEQ ID NO: 99, or comprises an amino acid sequence having at least 95%, 96%, 97%, 98%, or 99% sequence identity to the amino acid sequence set forth in SEQ ID NO: 99; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XVII) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XVIII) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(XIX) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(XX) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 104, or comprises an amino acid sequence having at least 95%, 96%, 97%, 98%, or 99% sequence identity to the amino acid sequence set forth in SEQ ID NO: 104; or
(XXI) the heavy chain variable region comprises an amino acid sequence set forth in SEQ ID NO: 142 or comprises an amino acid sequence having at least 95%, 96%, 97%, 98%, or 99% sequence identity to the amino acid sequence set forth in SEQ ID NO: 142; and the light chain variable region comprises an amino acid sequence set forth in SEQ ID NO: 92, or comprises an amino acid sequence having at least 95%, 96%, 97%, 98%, or 99% sequence identity to the amino acid sequence set forth in SEQ ID NO: 92.

In some embodiments, the antibody described herein is a humanized antibody.

In some embodiments, the antibody or the antigen-binding fragment thereof described herein comprises:
(I) a heavy chain variable region, comprising an amino acid sequence set forth in any one of SEQ ID NOs: 115, 116, 117, 118, 119, 120, 121, 124, 125, 128, 129, and 130; and
   a light chain variable region, comprising an amino acid sequence set forth in any one of SEQ ID NOs: 122, 123, 126, 127, 131, and 132; or
(II) a heavy chain variable region, comprising an amino acid sequence having at least 95%, 96%, 97%, 98%, or 99% sequence identity to the amino acid sequence set forth in any one of SEQ ID NOs: 115, 116, 117, 118, 119, 120, 121, 124, 125, 128, 129, and 130; and
   a light chain variable region, comprising an amino acid sequence having at least 95%, 96%, 97%, 98%, or 99% sequence identity to the amino acid sequence set forth in any one of SEQ ID NOs: 122, 123, 126, 127, 131, and 132; or
(III) a heavy chain variable region having an amino acid sequence set forth in SEQ ID NO: 118 and a light chain variable region having an amino acid sequence set forth in SEQ ID NO: 127; or
(IV) a heavy chain variable region having an amino acid sequence set forth in SEQ ID NO: 124 or 125 and a light chain variable region having an amino acid sequence set forth in SEQ ID NO: 126; or
(V) a heavy chain variable region having an amino acid sequence set forth in SEQ ID NO: 128 and a light chain variable region having an amino acid sequence set forth in SEQ ID NO: 126, 127, 131, or 132; or
(VI) a heavy chain variable region having an amino acid sequence set forth in SEQ ID NO: 129 and a light chain variable region having an amino acid sequence set forth in SEQ ID NO: 122, 123, 127, 131, or 132; or
(VII) a heavy chain variable region having an amino acid sequence set forth in SEQ ID NO: 118 and a light chain variable region having an amino acid sequence set forth in SEQ ID NO: 127; or
(VIII) a heavy chain variable region having an amino acid sequence set forth in SEQ ID NO: 128 and a light chain variable region having an amino acid sequence set forth in SEQ ID NO: 127; or
(IX) a heavy chain variable region having an amino acid sequence set forth in SEQ ID NO: 129 and a light chain variable region having an amino acid sequence set forth in SEQ ID NO: 131.

In some embodiments, the antibody or the antigen-binding fragment thereof described herein comprises:
(I) a heavy chain comprising an amino acid sequence set forth in any one of SEQ ID NOs: 134, 135, 137, 138, 140, and 141, and
   a light chain comprising an amino acid sequence set forth in any one of SEQ ID NOs: 133, 136, and 139; or
(II) a heavy chain comprising an amino acid sequence having at least 95%, 96%, 97%, 98%, or 99% sequence identity to the amino acid sequence set forth in any one of SEQ ID NOs: 134, 135, 137, 138, 140, and 141, and
   a light chain comprising an amino acid sequence having at least 95%, 96%, 97%, 98%, or 99% sequence identity to the amino acid sequence set forth in any one of SEQ ID NOs: 133, 136, and 139; or
(III) a heavy chain having an amino acid sequence set forth in SEQ ID NO: 134 or 135 and a light chain having an amino acid sequence set forth in SEQ ID NO: 133; or
(IV) a heavy chain having an amino acid sequence set forth in SEQ ID NO: 137 or 138 and a light chain having an amino acid sequence set forth in SEQ ID NO: 136; or
(V) a heavy chain having an amino acid sequence set forth in SEQ ID NO: 140 or 141 and a light chain having an amino acid sequence set forth in SEQ ID NO: 139.

In some embodiments, the antibody or the antigen-binding fragment thereof described herein is a murine antibody, a chimeric antibody, a humanized antibody or a fully human antibody, or an antigen-binding fragment thereof.

In some embodiments, for the antibody or the antigen-binding fragment thereof described herein, the antigen-binding fragment is a Fab, Fab', F(ab')₂, Fv, scFv, or sdAb.

In some embodiments, the antibody or the antigen-binding fragment thereof described herein is of any IgG subtype, such as IgG1, IgG2, IgG3, or IgG4, preferably IgG1 subtype or IgG4 subtype.

In another aspect, the present invention provides an isolated anti-CD112R antibody or an antigen-binding fragment thereof, having one or more of the following properties:
(1) binding to an epitope of human CD112R protein which is identical to, or completely or partially overlaps with that to which the anti-CD112R antibody or the antigen-binding fragment thereof described herein binds; and
(2) competing for binding to an epitope of human CD112R protein with the anti-CD112R antibody or the antigen-binding fragment thereof described herein.

In some embodiments, the antibody or the antigen-binding fragment thereof described herein has one or more of the following properties:
(1) binding with high affinity to human/cynomolgus monkey CD112R protein; and
(2) inhibiting or blocking the binding of human CD112R protein to human CD112 or cells expressing human CD112.

In some embodiments, the antibody described herein is a monoclonal antibody.

The present invention also provides a multispecific antibody comprising the light chain variable region and the heavy chain variable region of the antibody or the antigen-binding fragment thereof described herein.

The present invention also provides a single-chain antibody comprising the light chain variable region and the heavy chain variable region of the antibody or the antigen-binding fragment thereof described herein.

The present invention also provides an immunoconjugate comprising the antibody or the antigen-binding fragment thereof described herein conjugated to a therapeutic or diagnostic agent.

In yet another aspect, the present invention provides a polynucleotide molecule encoding the anti-CD112R antibody or the antigen-binding fragment thereof described herein.

In yet another aspect, the present invention provides an expression vector comprising the polynucleotide molecule described herein, wherein preferably, the vector is a eukaryotic expression vector.

In yet another aspect, the present invention provides a host cell comprising the polynucleotide molecule described herein or the expression vector described herein, wherein preferably, the host cell is a eukaryotic cell; more preferably, the host cell is a mammalian cell.

In yet another aspect, the present invention provides a method for preparing the anti-CD112R antibody or the antigen-binding fragment thereof described herein, comprising: expressing the antibody or the antigen-binding fragment thereof in the host cell described herein under conditions suitable for expression of the antibody or the antigen-binding fragment thereof, and isolating the expressed antibody or antigen-binding fragment thereof from the host cell.

In yet another aspect, the present invention provides a pharmaceutical composition, comprising the anti-CD112R antibody or the antigen-binding fragment thereof described herein, the polynucleotide molecule described herein, the expression vector described herein, or the host cell described herein, and a pharmaceutically acceptable carrier or excipient.

In yet another aspect, the present invention provides a drug combination, comprising the antibody or the antigen-binding fragment thereof described herein, or the pharmaceutical composition described herein, and one or more additional therapeutic agents.

In some embodiments, the drug combination described herein, wherein the additional therapeutic agent is selected from one or more of an anti-PD-1 antibody, an anti-PD-L1 antibody, and an anti-TIGIT antibody; preferably, the additional therapeutic agent is an anti-TIGIT antibody or a combination of an anti-TIGIT antibody and an anti-PD-1 antibody.

In yet another aspect, the present invention provides use of the antibody or the antigen-binding fragment thereof described herein, the polynucleotide molecule described herein, the expression vector described herein, the host cell described herein, the pharmaceutical composition described herein, or the drug combination in preparing a medicament for treating and/or preventing a CD112R-mediated disease or disorder, wherein preferably, the disease or disorder is a cancer; more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

In yet another aspect, the present invention provides the antibody or the antigen-binding fragment thereof described herein, the polynucleotide molecule described herein, the expression vector described herein, the host cell described herein, the pharmaceutical composition described herein, or the drug combination for treating and/or preventing a CD112R-mediated disease or disorder, wherein preferably, the disease or disorder is a cancer; more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

In yet another aspect, the present invention provides a method for treating and/or preventing a CD112R-mediated disease or disorder, comprising administering to a subject in need a therapeutically or prophylactically effective amount of the antibody or the antigen-binding fragment thereof described herein, the polynucleotide molecule described herein, the expression vector described herein, the host cell described herein, the pharmaceutical composition described herein, or drug combination described herein, wherein preferably, the disease or disorder is a cancer, and more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

In some embodiments, the antibody or the antigen-binding fragment thereof described herein is administered in combination with an additional therapeutic agent selected from one or more of an anti-PD-1 antibody, an anti-PD-L1 antibody, and an anti-TIGIT antibody; preferably, the additional therapeutic agent is an anti-TIGIT antibody or a combination of an anti-TIGIT antibody and an anti-PD-1 antibody.

In yet another aspect, the present invention provides a drug combination, comprising the antibody or the antigen-binding fragment thereof described herein, the polynucleotide molecule described herein, the expression vector described herein, the host cell described herein, or the pharmaceutical composition described herein, and one or more additional therapeutic agents.

In yet another aspect, the present invention provides a kit, comprising the antibody or the antigen-binding fragment thereof described herein, the polynucleotide molecule described herein, the expression vector described herein, the host cell described herein, the pharmaceutical composition described herein, or the drug combination described herein, wherein preferably, the kit further comprises a drug delivery device.

In yet another aspect, the present invention provides a method for detecting the presence of CD112R in a sample using the antibody or the antigen-binding fragment thereof described herein or a detection composition comprising the antibody or the antigen-binding fragment thereof described herein.

### BRIEF DESCRIPTION OF THE DRAWINGS

FIG. 1 illustrates the assay on the activity of hybridoma antibodies in combination with an anti-TIGIT antibody in a dual reporter gene system.
FIG. 2 illustrates the activity of hybridoma antibodies in combination with an anti-TIGIT antibody and an anti-PD-1 antibody in a triple reporter gene system.
FIG. 3 illustrates the assay on the binding of chimeric anti-CD112R antibodies to CHO-hCD112-expressing cells by FACS.
FIG. 4 illustrates the assay on the binding of chimeric anti-CD112R antibodies to cynomolgus monkey CD112R by ELISA.
FIG. 5 illustrates the assay on the blocking of the binding of a CD112R protein to human CHO-CD112 cells by chimeric CD112R antibodies by FACS.
FIG. 6 illustrates the activity of chimeric anti-CD112R antibodies in a CD112R/CD112 reporter gene assay system.
FIG. 7 illustrates the assay on the binding of humanized anti-CD112R antibodies to cynomolgus monkey and mouse CD112R by ELISA.
FIG. 8 illustrates the activity of humanized anti-CD112R antibodies in combination with an anti-TIGIT antibody in a dual reporter gene system.
FIG. 9 illustrates the activity of humanized anti-CD112R antibodies in combination with an anti-TIGIT antibody and an anti-PD-1 antibody in a triple reporter gene system.
FIG. 10 illustrates the tumor growth inhibition effect of humanized anti-CD112R antibodies, an anti-TIGIT antibody, and combinations thereof.

### DETAILED DESCRIPTION

### Definitions

Unless otherwise stated, embodiments of the present invention will employ conventional techniques of molecular biology (including recombinant techniques), microbiology, cytobiology, biochemistry, and immunology, which are all within the skill of the art.

In order to facilitate the understanding of the present invention, some technical and scientific terms are specifically defined as follows. Unless otherwise specifically defined herein, all technical and scientific terms used herein have the same meaning as commonly understood by those of ordinary skill in the art to which the present invention belongs. For definitions and terminology in the art, those skilled can refer specifically to Current Protocols in Molecular Biology (Ausubel). Abbreviations for amino acid residues are standard 3-letter and/or 1-letter codes used in the art to denote one of the 20 commonly used L-amino acids. The singular forms used herein (including claims) include their plural forms, unless otherwise specified in the context explicitly.

The term "about" used in combination with a numerical value is intended to encompass the numerical values in a range from a lower limit less than the specified numerical value by 5% to an upper limit greater than the specified numerical value by 5%, including but not limited to ±5%, ±2%, ±1%, and ±0.1%, as these variations are suitable for implementing the disclosed methods.

The term "and/or" should be understood as any one of the options or a combination of any two or more of the options.

As used herein, the term "or" should be understood as having the same meaning as "and/or" defined above. For example, when items in a list are separated, "or" or "and/or" should be interpreted as being inclusive, that is, including at least one number or one of a list of elements, but also including more than one, and, optionally, additional unlisted items. Only in cases where contrary terms such as "only one", "exactly one", or "consisting of ..." as used in the claims are explicitly indicated will it refer to the one number solely listed or one element of the list.

As used herein, the terms "a" and "an" should be interpreted as "at least one", unless otherwise specified.

The terms "CD112R", "PVR-associated immunoglobulin domain", "CD112 receptor", "poliovirus receptor-associated immunoglobulin domain", "Nectin-2 receptor", "C7orfl5", and "transmembrane protein PVRIG" are all used interchangeably and include variants, subtypes, species homologs of human CD112R or CD112R of other species, and analogs having at least one epitope in common with CD112R, unless otherwise stated. The term includes unprocessed full-length CD112R and CD112R in any form resulting from intracellular processing. The term encompasses "full-length" unprocessed CD112R and CD112R in any form resulting from intracellular processing or any fragment thereof, such as a splice variant or an allelic variant.

In one embodiment, CD112R refers to full-length CD112R from humans or cynomolgus monkeys, or fragments thereof (such as mature fragments thereof lacking a signal peptide).

The term "human CD112R" refers to the human sequence CD112R, such as the complete amino acid sequence of the human CD112R under NCBI accession No. NM_024070. A human CD112R sequence may differ from the human CD112R under NCBI accession No. NM_024070 by having a conservative mutation or a mutation in a non-conservative region, and the CD112R has substantially the same biological function as the human CD112R under NCBI accession No. NM_024070.

The term "percent (%) amino acid sequence identity", or simply "identity", is defined as the percentage of amino acid residues in a candidate amino acid sequence that are identical to those in a reference amino acid sequence after aligning the amino acid sequences (with gaps introduced if necessary) to achieve maximum percent sequence identity without considering any conservative substitution as part of sequence identity. Various methods in the art can be employed to perform sequence alignment so as to determine the percent amino acid sequence identity, for example, using computer software available to the public, such as BLAST, BLAST-2, ALIGN, or MEGALIGN (DNASTAR) software. Those skilled in the art can determine suitable parameters for measuring alignment, including any algorithm required to obtain maximum alignment for the full length of the aligned sequences.

The term "immune response" refers to the action of, for example, lymphocytes, antigen-presenting cells, phagocytes, granulocytes, and soluble macromolecules produced by the above cells or liver (including antibodies, cytokines and complements) that result in selective damage to, destruction of, or elimination from the human body of invading pathogens, cells or tissues infected with pathogens, cancerous cells, or, in the cases of autoimmunity or pathological inflammation, normal human cells or tissues.

The term "signal transduction pathway" or "signal transduction activity" refers to a biochemical causal relationship generally initiated by an interaction between proteins (such as binding of a growth factor to a receptor) and resulting in the transmission of a signal from one portion of a cell to another portion of the cell. In general, the transmission includes specific phosphorylation of one or more tyrosine, serine, or threonine residues on one or more proteins in a series of reactions causing signal transduction. The penultimate process typically involves a nuclear event, resulting in a change in gene expression.

The terms "activity" and "bioactivity", and the terms "biological property" and "biological characteristic" are used interchangeably herein and include, but are not limited to, epitope/antigen affinity and specificity, the ability to neutralize or antagonize CD112R activity *in vivo* or *in vitro,* IC₅₀, the *in vivo* stability of the antibody, and the immunogenic properties of the antibody. Other identifiable biological properties or characteristics of the antibody known in the art include, for example, cross-reactivity (i.e., cross-reactivity with non-human homologs of the targeted peptide, or with other proteins or tissues in general), and the ability to maintain a high expression level of the protein in mammalian cells. The aforementioned properties or characteristics are observed, determined, or assessed using techniques well known in the art, including but not limited to ELISA, FACS, or BIACORE plasma resonance analysis, unlimited *in vitro* or *in vivo* neutralization assays, receptor binding, cytokine or growth factor production and/or secretion, signal transduction, and immunohistochemistry of tissue sections of different sources (including human, primate or any other source).

The term "antibody" refers to any form of an antibody with the desired bioactivity. Thus, it is used in the broadest sense and specifically includes, but is not limited to, monoclonal antibodies (including full-length monoclonal antibodies), polyclonal antibodies, multispecific antibodies (e.g., bispecific antibodies), humanized antibodies, fully human antibodies, chimeric antibodies, and camelized single-domain antibodies.

The term "isolated antibody" refers to the purified state of a binding compound, and, in this case, means that the molecule is substantially free of other biomolecules, such as nucleic acids, proteins, lipids, sugars, or other substances such as cell debris and growth medium. The term "isolate(d)" does not mean the complete absence of such substances or the absence of water, buffers, or salts, unless they are present in amounts that will significantly interfere with the experimental or therapeutic use of the binding compounds described herein.

The term "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

The term "full-length antibody" refers to an immunoglobulin molecule comprising at least four peptide chains when present naturally, including two heavy (H) chains and two light (L) chains linked to each other by disulfide bonds. Each heavy chain consists of a heavy chain variable region (abbreviated herein as VH) and a heavy chain constant region (abbreviated herein as CH). The heavy chain constant region consists of 3 domains, CH1, CH2, and CH3. Each light chain consists of a light chain variable region (abbreviated herein as VL) and a light chain constant region. The light chain constant region consists of one domain CL. The VH and VL regions can be further divided into complementarity-determining regions (CDRs) with high variability and more conservative regions called framework regions (FRs) that are spaced apart by the CDRs. Each VH or VL region consists of 3 CDRs and 4 FRs arranged in the following order from the amino terminus to the carboxyl terminus: FR1, CDR1, FR2, CDR2, FR3, CDR3, and FR4. The variable regions of the heavy and light chains contain binding domains that interact with antigens. The constant regions of an antibody can mediate the binding of immunoglobulins to host tissues or factors, including various cells of the immune system (e.g., effector cells) and the first component (C1q) of classical complement system.

The term "antigen-binding fragment" of an antibody ("parent antibody") includes a fragment or a derivative of the antibody, generally including at least one fragment of an antigen-binding region or variable region (e.g., one or more CDRs) of a parent antibody, which retains at least some of the binding specificity of the parent antibody. Examples of binding fragments of an antibody include, but are not limited to, Fab, Fab', F(ab')₂, and Fv fragments; a diabody; a linear antibody; a single-chain antibody molecule, such as sc-Fv; and a nanobody and a multispecific antibody formed by fragments of the antibody. The binding fragment or the derivative generally retains at least 10% of the antigen-binding activity of the parent antibody when the antigen-binding activity is expressed on a molar concentration basis. Preferably, the binding fragment or the derivative retains at least 20%, 50%, 70%, 80%, 90%, 95% or 100%, or more of the antigen-binding affinity of the parent antibody. It is also contemplated that the antigen-binding fragment of the antibody may include conservative or non-conservative amino acid substitutions that do not significantly alter its bioactivity (referred to as "conservative variants" or "function-conservative variants" of the antibody). The term "binding compound" refers to both the antibody and the binding fragment thereof.

The term "single-chain Fv" or "scFv" antibody refers to an antibody fragment comprising the VH and VL domains of an antibody, where these domains are present in a single polypeptide chain. In general, an Fv polypeptide also comprises a polypeptide linker between the VH and VL domains that enables the scFv to form the desired structure for antigen binding.

The term "domain antibody" is an immunofunctional immunoglobulin fragment that contains only the heavy chain variable region or the light chain variable region. In certain cases, two or more VH regions are covalently linked to a peptide linker to form a bivalent domain antibody. The two VH regions of the bivalent domain antibody may target identical or different antigens.

The term "bivalent antibody" comprises two antigen-binding sites. In certain cases, the two binding sites have the same antigen specificity. However, a bivalent antibody may be bispecific.

The term "diabody" refers to a small antibody fragment having two antigen-binding sites and comprising a heavy chain variable domain (VH) linked to a light chain variable domain (VL) in the same polypeptide chain (VH-VL or VL-VH). By using a linker that is too short to allow pairing between two domains in one chain, the domains are forced to pair with the complementary domains of the other chain to form two antigen-binding sites.

The term "murine antibody" or "hybridoma antibody" in the present disclosure refers to an anti-human CD112R monoclonal antibody prepared according to the knowledge and skills in the art. The preparation is performed by injecting the test subject with the CD112R antigen and then isolating hybridomas expressing antibodies with the desired sequences or functional properties.

The term "chimeric antibody" is an antibody having variable domains of a first antibody and constant domains of a second antibody, wherein the first and second antibodies are from different species. Typically, the variable domains are obtained from an antibody of an animal such as a rodent ("parent antibody"), and the constant domain sequences are obtained from a human antibody, such that the resulting chimeric antibody is less likely to induce an adverse immune response in a human subject as compared to the parent rodent antibody.

The term "humanized antibody" refers to an antibody form containing sequences from both human and non-human (such as mouse and rat) antibodies. In general, a humanized antibody comprises substantially all of at least one, and typically two, variable domains, in which all or substantially all of the hypervariable loops are equivalent to those of a non-human immunoglobulin and all or substantially all of the framework regions (FRs) are those of a human immunoglobulin sequence. The humanized antibody may optionally comprise at least a portion of a human immunoglobulin constant region (Fc).

The term "fully human antibody" refers to an antibody that comprises only human immunoglobulin sequences. A fully human antibody may contain mouse glycochains if produced in mice, mouse cells or hybridomas derived from mouse cells. Likewise, "a mouse antibody" refers to an antibody that comprises only mouse immunoglobulin sequences. Alternatively, a fully human antibody may contain rat glycochains if produced in rats, rat cells or hybridomas derived from rat cells. Likewise, a "rat antibody" refers to an antibody that comprises only rat immunoglobulin sequences.

"Isotypes" of antibodies refer to types of antibodies (e.g., IgM, IgE and IgG (such as IgG1, IgG2 or IgG4)) provided by heavy chain constant region genes. Isotype also includes modified forms of one of these types in which modifications have been made to alter Fc function, for example, to enhance or attenuate effector function or binding to Fc receptors.

The term "Fc region" is used herein to define the C-terminal region of an immunoglobulin heavy chain comprising at least a part of constant regions. The term includes a native sequence Fc region and a variant Fc region. In some embodiments, the Fc region of a human IgG heavy chain extends from Cys226 or Pro230 to the carboxyl-terminus of the heavy chain. However, the C-terminal lysine (Lys447) of the Fc region may or may not exist (numbering in this paragraph is according to the EU numbering system, also known as the EU index, e.g. Rabat et al., Sequences of Proteins of Immunological Interest, 5th Ed. Public Health Service, National Institutes of Health, Bethesda, MD, 1991).

The term "epitope" refers to a protein determinant capable of specific binding to an antibody. Epitopes are usually composed of a variety of chemically active surface molecules such as amino acids or sugar side chains and usually have specific three-dimensional structural characteristics as well as specific charge characteristics. Conformational epitopes and non-conformational epitopes differ in that binding to the former rather than the latter fails in the presence of denaturing solvents.

The term "cross-reaction" as described herein refers to binding to antigenic fragments of the same target molecule of human, monkey and/or murine (mouse or rat) origin. Thus, "cross-reaction" should be understood as an interspecies reaction with the same molecule X expressed in different species. The cross-reaction specificity of monoclonal antibodies recognizing human CD112R, and monkey and/or murine (mouse or rat) CD112R can be determined by FACS analysis.

"Affinity" or "binding affinity" refers to inherent binding affinity that reflects the interaction between members of a binding pair. The affinity of molecule X for its partner Y can be generally represented by the equilibrium dissociation constant (K_{D}), which is a ratio of the dissociation rate constant (k_{dis}) to the association rate constant (kₒₙ). Affinity can be measured using common methods known in the art. One specific method for measuring affinity is the ForteBio kinetic binding assay herein.

The term "not bind to" a protein or cell means not binding to the protein or cell, or not binding to it with high affinity, that is, binding to the protein or cell with a K_{D} of 1.0 × 10⁻⁶ M or higher, more preferably 1.0 × 10⁻⁵ M or higher, more preferably 1.0 × 10⁻⁴ M or higher, 1.0 × 10⁻³ M or higher, and more preferably 1.0 × 10⁻² M or higher.

The term "high affinity" of IgG antibodies refers to a K_{D} for the antigen of 1.0 × 10⁻⁶ M or less, preferably 5.0 × 10⁻⁸ M or less, more preferably 1.0 × 10⁻⁸ M or less, 5.0 × 10⁻⁹ M or less, and more preferably 1.0 × 10⁻⁹ M or less. For other antibody subtypes, "high affinity" binding may vary. For example, "high affinity" binding of the IgM subtype refers to a K_{D} of 10⁻⁶ M or less, preferably 10⁻⁷ M or less, and more preferably 10⁻⁸ M or less.

The term "antibody-dependent cytotoxicity", "antibody-dependent cell-mediated cytotoxicity" or "ADCC" refer to a cell-mediated immune defense in which the effector cells of the immune system actively lyse target cells, such as cancer cells, whose cell membrane surface antigens bind to antibodies, such as a Claudin18.2 antibody.

The term "complement-dependent cytotoxicity" or "CDC" refers to the effector function of IgG and IgM antibodies, which, when bind to surface antigens, trigger typical complement pathways, including formation of membrane attack complexes and lysis of target cells. The antibody of the present invention, when binds to Claudin 18.2, triggers CDC against cancer cells.

The term "nucleic acid", "polynucleotide", "nucleic acid molecule" and "polynucleotide molecule" refer to deoxyribonucleic acid (DNA) or ribonucleic acid (RNA) and polymers thereof in either single- or double-stranded form. Unless explicitly limited, the term includes nucleic acids containing known analogs of natural nucleotides that have binding properties similar to that of the reference nucleic acid and are metabolized in a manner similar to naturally occurring nucleotides (see U.S. Pat. No. 8,278,036 to Kariko et al., which discloses an mRNA molecule with uridine replaced by pseudouridine, a method for synthesizing the mRNA molecule, and a method for delivering a therapeutic protein *in vivo*)*.* Unless otherwise specified, a particular nucleic acid sequence also implicitly includes conservatively modified variants thereof (e.g., degenerate codon substitutions), alleles, orthologs, SNPs, and complementary sequences as well as the sequence explicitly indicated. Specifically, degenerate codon substitutions can be achieved by generating sequences in which the third position of one or more selected (or all) codons is substituted with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" refers to any recombinant polynucleotide molecule (such as plasmid, cosmid, virus, autonomously replicating polynucleotide molecule, phage, or linear or circular single- or double-stranded DNA or RNA polynucleotide molecule) derived from any source, capable of genomic integration or autonomous replication, and comprising a polynucleotide molecule where one or more polynucleotide molecules have been linked in a functionally operative manner (i.e., operably linked). The recombinant construct typically comprises a polynucleotide of the present invention operably linked to transcription initiation regulatory sequences that will direct transcription of the polynucleotide in a host cell. Both heterologous and non-heterologous (i.e., endogenous) promoters can be used to direct expression of the nucleic acids of the present invention.

"Vector" refers to any recombinant polynucleotide construct that can be used for transformation purpose (i.e., the introduction of heterologous DNA into a host cell). One type of vector is a "plasmid", which refers to a circular double-stranded DNA loop into which additional DNA segments can be ligated. Another type of vector is a viral vector, in which additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into genome of the host cell upon introduction into a host cell, and are thereby replicated along with the host genome. In addition, certain vectors are capable of directing the expression of operably linked genes. Such vectors are referred to herein as "expression vectors".

The term "expression vector" as used herein refers to a nucleic acid molecule capable of replicating and expressing a target gene when transformed, transfected or transduced into a host cell. The expression vector comprises one or more phenotypic selectable markers and an origin of replication to ensure maintenance of the vector and to provide amplification in the host if needed.

Unless otherwise or explicitly specified in the context, "activation", "stimulation" and "treatment" for a cell or a receptor may have the same meaning. For example, the cell or the receptor is activated, stimulated or treated with a ligand. "Ligands" include natural and synthetic ligands, such as cytokines, cytokine variants, analogs, mutant proteins, and binding compounds derived from antibodies. "Ligands" also include small molecules, such as peptidomimetics of cytokines and peptidomimetics of antibodies. "Activation" may refer to the activation of a cell regulated by internal mechanisms and external or environmental factors. "Response/reaction", e.g., a response of a cell, a tissue, an organ or an organism, includes changes in biochemical or physiological behaviors (e.g., concentration, density, adhesion or migration, gene expression rate, or differentiation state within a biological compartment), where the changes are associated with activation, stimulation or treatment, or are associated with an internal mechanism such as genetic programming.

As used herein, the term "treat", "treating" or "treatment" of any disease or disorder refers in one embodiment to ameliorating the disease or disorder (i.e., slowing or arresting or reducing the progression of the disease or at least one of its clinical symptoms). In another embodiment, "treat", "treating" or "treatment" refers to ameliorating or alleviating at least one physical parameter, including those physical parameters that may not be discernible by the patient. In another embodiment, "treat", "treating" or "treatment" refers to modulating the disease or disorder, physically (e.g., stabilization of discernible symptoms), physiologically (e.g., stabilization of physical parameters), or both. Unless explicitly described herein, methods for assessing treatment and/or prevention of a disease are generally known in the art.

A "subject" includes any human or non-human animal. The term "non-human animal" includes all vertebrates, e.g., mammals and non-mammals, such as non-human primates, sheep, dog, cat, horse, cattle, chicken, amphibians, and reptiles. As used herein, the term "cyno" refers to a cynomolgus monkey.

Administration "in combination with" one or more other therapeutic agents includes simultaneous (co-) administration and sequential administration in any order.

"Therapeutically effective amount", "therapeutically effective dose" and "effective amount" refer to an amount of the anti-CD112R antibody or the antigen-binding fragment thereof of the present invention that is effective in preventing or ameliorating one or more symptoms of a disease or condition or the progression of the disease or condition when administered alone or in combination with other therapeutic drugs to a cell, a tissue or a subject. The therapeutically effective dose also refers to an amount of the antibody or the antigen-binding fragment thereof sufficient to cause amelioration of symptoms, e.g., an amount for treating, curing, preventing or ameliorating a related condition or promoting the treatment, cure, prevention or amelioration of such condition. When an active ingredient is administered to an individual alone, a therapeutically effective dose refers to the amount of the ingredient. In the case of administration in combination, a therapeutically effective dose refers to the combined amount of active ingredients that produces a therapeutic effect, regardless of whether these active ingredients are administered in combination, sequentially or simultaneously. An effective amount of a therapeutic agent will increase a diagnostic index or parameter by at least 10%, generally at least 20%, preferably at least about 30%, more preferably at least 40%, and most preferably at least 50%.

"Pharmaceutically acceptable carrier" refers to a component in a pharmaceutical formulation or composition other than the active component that is non-toxic to the subject. A pharmaceutically acceptable carrier includes, but is not limited to, buffer, excipient, stabilizer or preservative.

The term "cancer" is used herein to refer to a group of cells that exhibit abnormally high levels of proliferation and growth. The cancer may be benign (also referred to as benign tumor), premalignant or malignant. The cancer cell may be a solid cancer cell or a leukemia cancer cell. The term "tumor" as used herein refers to one or more cells comprising cancer. The term "tumor growth" is used herein to refer to the proliferation or growth of one or more cells comprising cancer, which results in a corresponding increase in the size or extent of the cancer.

### Anti-CD112R Antibody

In one aspect, the present invention provides an antibody or an antigen-binding fragment thereof specifically binding to CD112R. The term "anti-CD112R antibody", "anti-CD112R", "CD112R antibody" or "CD112R-binding antibody" refers to an antibody that is capable of binding to a CD112R protein or a fragment thereof with sufficient affinity such that the antibody can be used as a diagnostic and/or therapeutic agent targeting CD112R.

In some embodiments, the present invention provides an antibody binding to a CD112R protein. In some embodiments, the present invention provides an antibody blocking the binding of CD112R to a CD112 protein. In some embodiments, the present invention provides an antibody enhancing the activation of CD226, T cells, and/or NK cells.

In some embodiments, the antibody of the present invention binds to a human or cynomolgus monkey CD112R protein. In some embodiments, the antibody of the present invention binds to human CD112R and blocks the interaction between human CD112R and a human CD112 protein.

In some embodiments, the anti-CD112R antibody of the present invention comprises:
(I) a heavy chain variable region, comprising HCDR1, HCDR2, and HCDR3 having amino acid sequences set forth in SEQ ID NO: 1, SEQ ID NO: 2, and SEQ ID NO: 3, respectively; and a light chain variable region, comprising LCDR1, LCDR2, and LCDR3 having amino acid sequences set forth in SEQ ID NO: 4, SEQ ID NO: 5, and SEQ ID NO: 6, respectively; or
(II) a heavy chain variable region, comprising HCDR1, HCDR2, and HCDR3 having amino acid sequences set forth in SEQ ID NO: 7, SEQ ID NO: 8, and SEQ ID NO: 9, respectively; and a light chain variable region, comprising LCDR1, LCDR2, and LCDR3 having amino acid sequences set forth in SEQ ID NO: 10, SEQ ID NO: 11, and SEQ ID NO: 12, respectively; or
(III) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 4, SEQ ID NO: 5, and SEQ ID NO: 6, respectively; or
(IV) a heavy chain variable region, comprising HCDR1, HCDR2, and HCDR3 having amino acid sequences set forth in SEQ ID NO: 16, SEQ ID NO: 17, and SEQ ID NO: 18, respectively; and a light chain variable region, comprising LCDR1, LCDR2, and LCDR3 having amino acid sequences set forth in SEQ ID NO: 19, SEQ ID NO: 20, and SEQ ID NO: 21, respectively; or
(V) a heavy chain variable region, comprising HCDR1, HCDR2, and HCDR3 having amino acid sequences set forth in SEQ ID NO: 22, SEQ ID NO: 23, and SEQ ID NO: 24, respectively; and a light chain variable region, comprising LCDR1, LCDR2, and LCDR3 having amino acid sequences set forth in SEQ ID NO: 25, SEQ ID NO: 26, and SEQ ID NO: 27, respectively; or
(VI) a heavy chain variable region, comprising HCDR1, HCDR2, and HCDR3 having amino acid sequences set forth in SEQ ID NO: 28, SEQ ID NO: 29, and SEQ ID NO: 30, respectively; and a light chain variable region, comprising LCDR1, LCDR2, and LCDR3 having amino acid sequences set forth in SEQ ID NO: 31, SEQ ID NO: 32, and SEQ ID NO: 33, respectively; or
(VII) a heavy chain variable region, comprising HCDR1, HCDR2, and HCDR3 having amino acid sequences set forth in SEQ ID NO: 34, SEQ ID NO: 35, and SEQ ID NO: 36, respectively; and a light chain variable region, comprising LCDR1, LCDR2, and LCDR3 having amino acid sequences set forth in SEQ ID NO: 37, SEQ ID NO: 38, and SEQ ID NO: 39, respectively; or
(VIII) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(IX) a heavy chain variable region, comprising HCDR1, HCDR2, and HCDR3 having amino acid sequences set forth in SEQ ID NO: 28, SEQ ID NO: 29, and SEQ ID NO: 30, respectively; and a light chain variable region, comprising LCDR1, LCDR2, and LCDR3 having amino acid sequences set forth in SEQ ID NO: 46, SEQ ID NO: 47, and SEQ ID NO: 48, respectively; or
(X) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XI) a heavy chain variable region, comprising HCDR1, HCDR2, and HCDR3 having amino acid sequences set forth in SEQ ID NO: 55, SEQ ID NO: 56, and SEQ ID NO: 57, respectively; and a light chain variable region, comprising LCDR1, LCDR2, and LCDR3 having amino acid sequences set forth in SEQ ID NO: 58, SEQ ID NO: 59, and SEQ ID NO: 60, respectively; or
(XII) a heavy chain variable region, comprising HCDR1, HCDR2, and HCDR3 having amino acid sequences set forth in SEQ ID NO: 61, SEQ ID NO: 62, and SEQ ID NO: 63, respectively; and a light chain variable region, comprising LCDR1, LCDR2, and LCDR3 having amino acid sequences set forth in SEQ ID NO: 64, SEQ ID NO: 65, and SEQ ID NO: 66, respectively; or
(XIII) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XIV) a heavy chain variable region, comprising HCDR1, HCDR2, and HCDR3 having amino acid sequences set forth in SEQ ID NO: 73, SEQ ID NO: 74, and SEQ ID NO: 75, respectively; and a light chain variable region, comprising LCDR1, LCDR2, and LCDR3 having amino acid sequences set forth in SEQ ID NO: 76, SEQ ID NO: 77, and SEQ ID NO: 78, respectively; or
(XV) a heavy chain variable region, comprising HCDR1, HCDR2, and HCDR3 having amino acid sequences set forth in SEQ ID NO: 79, SEQ ID NO: 80, and SEQ ID NO: 81, respectively; and a light chain variable region, comprising LCDR1, LCDR2, and LCDR3 having amino acid sequences set forth in SEQ ID NO: 82, SEQ ID NO: 83, and SEQ ID NO: 84, respectively; or
(XVI) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XVII) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XVIII) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XIX) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XX) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XXI) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XXII) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XXIII) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XXIV) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 52, SEQ ID NO: 53, and SEQ ID NO: 54, respectively;
(XV) HCDR1, HCDR2, and HCDR3 having amino acid sequences set forth in SEQ ID NO: 143, SEQ ID NO: 144, and SEQ ID NO: 145, respectively; or HCDR1, HCDR2, and HCDR3 having 1, 2, or 3 amino acid alterations as compared with the amino acid sequences set forth in SEQ ID NO: 143, SEQ ID NO: 144, and SEQ ID NO: 145, respectively.

In some embodiments, the antibody or the antigen-binding fragment thereof described herein comprises a heavy chain variable region and/or a light chain variable region, wherein:
the heavy chain variable region comprises:
   (I) HCDR1, HCDR2, and HCDR3 having amino acid sequences set forth in SEQ ID NO: 13, SEQ ID NO: 14, and SEQ ID NO: 15, respectively; or
   (II) HCDR1, HCDR2, and HCDR3 having amino acid sequences set forth in SEQ ID NO: 40, SEQ ID NO: 41, and SEQ ID NO: 42, respectively; or
   (III) HCDR1, HCDR2, and HCDR3 having amino acid sequences set forth in SEQ ID NO: 49, SEQ ID NO: 50, and SEQ ID NO: 51, respectively; or
   (IV) HCDR1, HCDR2, and HCDR3 having amino acid sequences set forth in SEQ ID NO: 67, SEQ ID NO: 68, and SEQ ID NO: 69, respectively;
the light chain variable region comprises:
   (I) LCDR1, LCDR2, and LCDR3 having amino acid sequences set forth in SEQ ID NO: 43, SEQ ID NO: 44, and SEQ ID NO: 45, respectively; or
   (II) LCDR1, LCDR2, and LCDR3 having amino acid sequences set forth in SEQ ID NO: 52, SEQ ID NO: 53, and SEQ ID NO: 54, respectively; or
   (III) LCDR1, LCDR2, and LCDR3 having amino acid sequences set forth in SEQ ID NO: 70, SEQ ID NO: 71, and SEQ ID NO: 72, respectively.

In some embodiments, the antibody or the antigen-binding fragment thereof described herein comprises:
(1) HCDR1, HCDR2, and HCDR3 of a heavy chain variable region set forth in SEQ ID NO: 118, and LCDR1, LCDR2, and LCDR3 of a light chain variable region set forth in SEQ ID NO: 127; or
(2) HCDR1, HCDR2, and HCDR3 of a heavy chain variable region set forth in SEQ ID NO: 124 or 125, and LCDR1, LCDR2, and LCDR3 of a light chain variable region set forth in SEQ ID NO: 126; or
(3) HCDR1, HCDR2, and HCDR3 of a heavy chain variable region set forth in SEQ ID NO: 128, and LCDR1, LCDR2, and LCDR3 of a light chain variable region set forth in SEQ ID NO: 126, 127, 131, or 132; or
(4) HCDR1, HCDR2, and HCDR3 of a heavy chain variable region set forth in SEQ ID NO: 129, and LCDR1, LCDR2, and LCDR3 of a light chain variable region set forth in SEQ ID NO: 122, 123, 127, 131, or 132.

In some embodiments, the antibody or the antigen-binding fragment thereof described herein comprises a heavy chain variable region and a light chain variable region, wherein:
(I) the heavy chain variable region comprises an amino acid sequence set forth in SEQ ID NO: 85, or comprises an amino acid sequence having at least 95%, 96%, 97%, 98%, or 99% sequence identity to the amino acid sequence set forth in SEQ ID NO: 85; and the light chain variable region comprises an amino acid sequence set forth in SEQ ID NO: 86, or comprises an amino acid sequence having at least 95%, 96%, 97%, 98%, or 99% sequence identity to the amino acid sequence set forth in SEQ ID NO: 86; or
(II) the heavy chain variable region comprises an amino acid sequence set forth in SEQ ID NO: 87, or comprises an amino acid sequence having at least 95%, 96%, 97%, 98%, or 99% sequence identity to the amino acid sequence set forth in SEQ ID NO: 87; and the light chain variable region comprises an amino acid sequence set forth in SEQ ID NO: 88, or comprises an amino acid sequence having at least 95%, 96%, 97%, 98%, or 99% sequence identity to the amino acid sequence set forth in SEQ ID NO: 88; or
(III) the heavy chain variable region comprises an amino acid sequence set forth in SEQ ID NO: 89, or comprises an amino acid sequence having at least 95%, 96%, 97%, 98%, or 99% sequence identity to the amino acid sequence set forth in SEQ ID NO: 89; and the light chain variable region comprises an amino acid sequence set forth in SEQ ID NO: 90, or comprises an amino acid sequence having at least 95%, 96%, 97%, 98%, or 99% sequence identity to the amino acid sequence set forth in SEQ ID NO: 90; or
(IV) the heavy chain variable region comprises an amino acid sequence set forth in SEQ ID NO: 91, or comprises an amino acid sequence having at least 95%, 96%, 97%, 98%, or 99% sequence identity to the amino acid sequence set forth in SEQ ID NO: 91; and the light chain variable region comprises an amino acid sequence set forth in SEQ ID NO: 92, or comprises an amino acid sequence having at least 95%, 96%, 97%, 98%, or 99% sequence identity to the amino acid sequence set forth in SEQ ID NO: 92; or
(V) the heavy chain variable region comprises an amino acid sequence set forth in SEQ ID NO: 93, or comprises an amino acid sequence having at least 95%, 96%, 97%, 98%, or 99% sequence identity to the amino acid sequence set forth in SEQ ID NO: 93; and the light chain variable region comprises an amino acid sequence set forth in SEQ ID NO: 94, or comprises an amino acid sequence having at least 95%, 96%, 97%, 98%, or 99% sequence identity to the amino acid sequence set forth in SEQ ID NO: 94; or
(VI) the heavy chain variable region comprises an amino acid sequence set forth in SEQ ID NO: 95, or comprises an amino acid sequence having at least 95%, 96%, 97%, 98%, or 99% sequence identity to the amino acid sequence set forth in SEQ ID NO: 95; and the light chain variable region comprises an amino acid sequence set forth in SEQ ID NO: 96, or comprises an amino acid sequence having at least 95%, 96%, 97%, 98%, or 99% sequence identity to the amino acid sequence set forth in SEQ ID NO: 96; or
(VII) the heavy chain variable region comprises an amino acid sequence set forth in SEQ ID NO: 97, or comprises an amino acid sequence having at least 95%, 96%, 97%, 98%, or 99% sequence identity to the amino acid sequence set forth in SEQ ID NO: 97; and the light chain variable region comprises an amino acid sequence set forth in SEQ ID NO: 98, or comprises an amino acid sequence having at least 95%, 96%, 97%, 98%, or 99% sequence identity to the amino acid sequence set forth in SEQ ID NO: 98; or
(VIII) the heavy chain variable region comprises an amino acid sequence set forth in SEQ ID NO: 99, or comprises an amino acid sequence having at least 95%, 96%, 97%, 98%, or 99% sequence identity to the amino acid sequence set forth in SEQ ID NO: 99; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(IX) the heavy chain variable region comprises an amino acid sequence set forth in SEQ ID NO: 101 or comprises an amino acid sequence having at least 95%, 96%, 97%, 98%, or 99% sequence identity to the amino acid sequence set forth in SEQ ID NO: 101; and the light chain variable region comprises an amino acid sequence set forth in SEQ ID NO: 102, or comprises an amino acid sequence having at least 95%, 96%, 97%, 98%, or 99% sequence identity to the amino acid sequence set forth in SEQ ID NO: 102; or
(X) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 104, or comprises an amino acid sequence having at least 95%, 96%, 97%, 98%, or 99% sequence identity to the amino acid sequence set forth in SEQ ID NO: 104; or
(XI) the heavy chain variable region comprises an amino acid sequence set forth in SEQ ID NO: 105, or comprises an amino acid sequence having at least 95%, 96%, 97%, 98%, or 99% sequence identity to the amino acid sequence set forth in SEQ ID NO: 105; and the light chain variable region comprises an amino acid sequence set forth in SEQ ID NO: 106, or comprises an amino acid sequence having at least 95%, 96%, 97%, 98%, or 99% sequence identity to the amino acid sequence set forth in SEQ ID NO: 106; or
(XII) the heavy chain variable region comprises an amino acid sequence set forth in SEQ ID NO: 107 or comprises an amino acid sequence having at least 95%, 96%, 97%, 98%, or 99% sequence identity to the amino acid sequence set forth in SEQ ID NO: 107; and the light chain variable region comprises an amino acid sequence set forth in SEQ ID NO: 108, or comprises an amino acid sequence having at least 95%, 96%, 97%, 98%, or 99% sequence identity to the amino acid sequence set forth in SEQ ID NO: 108; or
(XIII) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XIV) the heavy chain variable region comprises an amino acid sequence set forth in SEQ ID NO: 111, or comprises an amino acid sequence having at least 95%, 96%, 97%, 98%, or 99% sequence identity to the amino acid sequence set forth in SEQ ID NO: 111; and the light chain variable region comprises an amino acid sequence set forth in SEQ ID NO: 112, or comprises an amino acid sequence having at least 95%, 96%, 97%, 98%, or 99% sequence identity to the amino acid sequence set forth in SEQ ID NO: 112; or
(XV) the heavy chain variable region comprises an amino acid sequence set forth in SEQ ID NO: 113, or comprises an amino acid sequence having at least 95%, 96%, 97%, 98%, or 99% sequence identity to the amino acid sequence set forth in SEQ ID NO: 113; and the light chain variable region comprises an amino acid sequence set forth in SEQ ID NO: 114, or comprises an amino acid sequence having at least 95%, 96%, 97%, 98%, or 99% sequence identity to the amino acid sequence set forth in SEQ ID NO: 114.
(XVI) the heavy chain variable region comprises an amino acid sequence set forth in SEQ ID NO: 99, or comprises an amino acid sequence having at least 95%, 96%, 97%, 98%, or 99% sequence identity to the amino acid sequence set forth in SEQ ID NO: 99; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110.
(XVII) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110.
(XVIII) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100.
(XIX) the heavy chain variable region comprises an amino acid sequence set forth in SEQ ID NO: 109 or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100.
(XX) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 104, or comprises an amino acid sequence having at least 95%, 96%, 97%, 98%, or 99% sequence identity to the amino acid sequence set forth in SEQ ID NO: 104; or
(XXI) the heavy chain variable region comprises an amino acid sequence set forth in SEQ ID NO: 142 or comprises an amino acid sequence having at least 95%, 96%, 97%, 98%, or 99% sequence identity to the amino acid sequence set forth in SEQ ID NO: 142; and the light chain variable region comprises an amino acid sequence set forth in SEQ ID NO: 92, or comprises an amino acid sequence having at least 95%, 96%, 97%, 98%, or 99% sequence identity to the amino acid sequence set forth in SEQ ID NO: 92.

In some embodiments, the antibody or the antigen-binding fragment thereof described herein comprises HCDR1, HCDR2, HCDR3, LCDR1, LCDR2, and LCDR3 as follows:
HCDR1: SYHMS (SEQ ID NO: 13);
HCDR2: AIX₁X₂X₃X₄X₅X₆TYYX₇DTVKG (SEQ ID NO: 146), wherein X₁ is N or S, X₂ is S or R, X₃ is N or D, X₄ is D, G or V, X₅ is I or D, X₆ is N or S, and X₇ is L or P;
HCDR3: HEDYX₈GFAMDX₉ (SEQ ID NO: 147), wherein X₈ is Y or F, and X₉ is Y or S;
LCDR1: KASQSVX₁₀NDVA (SEQ ID NO: 148), wherein X₁₀ is T or S;
LCDR2: YVSX₁₁X₁₂X₁₃T (SEQ ID NO: 149), wherein X₁₁ is N or H, X₁₂ is H or R, and X₁₃ is Y or F;
LCDR3: X₁₄QAYRSPWT (SEQ ID NO: 150), wherein X₁₄ is Q or H.

In the above substitutions, N, T, S, etc., are all polar and uncharged aliphatic amino acids; S, R, N, D, etc., are polar aliphatic amino acids; D, G, V, I, etc., are all aliphatic amino acids; L, P, etc., are all non-polar amino acids; Y, F, etc., are all aromatic amino acids; Y, S, etc., are all polar and uncharged amino acids; N, Q, H, etc., are all polar amino acids; H, R, etc., are all polar charged amino acids. Thus, these amino acids can be substituted with each other, and the obtained CDRs retain the binding activity described herein when used to construct an anti-CD112R antibody.

In some embodiments, the antibody or the antigen-binding fragment thereof described herein comprises HCDR1 having an amino acid sequence set forth in SEQ ID NO: 13; HCDR2 having an amino acid sequence shown as an amino acid sequence set forth in SEQ ID NO: 14, 41, 50, or 68; HCDR3 having an amino acid sequence shown as an amino acid sequence set forth in SEQ ID NO: 15 or 42; LCDR1 having an amino acid sequence shown as an amino acid sequence set forth in SEQ ID NO: 43 or 52; LCDR2 having an amino acid sequence shown as an amino acid sequence set forth SEQ ID NO: 44, 53, or 71; and LCDR3 having an amino acid sequence as shown in an amino acid sequence set forth in SEQ ID NO: 54 or 72.

In some embodiments, the antibody or the antigen-binding fragment thereof described herein comprises:
a heavy chain variable region, comprising an amino acid sequence set forth in any one of SEQ ID NOs: 115, 116, 117, 118, 119, 120, 121, 124, 125, 128, 129, and 130; and
a light chain variable region, comprising an amino acid sequence set forth in any one of SEQ ID NOs: 122, 123, 126, 127, 131, and 132.

In some embodiments, the antibody or the antigen-binding fragment thereof described herein comprises:
a heavy chain variable region, comprising an amino acid sequence having at least 95%, 96%, 97%, 98%, or 99% sequence identity to the amino acid sequence set forth in any one of SEQ ID NOs: 115, 116, 117, 118, 119, 120, 121, 124, 125, 128, 129, and 130; and
a light chain variable region, comprising an amino acid sequence having at least 95%, 96%, 97%, 98%, or 99% sequence identity to the amino acid sequence set forth in any one of SEQ ID NOs: 122, 123, 126, 127, 131, and 132.

In some embodiments, the antibody or the antigen-binding fragment thereof described herein comprises:
(I) a heavy chain variable region having an amino acid sequence set forth in SEQ ID NO: 118 and a light chain variable region having an amino acid sequence set forth in SEQ ID NO: 127; or
(II) a heavy chain variable region having an amino acid sequence set forth in SEQ ID NO: 124 or 125 and a light chain variable region having an amino acid sequence set forth in SEQ ID NO: 126; or
(III) a heavy chain variable region having an amino acid sequence set forth in SEQ ID NO: 128 and a light chain variable region having an amino acid sequence set forth in SEQ ID NO: 126, 127, 131, or 132; or
(IV) a heavy chain variable region having an amino acid sequence set forth in SEQ ID NO: 129 and a light chain variable region having an amino acid sequence set forth in SEQ ID NO: 122, 123, 127, 131, or 132.

In some embodiments, the antibody or the antigen-binding fragment thereof described herein comprises:
(I) a heavy chain variable region having an amino acid sequence set forth in SEQ ID NO: 118 and a light chain variable region having an amino acid sequence set forth in SEQ ID NO: 127; or
(II) a heavy chain variable region having an amino acid sequence set forth in SEQ ID NO: 128 and a light chain variable region having an amino acid sequence set forth in SEQ ID NO: 127; or
(III) a heavy chain variable region having an amino acid sequence set forth in SEQ ID NO: 129 and a light chain variable region having an amino acid sequence set forth in SEQ ID NO: 131.

In some embodiments, the antibody or the antigen-binding fragment thereof described herein comprises:
a heavy chain comprising an amino acid sequence set forth in any one of SEQ ID NOs: 134, 135, 137, 138, 140, and 141, and
a light chain comprising an amino acid sequence set forth in any one of SEQ ID NOs: 133, 136, and 139.

In some embodiments, the antibody or the antigen-binding fragment thereof described herein comprises:
a heavy chain comprising an amino acid sequence having at least 95%, 96%, 97%, 98%, or 99% sequence identity to the amino acid sequence set forth in any one of SEQ ID NOs: 134, 135, 137, 138, 140, and 141, and
a light chain comprising an amino acid sequence having at least 95%, 96%, 97%, 98%, or 99% sequence identity to the amino acid sequence set forth in any one of SEQ ID NOs: 133, 136, and 139.

In some embodiments, the antibody or the antigen-binding fragment thereof described herein comprises:
(I) a heavy chain having an amino acid sequence set forth in SEQ ID NO: 134 or 135 and a light chain having an amino acid sequence set forth in SEQ ID NO: 133; or
(II) a heavy chain having an amino acid sequence set forth in SEQ ID NO: 137 or 138 and a light chain having an amino acid sequence set forth in SEQ ID NO: 136; or
(III) a heavy chain having an amino acid sequence set forth in SEQ ID NO: 140 or 141 and a light chain having an amino acid sequence set forth in SEQ ID NO: 139.

In some embodiments, the antibody or the antigen-binding fragment thereof described herein is a murine antibody, a chimeric antibody, a humanized antibody or a fully human antibody, or an antigen-binding fragment thereof.

Any suitable method for producing antibodies may be employed to produce the antibody of the present invention. CD112R in any suitable form may be used as an immunogen (antigen) for antibody production. By way of example rather than limitation, any CD112R variant or a fragment thereof may be used as an immunogen. In some embodiments, hybridoma cells that produce murine monoclonal anti-human CD112R antibodies can be produced by methods well known in the art.

Antibodies derived from rodents (e.g., mice) may induce undesired immunogenicity of the antibodies when used as therapeutic agents *in vivo.* Repeated use of these antibodies induces immune responses in the human body to therapeutic antibodies. Such immune responses result in at least a loss of therapeutic efficacy and, in severe cases, a potentially lethal allergic reaction. One method for reducing the immunogenicity of rodent antibodies includes producing chimeric antibodies, in which the mouse variable region is fused to the human constant region (Liu et al., (1987) *Proc. Natl. Acad. Sci. USA* 84:3439-43). However, the preservation of intact rodent variable regions in a chimeric antibody can still induce deleterious immunogenicity in patients. Grafting of the complementarity-determining region (CDR) loops of the rodent variable domain onto the human framework (i.e., humanization) has been used to further minimize rodent sequences (Jones et al., (1986) *Nature* 321:522; Verhoeyen et al., (1988) *Science* 239:1534).

The amino acid sequences of the light and heavy chain variable regions and CDRs of the anti-human CD112R murine antibodies of the present invention are shown in Table 1.

**Table 1: Amino acid sequences of CDRs and variable regions of anti-CD112R murine antibodies (KABAT scheme**

| Murine Antibody | HCDR1 | HCDR2 | HCDR3 | LCDR1 | LCDR2 | LCDR3 | VH | VL |
|---|---|---|---|---|---|---|---|---|
| 1D11 | SEQ ID NO:1 | SEQ ID NOT | SEQ ID NOT | SEQ ID NO:4 | SEQ ID NO:5 | SEQ ID NO:6 | SEQ ID NO:85 | SEQ ID NO:86 |
| 2G10 | SEQ ID NOT | SEQ ID NO:8 | SEQ ID NO:9 | SEQ ID NO: 10 | SEQ ID NO:11 | SEQ ID NO:12 | SEQ ID NO:87 | SEQ ID NO:88 |
| 3D2 | SEQ ID NO: 13 | SEQ ID NO: 14 | SEQ ID NO: 15 | SEQ ID NO:4 | SEQ ID NO:5 | SEQ ID NO:6 | SEQ ID NO:89 | SEQ ID NO:90 |
| 4E2 | SEQ ID NO: 16 | SEQ ID NO: 17 | SEQ ID NO: 18 | SEQ ID NO:19 | SEQ ID NO:20 | SEQ ID NO:21 | SEQ ID NO:91 | SEQ ID NO:92 |
| | SEQ ID NO: 143 | SEQ ID NO: 144 | SEQ ID NO: 145 | | | | SEQ ID NO: 142 | |
| 4E5 | SEQ ID NO:22 | SEQ ID NO:23 | SEQ ID NO:24 | SEQ ID NO:25 | SEQ ID NO:26 | SEQ ID NO:27 | SEQ ID NO:93 | SEQ ID NO:94 |
| 5B1 | SEQ ID NO:28 | SEQ ID NO:29 | SEQ ID NO:30 | SEQ ID NO:31 | SEQ ID NO:32 | SEQ ID NO:33 | SEQ ID NO:95 | SEQ ID NO:96 |
| 5B2 | SEQ ID NO:34 | SEQ ID NO:35 | SEQ ID NO:36 | SEQ ID NO:37 | SEQ ID NO:38 | SEQ ID NO:39 | SEQ ID NO:97 | SEQ ID NO:98 |
| 5C10 | SEQ ID NO:40 | SEQ ID NO:41 | SEQ ID NO:42 | SEQ ID NO:43 | SEQ ID NO:44 | SEQ ID NO:45 | SEQ ID NO:99 | SEQ ID NO:100 |
| 6A6 | SEQ ID NO:28 | SEQ ID NO:29 | SEQ ID NO:30 | SEQ ID NO:46 | SEQ ID NO:47 | SEQ ID NO:48 | SEQ ID NO:101 | SEQ ID NO:102 |
| 6A10 | SEQ ID NO:49 | SEQ ID NO:50 | SEQ ID NO:51 | SEQ ID NO:52 | SEQ ID NO:53 | SEQ ID NO:54 | SEQ ID NO: 103 | SEQ ID NO:104 |
| 6C6 | SEQ ID NO:55 | SEQ ID NO:56 | SEQ ID NO:57 | SEQ ID NO:58 | SEQ ID NO:59 | SEQ ID NO:60 | SEQ ID NO:105 | SEQ ID NO:106 |
| 6D2 | SEQ ID NO:61 | SEQ ID NO:62 | SEQ ID NO:63 | SEQ ID NO:64 | SEQ ID NO:65 | SEQ ID NO:66 | SEQ ID NO:107 | SEQ ID NO:108 |
| 7C2 | SEQ ID NO:67 | SEQ ID NO:68 | SEQ ID NO:69 | SEQ ID NO:70 | SEQ ID NO:71 | SEQ ID NO:72 | SEQ ID NO:109 | SEQ ID NO: 110 |
| 8D3 | SEQ ID NO:73 | SEQ ID NO:74 | SEQ ID NO:75 | SEQ ID NO:76 | SEQ ID NO:77 | SEQ ID NO:78 | SEQ ID NO:111 | SEQ ID NO:112 |
| 8H8 | SEQ ID NO:79 | SEQ ID NO:80 | SEQ ID NO:81 | SEQ ID NO:82 | SEQ ID NO:83 | SEQ ID NO:84 | SEQ ID NO: 113 | SEQ ID NO: 114 |

1D11VH: 
EVQLQQSGPELVKPGASVKISCKASGYPFINYNIHWLRQSRGKSLEWIGYVYPYNGV
PGYNQKFKSKATLTADNSFSTAYMEFRSLTSEDSAVYFCARERGRDYFDSWGQGTSLT
VSS (SEQ ID NO: 85)
HCDR1: **NYNIH** (SEQ ID NO: 1)
HCDR2: **YVYPYNGVPGYNOKFKS** (SEQ ID NO: 2)
HCDR3: **ERGRDYFDS** (SEQ ID NO: 3)
1D11VL: (identical to 3D2 VL) 
DIQLTQSPASLSVSVGETVTITCRVSANIYSNLVWYHQRQGKSPQLLVYGGTNLAD
GVPSRFSGSGSGTQFSLKINSLQSEDFGSYHCQHFWSTPYTFGGGTKLEIK (SEQ ID NO:
86)
LCDR1: **RVSANIYSNLV** (SEQ ID NO: 4)
LCDR2: **GGTNLAD** (SEQ ID NO: 5)
LCDR3: **OHFWSTPYT** (SEQ ID NO: 6)
2G10VH: 
QVQLQQSGAELVKPGASVKLSCKASGYTFTSHYMYWLKQRPGQGLEWIGEINPSN
GDTKFNEKFKSKATLTVDKSSSTAYMQLSSLTSEDSAVYYCTREKDAFSALDYWGQGT
SVTDSS (SEQ ID NO: 87)
HCDR1: **SHYMY** (SEQ ID NO: 7)
HCDR2: **EINPSNGDTKFNEKFKS** (SEQ ID NO: 8)
HCDR3: **EKDAFSALDY** (SEQ ID NO: 9)
2G10VL: 
DIQMIQSPSSMFASLGDRVSLSCRASQGIRGHLDWYQQKPGGTIKLLIYSTSILNFGV
PSRFSGSGSGSDYSLTISSLESEDFADYYCLQRNAYPLTFGAGTKLELK (SEQ ID NO: 88)
LCDR1: **RASOGIRGHLD** (SEQ ID NO: 10)
LCDR2: **STSILNF** (SEQ ID NO: 11)
LCDR3: **LORNAYPLT** (SEQ ID NO: 12)
3D2VH: 
DVQLVESGGGLVKLGGSLKLSCVASGFTFSSYHMSWIRQTPEKRLELVAAINSNDIN
TYYLDTVKGRFTISRDIAKNTLHLQMSSLKSEDTALYYCARHEDYYGFAMDYWGQGTS
VTVSS (SEQ ID NO: 89)
HCDR1: **SYHMS** (SEQ ID NO: 13)
HCDR2: **AINSNDINTYYLDTVKG** (SEQ ID NO: 14)
HCDR3: **HEDYYGFAMDY** (SEQ ID NO: 15)
3D2VL (identical to 1D11 VL): 
DIQLTQSPASLSVSVGETVTITCRVSANIYSNLVWYHQRQGKSPQLLVYGGTNLAD
GVPSRFSGSGSGTQFSLKINSLQSEDFGSYHCQHFWSTPYTFGGGTKLEIK (SEQ ID NO:
90)
LCDR1: **RVSANIYSNLV** (SEQ ID NO: 4)
LCDR2: **GGTNLAD** (SEQ ID NO: 5)
LCDR3: **QHFWSTPYT** (SEQ ID NO: 6)
4E2VH1: 
EVQLQQSGAELVRSGTSVTLSCTASGFNIRDYFIHWVKQRPEQGLEWIGWIIPASGD
TDCAPIFQGKATLTADTFSNTAYLHLNSLTSEDTAVYYCNTWVWSPYAVDYWGQGTSV
TVSS (SEQ ID NO: 91)
HCDR1: **DYFIH** (SEQ ID NO: 16)
HCDR2: **WIIPASGDTDCAPIFQG** (SEQ ID NO: 17)
HCDR3: **WVWSPYAVDY** (SEQ ID NO: 18)
4E2VH2: 
EVQLQQSGAELVKPGASVKISCKASGYTFTDYNMDWVKQSHGKSLEWIGDIHPKY
DRTSYNQNFKGKATLTVDKSSRTAYMELRSLTSEDTAVYFCVRINDFDEGFAMDYWG
QGTSVTVSS (SEQ ID NO: 142)
HCDR1: DYNMD (SEQ ID NO: 143)
HCDR2: **DIHPKYDRTSYNQNFKG** (SEQ ID NO: 144)
HCDR3: **INDFDEGFAMDY** (SEQ ID NO: 145)
4E2VL: 
QIVLTQSPAIMSVSPGEKVTMTCSATSSINYMHWYQQKSGTSPKRWIYDTSKLASG
VPARFSGSGSGTSYSLTISNMEAEDAATYYCQQWTSNPLTFGAGTKLELK (SEQ ID NO:
92)
LCDR1: **SATSSINYMH** (SEQ ID NO: 19)
LCDR2: **DTSKLAS** (SEQ ID NO: 20)
LCDR3: **QQWTSNPLT** (SEQ ID NO: 21)
4E5VH: 
EVMLVESGGGLVKPGGSLKLSCAASGFTFSNYIMSWVRQTPDMRLEWVATISSGGS
STYYVDRVKGGFPFSRDSVKYILYVHTSSLRSADTAIYYCAKHEDWDPLYYYSMDYWG
QGTSVTVSS (SEQ ID NO: 93)
HCDR1: **NYIMS** (SEQ ID NO: 22)
HCDR2: **TISSGGSSTYYVDRVKG** (SEQ ID NO: 23)
HCDR3: **HEDWDPLYYYSMDY** (SEQ ID NO: 24)
4E5VL: 
QIVLTQSPTVMSASPGDKVTMTCSASSSVSYMYWFQQKPGSSPRLLIYDTSHLAFGV
PSRFSGSRSGTSYSLTISRMEAEDTATYYCQQWTTYPLTFGAGTKLELK (SEQ ID NO: 94)
LCDR1: **SASSSVSYMY** (SEQ ID NO: 25)
LCDR2: **DTSHLAF** (SEQ ID NO: 26)
LCDR3: **QQWTTYPLT** (SEQ ID NO: 27)
5B1VH (identical to 6A6 VH): 
EVQLQQSGAELVRSGTSVTLSCTASGFNIRDYFIHWVKQRPEQGLEWIGWIIPASGD
TDCAPIFQGKATLTADTFSNTAYLHLNSLTSEDTAVYYCNTWVWSPYAVDYWGQGTSV
TVSS (SEQ ID NO: 95)
HCDR1: **DYFIH** (SEQ ID NO: 28)
HCDR2: **WIIPASGDTDCAPIFQG** (SEQ ID NO: 29)
HCDR3: **WVWSPYAVDY** (SEQ ID NO: 30)
5B1VL: 
DIVMTQFPSSLSVPVGEKVTMNCKSSQSLLNSGNQKNFLAWYQQKPGQPPKLLIYG
ASTRESGVPYRFTGSGSGTDFTLTISSVQAEDLAVYYCQNDHSYPWTFGGGTKLEIK
(SEQ ID NO: 96)
LCDR1: **KSSOSLLNSGNOKNFLA** (SEQ ID NO: 31)
LCDR2: **GASTRES** (SEQ ID NO: 32)
LCDR3: **QNDHSYPWT** (SEQ ID NO: 33)
5B2VH: 
EVQLQQSGPELVKPGASVRISCKASGYTFTDYNMHWVKQSHGKSLEWIGYIYPYNG
GTGHNQNFKSKATLTVDKSSSTAYMEFRSLTSDDSAVYYCAREDFRHEKMDYWGQGT
SVTVSS (SEQ ID NO: 97)
HCDR1: **DYNMH** (SEQ ID NO: 34)
HCDR2: **YIYPYNGGTGHNQNFKS** (SEQ ID NO: 35)
HCDR3: **EDFRHEKMDY** (SEQ ID NO: 36)
5B2VL: 
DIQMTQSPASLSASVGETVTITCRTSDNIYVYLSWYQQKQGKSPQLLVYNAKTLTE
NVPSRFSGSGSGTQFSLKINSLQPEDFGSYYCQHHYGTPYTFGGGTKLEIK (SEQ ID NO:
98)
LCDR1: **RTSDNIYVYLS** (SEQ ID NO: 37)
LCDR2: **NAKTLTE** (SEQ ID NO: 38)
LCDR3: **QHHYGTPYT** (SEQ ID NO: 39)
5C10VH: 
DVKLVESGGGLVKLGGSLKLSCAASGFTFSSYHMSWVRQTPEKRLELVAAISRDGD
STYYPDTVKGRFTISRDDAENTLYLQMSSLKSEDTALYYCSRHEDYFGFAMDSWGQGT
SVTVSS (SEQ ID NO: 99)
HCDR1: **SYHMS** (SEQ ID NO: 40)
HCDR2: **AISRDGDSTYYPDTVKG** (SEQ ID NO: 41)
HCDR3: **HEDYFGFAMDS** (SEQ ID NO: 42)
5C10VL: 
SIVMTQTPKFLLVSAGDRVTITCKASQSVTNDVAWYQQKPGQSPKLLMYYVSNHY
TGVPDRFIGSGYGTDFTFTISTVQAEDLAVYFCQQAYRSPWTFGGGTKLEIK (SEQ ID
NO: 100)
LCDR1: **KASQSVTNDVA** (SEQ ID NO: 43)
LCDR2: **YVSNHYT** (SEQ ID NO: 44)
LCDR3: **QQAYRSPWT** (SEQ ID NO: 45)
6A6VH (identical to 5B1 VH): 
EVQLQQSGAELVRSGTSVTLSCTASGFNIRDYFIHWVKQRPEQGLEWIGWIIPASGD
TDCAPIFQGKATLTADTFSNTAYLHLNSLTSEDTAVYYCNTWVWSPYAVDYWGQGTSV
TVSS (SEQ ID NO: 101)
HCDR1: **DYFIH** (SEQ ID NO: 28)
HCDR2: **WIIPASGDTDCAPIFQG** (SEQ ID NO: 29)
HCDR3: **WVWSPYAVDY** (SEQ ID NO: 30)
6A6VL: 
DVLMTQTPLSLPVSLGDQTSISCRSSQSIVHSNGNTYLHWYLQKPGQSPKLLIYKVS
NRFSGVPDRFSGSGSGTDFTLKISRVEAEDLGVYYCFQGSHVPWTFGGGTRLEIK (SEQ
ID NO: 102)
LCDR1: **RSSQSIVHSNGNTYLH** (SEQ ID NO: 46)
LCDR2: **KVSNRFS** (SEQ ID NO: 47)
LCDR3: **FQGSHVPWT** (SEQ ID NO: 48)
6A10VH: 
DVKLVESGGGLVKLGGSLKLSCAASGFTFSSYHMSWVRQTPEKRLELVAAINSNVIS
TYYLDTVKGRFTISRDNAKNTLYLQMSSLKSEDTALYYCARHEDYYGFAMDYWGQGT
SVTVSS (SEQ ID NO: 103)
HCDR1: **SYHMS** (SEQ ID NO: 49)
HCDR2: **AINSNVISTYYLDTVKG** (SEQ ID NO: 50)
HCDR3: **HEDYYGFAMDY** (SEQ ID NO: 51)
6A10VL: 
SIVMTQTPKFLLVSAGDRVTITCKASQSVSNDVAWYQEKPGQSPKLLMYYVSHRFT
GVPDRFTGSGYGTDFTFTINTVQAEDLAVYFCHQAYRSPWTFGGGTKLEIK (SEQ ID NO:
 104)
LCDR1: **KASQSVSNDVA** (SEQ ID NO: 52)
LCDR2: **YVSHRFT** (SEQ ID NO: 53)
LCDR3: **HOAYRSPWT** (SEQ ID NO: 54)
6C6VH:

EVQLQQSGPELVRPGASVRISCKASGYTFTDYNMNWVKQSHGKSLEWIGYIYPYNG
GAGYNQNFKNKATLTVDTYSRAAYMELRSLTSEDSAVYYCARERGMQRDYFDYWGQ
GTILTVSS (SEQ ID NO: 105)

HCDR1: **DYNMN** (SEQ ID NO: 55)
HCDR2: **YIYPYNGGAGYNONFKN** (SEQ ID NO: 56)
HCDR3: **ERGMORDYFDY** (SEQ ID NO: 57)
6C6VL: 
DIQMTQSPASLSVSVGETVTITCRTSENIYSNLAWYQQKQGKSPQLLVYAATNLAD
GVPSRFSGSGSGTQFSLKINSLQSGDFGSYFCQHFWGTPYTFGGGTKLEIK (SEQ ID NO:
106)
LCDR1: **RTSENIYSNLA** (SEQ ID NO: 58)
LCDR2: **AATNLAD** (SEQ ID NO: 59)
LCDR3: **QHFWGTPYT** (SEQ ID NO: 60)
6D2VH: 
EVQLQQSGPELVKPGASVKISCKASGYTFTDYNIHWVKQSHGKRPDWIGYNYPYNG
GAGYNQNFRSRATLTVDKFSSTAYMELRSLTSEDSAVYYCAGEEVRRGPFYFWGQGTS
VTVSS (SEQ ID NO: 107)
HCDR1: **DYNIH** (SEQ ID NO: 61)
HCDR2: **YNYPYNGGAGYNQNFRS** (SEQ ID NO: 62)
HCDR3: **GEEVRRGPFYF** (SEQ ID NO: 63)
6D2VL: 
DIQMTQSPDSLSASVGETVTITCRTSENIYSFLVWYQQKQGKSPQLLVYNAKTLAE
GVPSRFSGSGSGTQFSLKINSLQPEDFGTYYCQHYYGTPYTFGGGTKLEIK (SEQ ID NO:
108)
LCDR1: **RTSENIYSFLV** (SEQ ID NO: 64)
LCDR2: **NAKTLAE** (SEQ ID NO: 65)
LCDR3: **QHYYGTPYT** (SEQ ID NO: 66)
7C2VH: 
DVKLVESGGGLVKLGGSLKLSCAASGFTFSSYHMSWIRQTPEKRLELVAAINSNGIN
TYYLDTVKGRFTISRDNAKNTLYLQMSSLKSEDTALYYCARHEDYYGFAMDYWGQGT
SVTVSS (SEQ ID NO: 109)
HCDR1: **SYHMS** (SEQ ID NO: 67)
HCDR2: **AINSNGINTYYLDTVKG** (SEQ ID NO: 68)
HCDR3: **HEDYYGFAMDY** (SEQ ID NO: 69)
7C2VL: 
SIVMTQTPKFLLVSAGDRVTITCKASQSVSNDVAWYQQKPGQSPKLLMYYVSNRFT
GVPDRFTGSGYGTDFTFTINTVQAEDLAVYFCQQAYRSPWTFGGGTKLEIK (SEQ ID NO:
110)
LCDR1: **KASQSVSNDVA** (SEQ ID NO: 70)
LCDR2: **YVSNRFT** (SEQ ID NO: 71)
LCDR3: **QQAYRSPWT** (SEQ ID NO: 72)
8D3VH: 
QIQLVQSGPELKKPGETVKISCKASGYTFTDYSMHWVKQAPGKGLKWVGWISTET
AQPTYADDFRGRFAFSLETSASTAYLQINNLRNEDTATYFCATGNYGWYFSVWGAGTT
VTVSS (SEQ ID NO: 111)
HCDR1: **DYSMH** (SEQ ID NO: 73)
HCDR2: **WISTETAQPTYADDFRG** (SEQ ID NO: 74)
HCDR3: **TGNYGWYFSV** (SEQ ID NO: 75)
8D3VL: 
ENVLTQSPAIMSASPGEKVTMTCRATSGVSSSYLFWYQQKSGASPKIWIYSTSNLAS
GVPTRFSGSGSGTSYSLTISSVEAEDAATYYCQQYSGYPYTFGGGTKLEIK (SEQ ID NO:
112)
LCDR1: **RATSGVSSSYLF** (SEQ ID NO: 76)
LCDR2: **STSNLAS** (SEQ ID NO: 77)
LCDR3: **QQYSGYPYT** (SEQ ID NO: 78)
8H8VH: 
EVKLVESGGGLVKPGGSLKVSCAASGFTFSDYPMSWVRQTPDKRLEWVACIDTDE
NTYYPDSVKGRFTISRDNGRNIVNLQMSSLRSEDAAMYYCARGWDDYAAWFAYWGQ
GTLVTVSA (SEQ ID NO: 113)
HCDR1: **DYPMS** (SEQ ID NO: 79)
HCDR2: **CIDTDENTYYPDSVKG** (SEQ ID NO: 80)
HCDR3: **GWDDYAAWFAY** (SEQ ID NO: 81)
8H8VL: 
ENVLTQFPAIMSVFLGERVTMSCRASSSVNYMYWYQQKSDASPKLWIYHTSNLAP
GVPARFSGSGSGNSYSLTISSMEGEDAATYYCQQFTSSPWTFGGGTKLEIK (SEQ ID NO:
114)
LCDR1: **RASSSVNYMY** (SEQ ID NO: 82)
LCDR2: **HTSNLAP** (SEQ ID NO: 83)
LCDR3: **QQFTSSPWT** (SEQ ID NO: 84)

In some embodiments, the chimeric or humanized antibodies of the present invention can be prepared based on the sequences of the prepared murine monoclonal hybridoma antibodies. DNA encoding the immunoglobulin heavy and light chains can be obtained from a murine hybridoma of interest and engineered to comprise non-murine (e.g., human) immunoglobulin sequences using standard molecular biology techniques.

In some embodiments, to prepare the chimeric CD112R antibodies of the present invention, the chimeric heavy chains and the chimeric light chains can be obtained by operably linking the immunoglobulin heavy chain and light chain variable regions of hybridoma origin to human IgG constant regions respectively using methods known in the art (see, e.g., U.S. Pat. No.4,816,567 to Cabilly et al.). In some embodiments, the chimeric antibodies of the present invention comprise constant regions which can be selected from subtype of any human IgG, such as IgG1, IgG2, IgG3 and IgG4, preferably IgG4.

In some embodiments, the chimeric CD112R antibodies of the present invention can be obtained by "mixing and matching" a chimeric light chain expression plasmid with a chimeric heavy chain expression plasmid to transfect expression cells. The CD112R binding of such "mixed and matched" antibodies can be assayed using the above binding assays and other conventional binding assays (e.g., ELISA).

The precise amino acid sequence boundaries of the variable region CDRs of the antibodies of the present invention can be determined using any of many well-known schemes, including Chothia based on the three-dimensional structure of antibodies and the topology of the CDR loops (Chothia et al., (1989) Nature 342: 877-883; Al-Lazikani et al., Standard conformations for the canonical structures of immunoglobulins, Journal of Molecular Biology, 273, 927-948(1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and North CDR definition based on the affinity propagation clustering using a large number of crystal structures.

Unless otherwise specified, the boundaries of the CDRs of the antibodies of the present invention can be determined by one skilled in the art according to any scheme (e.g., different assignment systems or combinations) in the art.

It should be noted that the boundaries of the CDRs of the variable regions of the same antibody obtained based on different assignment systems may differ. That is, the CDR sequences of the variable regions of the same antibody defined under different assignment systems are different. Thus, when it comes to defining an antibody with a particular CDR sequence defined in the present invention, the scope of the antibody also encompasses antibodies whose variable region sequences comprise the particular CDR sequence but whose claimed CDR boundaries differ from the particular CDR boundaries defined in the present invention due to the application of different schemes (e.g., different assignment systems or combinations).

Antibodies with different specificities (i.e., different binding sites for different antigens) have different CDRs. However, although CDRs vary from antibody to antibody, only a limited number of amino acid positions within a CDR are directly involved in antigen binding. The smallest overlapping region can be determined using at least two of the Kabat, Chothia, AbM, Contact and North methods, thereby providing a "smallest binding unit" for antigen binding. The smallest binding unit may be a sub-portion of the CDR. As will be clear to those skilled in the art, the residues in the remainder of the CDR sequences can be determined by the antibody structure and protein folding. Thus, variants of any of the CDRs presented herein are also contemplated by the present invention. For example, in a variant of one CDR, the amino acid residue of the smallest binding unit may remain unchanged, while the remaining CDR residues defined according to Kabat or Chothia may be replaced by conservative amino acid residues.

In the present invention, the expression plasmids for the various chimeric heavy chain and light chains were paired and matched to transfect expression cells, and 72 chimeric anti-human CD112R antibodies were obtained. Their numbers and matching modes are shown in Table 2.

**Table 2: Numbers of chimeric antibodies and sources of their heavy chain variable regions and light chain variable regions**

| | 2G10-VH | 4E2-VH1 | 4E2-VH2 | 4E5-VH | 5B2-VH | 6A10-VH | 7C2-VH | 5C10-VH | 3D2-VH |
|---|---|---|---|---|---|---|---|---|---|
| 2G10-VL | Chi-1 | Chi-9 | Chi-17 | Chi-25 | Chi-33 | Chi-41 | Chi-49 | Chi-57 | Chi-65 |
| 4E2-VL | Chi-2 | Chi-10 | Chi-18 | Chi-26 | Chi-34 | Chi-42 | Chi-50 | Chi-58 | Chi-66 |
| 4E5-VL | Chi-3 | Chi-11 | Chi-19 | Chi-27 | Chi-35 | Chi-43 | Chi-51 | Chi-59 | Chi-67 |
| 5B2-VL | Chi-4 | Chi-12 | Chi-20 | Chi-28 | Chi-36 | Chi-44 | Chi-52 | Chi-60 | Chi-68 |
| 6A10-VL | Chi-5 | Chi-13 | Chi-21 | Chi-29 | Chi-37 | Chi-45 | Chi-53 | Chi-61 | Chi-69 |
| 7C2-VL | Chi-6 | Chi-14 | Chi-22 | Chi-30 | Chi-38 | Chi-46 | Chi-54 | Chi-62 | Chi-70 |
| 5C10-VL | Chi-7 | Chi-15 | Chi-23 | Chi-31 | Chi-39 | Chi-47 | Chi-55 | Chi-63 | Chi-71 |
| 1D11-VL | Chi-8 | Chi-16 | Chi-24 | Chi-32 | Chi-40 | Chi-48 | Chi-56 | Chi-64 | Chi-72 |

For the humanized antibodies of the present invention, murine CDR regions can be inserted into human germline framework regions using methods known in the art. See U.S. Pat. No. 5,225,539 to Winter et al., and U.S. Pat. Nos. 5,530,101; 5,585,089; 5,693,762 and 6,180,370 to Queen et al.

The amino acid sequences of the light and heavy chain variable regions and CDRs of the humanized anti-human CD112R antibodies of the present invention are as follows:
3D2VH-1: SEQ ID NO: 115 
EVQLVESGGGLVQPGGSLRLSCAASGFTFSSYHMSWVRQAPGKGLELVAAINSNDIN
TYYLDTVKGRFTISRDIAKNSLYLQMNSLRAEDTAVYYCARHEDYYGFAMDYWGQGTL
VTVSS
3D2VH-2: SEQ ID NO: 116
3D2VH-3: SEQ ID NO: 117
5C10 VH-1: SEQ ID NO: 118
5C10 VH-2: SEQ ID NO: 119
5C10 VH-3: SEQ ID NO: 120
5C10 VH-4: SEQ ID NO: 121 
EVQLVESGGGLVQPGGSLRLSCAASGFTFSSYHMSWVRQAPGKGLELVAAISRDGD
STYYPDTVKGRFTISRDDAENSLYLQMNSLRAEDTAVYYCSRHEDYFGFAMDSWGQGTL
VTVSS
5C10 VL-1: SEQ ID NO: 122 
DIVMTQSPDSLAVSLGERATINCKASQSVTNDVAWYQQKPGQPPKLLIYYVSNHYTG
VPDRFSGSGSGTDFTLTISSLQAEDVAVYFCQQAYRSPWTFGQGTKLEIK
5C10 VL-2: SEQ ID NO: 123 
AIQMTQSPSSLSASVGDRVTITCKASQSVTNDVAWYQQKPGKAPKLLIYYVSNHYTG
VPSRFSGSGSGTDFTLTISSLQPEDFATYFCQQAYRSPWTFGQGTKLEIK
6A10 VH-1: SEQ ID NO: 124 
EVQLVESGGGLVQPGGSLRLSCAASGFTFSSYHMSWVRQAPGKGLELVSAINSNVIS
TYYLDTVKGRFTISRDNAKNSLYLQMNSLRAEDTAVYYCARHEDYYGFAMDYWGQGTL
VTVSS
6A10 VH-2: SEQ ID NO: 125 
EVQLVESGGGLVKPGGSLRLSCAASGFTFSSYHMSWVRQAPGKGLELVSAINSNVIS
TYYLDTVKGRFTISRDNAKNSLYLQMNSLRAEDTAVYYCARHEDYYGFAMDYWGQGTL
VTVSS
6A10 VL-1: SEQ ID NO: 126 
EIVMTQSPPTLSLSPGERVTLSCKASQSVSNDVAWYQEKPGQAPRLLIYYVSHRFTGI
PARFSGSGSGTDFTLTISSLQPEDFAVYYCHQAYRSPWTFGQGTKLEIK
6A10 VL-2: SEQ ID NO: 127 
DIVMTQSPDSLAVSLGERATINCKASQSVSNDVAWYQQKPGQPPKLLIYYVSHRFTG
VPDRFSGSGSGTDFTLTISSLQAEDVAVYFCHQAYRSPWTFGQGTKLEIK
7C2 VH-1: SEQ ID NO: 128 
EVQLVESGGGLVKPGGSLRLSCAASGFTFSSYHMSWVRQAPGKGLELVSAINSNGIN
TYYLDTVKGRFTISRDNAKNSLYLQMNSLRAEDTAVYYCARHEDYYGFAMDYWGQGTL
VTVSS
7C2 VH-2: SEQ ID NO: 129 
EVQLVESGGGLVQPGGSLRLSCAASGFTFSSYHMSWIRQAPGKGLELVAAINSNGIN
TYYLDTVKGRFTISRDNAKNSLYLQMNSLRAEDTAVYYCARHEDYYGFAMDYWGQGTL
VTVSS
7C2 VH-3: SEQ ID NO: 130 
EVQLVESGGGLVQPGGSLRLSCAASGFTFSSYHMSWIRQAPGKGLELVAAINSNDIN
TYYLDTVKGRFTISRDNAKNSLYLQMNSLRAEDTAVYYCARHEDYYGFAMDYWGQGTL
VTVSS
7C2 VL-1: SEQ ID NO: 131 
DIQMTQSPSSLSASVGDRVTITCKASQSVSNDVAWYQQKPGKAPKLLIYYVSNRFTG
VPSRFSGSGSGTDFTLTISSLQPEDFATYFCQQAYRSPWTFGQGTKLEIK
7C2 VL-2: SEQ ID NO: 132

EIVMTQSPPTLSLSPGERVTLSCKASQSVSNDVAWYQQKPGQAPRLLIYYVSNRFTGI
PARFSGSGSGTDFTLTISSLQPEDFAVYFCQQAYRSPWTFGQGTKLEIK

The numbers of the humanized anti-CD112R antibodies of the present invention and sources of their heavy chain variable regions and light chain variable regions are shown in Table 3.

**Table 3: Numbers of humanized anti-CD112R antibodies and sources of their heavy chain variable regions and light chain variable regions**

| **Number** | **3D2VH1** | **3D2VH2** | **5C10VH1** | **5C10VH2** | **5C10VH3** | **5C10VH4** | **6A10VH1** | **6A10VH2** | **7C2VH1** | **7C2VH2** |
|---|---|---|---|---|---|---|---|---|---|---|
| **5C10VL1** | Hu1 | Hu7 | Hu13 | Hu19 | Hu25 | Hu31 | Hu37 | Hu43 | Hu49 | Hu55 |
| **5C10VL2** | Hu2 | Hu8 | Hu14 | Hu20 | Hu26 | Hu32 | Hu38 | Hu44 | Hu50 | Hu56 |
| **6A10VL1** | Hu3 | Hu9 | Hu15 | Hu21 | Hu27 | Hu33 | Hu39 | Hu45 | Hu51 | Hu57 |
| **6A10VL2** | Hu4 | Hu10 | Hu16 | Hu22 | Hu28 | Hu34 | Hu40 | Hu46 | Hu52 | Hu58 |
| **7C2VL1** | Hu5 | Hu11 | Hu17 | Hu23 | Hu29 | Hu35 | Hu41 | Hu47 | Hu53 | Hu59 |
| **7C2VL2** | Hu6 | Hu12 | Hu18 | Hu24 | Hu30 | Hu36 | Hu42 | Hu48 | Hu54 | Hu60 |

The light and heavy chain amino acid sequences of antibodies Hu16, Hu52 and Hu59 are shown below, and the sequences outside the variable regions of LC and HC of the rest humanized antibodies are identical to the corresponding sequences of Hu16, Hu52 and Hu59, wherein the humanized antibodies for biological tests all have IgG4 heavy chains.
Hu16-LC: SEQ ID NO: 133 (LCDR1-LCDR3 are SEQ ID NOs: 52, 53, and 54, respectively) 
DIVMTQSPDSLAVSLGERATINCKASQSVSNDVAWYQQKPGQPPKLLIYYVSHRFT
GVPDRFSGSGSGTDFTLTISSLQAEDVAVYFCHQAYRSPWTFGQGTKLEIKRTVAAPSVFI
FPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
Hu16-HC-IgG4: SEQ ID NO: 134 (HCDR1-HCDR3 are SEQ ID NOs: 40, 41, and 42, respectively) 
EVQLVESGGGLVKPGGSLRLSCAASGFTFSSYHMSWVRQAPGKGLELVSAISRDGD
STYYPDTVKGRFTISRDNAKNSLYLQMNSLRAEDTAVYYCSRHEDYFGFAMDSWGQG
TLVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEFL
GGPSVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTVDK
 SRWQEGNVFSCSVMHEALHNHYTQKSLSLSLGK
Hu16-HC-IgG1: SEQ ID NO: 135 (HCDR1-HCDR3 are SEQ ID NOs: 40, 41, and 42, respectively) 
EVQLVESGGGLVKPGGSLRLSCAASGFTFSSYHMSWVRQAPGKGLELVSAISRDGD
STYYPDTVKGRFTISRDNAKNSLYLQMNSLRAEDTAVYYCSRHEDYFGFAMDSWGQG
TLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPREPQVYTLP
PSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK
Hu52-LC: SEQ ID NO: 136 (LCDR1-LCDR3 are SEQ ID NOs: 52, 53, and 54, respectively) 
DIVMTQSPDSLAVSLGERATINCKASQSVSNDVAWYQQKPGQPPKLLIYYVSHRFT
GVPDRFSGSGSGTDFTLTISSLQAEDVAVYFCHQAYRSPWTFGQGTKLEIKRTVAAPSVFI
FPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
Hu52-HC-IgG4: SEQ ID NO: 137 (HCDR1-HCDR3 are SEQ ID NOs: 67, 68, and 69, respectively) 
EVQLVESGGGLVKPGGSLRLSCAASGFTFSSYHMSWVRQAPGKGLELVSAINSNGI
NTYYLDTVKGRFTISRDNAKNSLYLQMNSLRAEDTAVYYCARHEDYYGFAMDYWGQ
GTLVTVSSASTKGPSVFPLAPCSRSTSESTAALGCLVKDYFPEPVTVSWNSGALTSGVHTF
PAVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTVDK
SRWQEGNVFSCSVMHEALHNHYTQKSLSLSLGK
Hu52-HC-IgG1: SEQ ID NO: 138 (HCDR1-HCDR3 are SEQ ID NOs: 67, 68, and 69, respectively) 
EVQLVESGGGLVKPGGSLRLSCAASGFTFSSYHMSWVRQAPGKGLELVSAINSNGI
NTYYLDTVKGRFTISRDNAKNSLYLQMNSLRAEDTAVYYCARHEDYYGFAMDYWGQ
GTLVTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPPCPA
PELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYT
LPPSRDELTKNQVSLTCLVKGFYPSDIAVEWESNGQPENNYKTTPPVLDSDGSFFLYSKLT
 VDKSRWQQGNVFSCSVMHEALHNHYTQKSLSLSPGK
Hu59-LC: SEQ ID NO: 139 (LCDR1-LCDR3 are SEQ ID NOs: 70, 71, and 72, respectively) 
DIQMTQSPSSLSASVGDRVTITCKASQSVSNDVAWYQQKPGKAPKLLIYYVSNRFTG
VPSRFSGSGSGTDFTLTISSLQPEDFATYFCQQAYRSPWTFGQGTKLEIKRTVAAPSVFIFP
PSDEQLKSGTASVVCLLNNFYPREAKVQWKVDNALQSGNSQESVTEQDSKDSTYSLSSTL
TLSKADYEKHKVYACEVTHQGLSSPVTKSFNRGEC
Hu59-HC-IgG4: SEQ ID NO: 140 (HCDR1-HCDR3 are SEQ ID NOs: 67, 68, and 69, respectively) 
EVQLVESGGGLVQPGGSLRLSCAASGFTFSSYHMSWIRQAPGKGLELVAAINSNGIN
TYYLDTVKGRFTISRDNAKNSLYLQMNSLRAEDTAVYYCARHEDYYGFAMDYWGQGT
LVTVSSASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCPAPEFLG
GPSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKSR
WQEGNVFSCSVMHEALHNHYTQKSLSLSLGK
Hu59-HC-IgG1: SEQ ID NO: 141 (HCDR1-HCDR3 are SEQ ID NOs: 67, 68, and 69, respectively)

EVQLVESGGGLVQPGGSLRLSCAASGFTFSSYHMSWIRQAPGKGLELVAAINSNGIN
TYYLDTVKGRFTISRDNAKNSLYLQMNSLRAEDTAVYYCARHEDYYGFAMDYWGQGT
LVTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCPPCPAPE
LLGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLPP
SRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK

In some embodiments, the amino acid change includes an amino acid deletion, insertion, or substitution. In some embodiments, the anti-CD112R antibodies or the antigen-binding fragments thereof of the present invention include those antibodies having an amino acid sequence that has been mutated by amino acid deletion, insertion or substitution but still has at least about 90%, 91%, 92%, 93%, 94%, 95%, 96%, 97%, 98%, 99%, or 100% identity to amino acid sequences of the antibodies described above (particularly in the CDR regions set forth in the above sequences). In some embodiments, when compared to the CDR regions set forth in a particular sequence, the antibodies of the present invention have no more than 1, 2, 3, 4, or 5 amino acid mutations (deletions, insertions or substitutions) in the CDR regions. In some embodiments, when compared to the frame regions in a particular sequence, the antibodies of the present invention have no more than 1, 2, 3, 4, or 5 amino acid mutations (deletions, insertions or substitutions) in the frame regions.

In some embodiments, polynucleotide molecules encoding the antibodies of the present invention include those that have been mutated by nucleotide deletion, insertion or substitution but still have at least about 60%, 70%, 80%, 90%, 95%, or 100% identity to the corresponding CDR coding regions set forth in the above sequences.

In some embodiments, the CD112R antibodies are IgG antibodies, e.g., IgG1, IgG2, IgG3, or IgG4 antibodies, or modified forms thereof, as described in the section below.

In some embodiments, one or more amino acid modifications may be introduced into an Fc region of an antibody provided herein, thus producing an Fc region variant. The Fc region variant may comprise human Fc region sequences (e.g., human IgG1, IgG2, IgG3, or IgG4 Fc regions) which comprise amino acid modifications (e.g., substitutions) at one or more amino acid positions.

In some embodiments, antibodies modified by cysteine engineering may need to be produced, such as "sulfo-MAb", wherein one or more residues of the antibodies are substituted by cysteine residues.

In some embodiments, the antibodies provided herein can be further modified to contain other non-protein moieties known in the art and readily available. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

### Expression of Antibodies

In yet another aspect, the present invention provides a polynucleotide encoding the anti-CD112R antibody or the antigen-binding fragment thereof of the present invention. The polynucleotide molecule can comprise a polynucleotide molecule encoding an amino acid sequence of the light chain variable region and/or heavy chain variable region of the antibody, or a polynucleotide molecule encoding an amino acid sequence of the light chain and/or heavy chain of the antibody.

In yet another aspect, the present invention provides an expression vector comprising the polynucleotide of the present invention, wherein preferably, the vector is a eukaryotic expression vector. In some embodiments, the polynucleotide molecule of the present invention is comprised in one or more expression vectors.

In yet another aspect, the present invention provides a host cell comprising the polynucleotide molecule of the present invention or the expression vector of the present invention, wherein preferably, the host cell is a eukaryotic cell, and more preferably, the host cell is a mammalian cell.

In yet another aspect, the present invention provides a method for preparing the anti-CD112R antibody or the antigen-binding fragment thereof described herein, comprising expressing the antibody or the antigen-binding fragment thereof in the host cell of the present invention under conditions suitable for expression of the antibody or the antigen-binding fragment thereof, and isolating the expressed antibody or antigen-binding fragment thereof from the host cell.

The present invention provides a mammalian host cell used for expressing the recombinant antibody of the present invention, which includes a number of immortalized cell lines available from American Type Culture Collection (ATCC). Those cell lines include, in particular, Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells and many other cell lines. Mammalian host cells include human, mouse, rat, dog, monkey, pig, goat, cow, horse and hamster cells. Particularly preferred cell lines are selected by determining which cell line has high expression level.

In one embodiment, the present invention provides a method for preparing an anti-CD112R antibody, comprising introducing an expression vector into a mammalian host cell, and culturing the host cell for a period of time sufficient to allow expression of the antibody in the host cell or more preferably to allow secretion of the antibody into a medium in which the host cell is grown, thereby producing the antibody. The antibody can be isolated from the medium using standard protein purification methods.

It is likely that antibodies expressed by different cell lines or in transgenic animals have different glycosylations from each other. However, all antibodies encoded by the nucleic acid molecules provided herein or comprising the amino acid sequences provided herein are integral parts of the present invention, regardless of the glycosylation of the antibody. Likewise, in certain embodiments, nonfucosylated antibodies are advantageous because they generally have more potent efficacy *in vitro* and *in vivo* than their fucosylated counterparts, and are unlikely to be immunogenic because their glycan structures are normal components of natural human serum IgG.

### Pharmaceutical Composition and Pharmaceutical Formulation

In yet another aspect, the present invention provides a pharmaceutical composition comprising the anti-CD112R antibody or the antigen-binding fragment thereof described herein, the polynucleotide molecule described herein, the expression vector described herein, or the host cell described herein, and a pharmaceutically acceptable carrier or excipient. It should be understood that the anti-CD112R antibody or the pharmaceutical composition thereof provided herein can be integrated into a suitable carrier, an excipient and other reagents in a formulation for administration in combination, thus providing improved transfer, delivery, tolerance, etc.

The term "pharmaceutical composition" refers to a formulation that allows the biological activity of active ingredients comprised therein to be present in an effective form and does not comprise additional ingredients having toxicity unacceptable to a subject to which the formulation is administered.

The pharmaceutical formulation comprising the anti-CD112R antibody described herein, preferably in the form of an aqueous solution or a lyophilized formulation, may be prepared by mixing the anti-CD112R antibody of the present invention having the desired purity with one or more optional pharmaceutical excipients (Remington's Pharmaceutical Sciences, 16th edition, Osol, A. Ed. (1980)).

The pharmaceutical composition or formulation of the present invention can further comprise one or more additional active ingredients which are required for a specific indication being treated, preferably active ingredients having complementary activities that do not adversely affect one another. In some embodiments, the additional active ingredients are chemotherapeutic agents, immune checkpoint inhibitors, growth inhibitors, antibiotics or various known anti-tumor or anti-cancer agents, which are suitably present in combination in amounts that are effective for purpose intended. In some embodiments, the pharmaceutical composition of the present invention also comprises a composition of a polynucleotide molecule encoding the anti-CD112R antibody.

In yet another aspect, the present invention provides a drug combination comprising the antibody or the antigen-binding fragment thereof described herein, the polynucleotide molecule described herein, the expression vector described herein, the host cell described herein, or the pharmaceutical composition described herein, and one or more additional therapeutic agents.

In one embodiment, in the drug combination described herein, the additional therapeutic agent is selected from one or more of an anti-PD-1 antibody, an anti-PD-L1 antibody, and an anti-TIGIT antibody; preferably, the additional therapeutic agent is an anti-TIGIT antibody, or a combination of an anti-TIGIT antibody and an anti-PD-1 antibody.

In one embodiment, the anti-PD-1 antibody described herein is selected from one or more of nivolumab, pembrolizumab, toriplalimab, sintilimab, camrelizumab, tislelizumab and cemipilimab; preferably toriplalimab.

In one embodiment, the anti-PD-L1 antibody described herein is selected from atezolizumab, avelumab and durvalumab.

In one embodiment, the embodiments of the anti-TIGIT antibody of the present invention are set forth in PCT/CN2020/101883. The anti-TIGIT antibody described herein includes any one of the anti-TIGIT antibodies described in PCT/CN2020/101883, preferably humanized antibody hu20.

In yet another aspect, the present invention provides a kit comprising the antibody or the antigen-binding fragment thereof described herein, the polynucleotide molecule described herein, the expression vector described herein, the host cell described herein, or the pharmaceutical composition described herein.

### Pharmaceutical Use and Treatment Method

Any of the anti-CD112R antibodies provided herein can be used in a treatment method. It should also be understood that when discussing "antibodies", compositions comprising antibodies are also included. The anti-CD112R antibodies of the present invention may be used in a therapeutically or prophylactically effective amount for any treatment or prophylactic method described in any one of the embodiments of the present invention.

In yet another aspect, the present invention provides use of the antibody or the antigen-binding fragment thereof described herein, the polynucleotide molecule described herein, the expression vector described herein, the host cell described herein, or the pharmaceutical composition described herein in preparing a medicament for treating and/or preventing a CD112R-mediated disease or disorder, wherein preferably, the disease or disorder is a cancer, and more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer. In some embodiments, the medicament is used for enhancing, increasing, and/or maintaining an anti-tumor immune response in a subject with a tumor. In some embodiments, the medicament is used for enhancing the interaction of CD112 with CD226.

In yet another aspect, the present invention provides the antibody or the antigen-binding fragment thereof described herein, the polynucleotide molecule described herein, the expression vector described herein, the host cell described herein, or the pharmaceutical composition described herein for use in the treatment and/or prevention of a CD112R-mediated disease or disorder, wherein preferably, the disease or disorder is a cancer, and more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer. In some embodiments, the present invention provides an anti-CD112R antibody for use in enhancing, increasing, and/or maintaining an anti-tumor immune response in a subject with a tumor. In some embodiments, the present invention provides an anti-CD112R antibody for use in enhancing the interaction of CD226 with CD112.

In yet another aspect, the present invention provides a method for treating and/or preventing a CD112R-mediated disease or disorder, comprising administering to a subject in need the antibody or the antigen-binding fragment thereof described herein, the polynucleotide molecule described herein, the expression vector described herein, the host cell described herein, or the pharmaceutical composition or drug combination described herein, wherein preferably, the disease or disorder is a cancer, and more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer. In some embodiments, the present invention provides a method for treating a disease and/or a disorder requiring blockade of CD112R, comprising administering to a subject in need the anti-CD112R antibody thereof of the present invention. In some embodiments, provided is a method for enhancing, increasing, and/or maintaining an anti-tumor immune response in a subject with a tumor, comprising administering to a subject in need the anti-CD112R antibody described herein.

In some embodiments, the cancer or tumor described herein can be selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

In some embodiments, the routes of administration of the present invention include, but are not limited to, oral administration, intravenous administration, subcutaneous administration, intramuscular administration, intra-arterial administration, intra-articular administration (e.g., in arthritic joints), administration by inhalation or aerosol delivery, intratumoral administration, and the like.

The present invention also provides co-administration of a therapeutically effective amount of one or more therapies (e.g., treatment modalities and/or additional therapeutic agents) to a subject. In some embodiments, the therapies include surgical treatment and/or radiation therapy. The antibody of the present invention can be used alone or used in combination with additional therapeutic agents in a therapy. In some embodiments, the antibody of the present invention is co-administered with at least one additional therapeutic agent, for example, one or more of an anti-PD-1 antibody, an anti-PD-L1 antibody, and an anti-TIGIT antibody. In some embodiments, the antibody of the present invention is administered in combination with the anti-TIGIT antibody. In some embodiments, the antibody of the present invention is administered in combination with the anti-PD-1 antibody and the anti-TIGIT antibody.

### Methods for Diagnosis and Detection

In yet another aspect, the present invention provides a method for detecting the presence of CD112R in a sample using the antibody or the antigen-binding fragment thereof described herein. The term "detection" as used herein includes quantitative or qualitative detection. In some embodiments, the sample is a biological sample. In certain embodiments, the biological sample is blood, serum, or other liquid samples of biological origin. In certain embodiments, the biological sample includes cells or tissues. The method comprises the steps of contacting the antibody or the antigen-binding fragment thereof described herein or a detection composition comprising the antibody or the antigen-binding fragment thereof with the sample, and detecting the presence or absence of a conjugate or a binding signal produced by the binding of the antibody or the antigen-binding fragment thereof to CD112R. For a detection purpose, the antibody or the antigen-binding fragment thereof described herein may be labeled to indicate whether the conjugate is formed.

The present invention includes all combinations of the specific embodiments. Further embodiments of the present invention and the full scope of applicability will become apparent from the detailed description provided below. However, it should be understood that the detailed description and the specific examples, while indicating preferred embodiments of the present invention, are provided by way of illustration only, as various changes and modifications within the spirit and scope of the present invention will become apparent to those skilled in the art from the detailed description. All publications, patents and patent applications cited herein, including the citations, are hereby incorporated by reference in their entirety for all purposes.

The compounds of the present invention can be prepared using a variety of synthesis methods well known to those skilled in the art, including the specific embodiments listed below, embodiments formed by combinations thereof with other methods, and equivalents well known to those skilled in the art; preferred embodiments include, but are not limited to, the examples of the present invention.

The following abbreviations are used in the present invention:
His-tag for a histidine tag;
Fc tag for a crystallizable fragment tag;
ECD for an extracellular domain;
PEI for polyethyleneimine;
BSA for bovine serum albumin;
PBS for phosphate buffered saline;
FBS for fetal bovine serum;
CFSE for carboxyfluorescein diacetate succinimidyl ester;
APC for allophycocyanin;
NA-PE for phycoerythrin-labeled neutral avidin;
PE for phycoerythrin;
TMB for 3,3',5,5'-tetramethylbenzidine;
HEPES: hydroxyethylpiperazine ethanethiosulfonic acid buffer;
DTT for dithiothreitol.

### EXAMPLE

The present invention is illustrated by the following examples, which, however, are not intended to be limiting in any way. The present invention has been described in detail, and the specific embodiments are also disclosed. It will be apparent to those skilled in the art that various changes and modifications can be made to the specific embodiments of the present invention without departing from the spirit and scope of the present invention.

### Example 1. Preparation of Recombinant Protein for Assay on Activity of Anti-CD112R Antibody

A cDNA sequence encoding a CD112R extracellular domain was amplified by a conventional PCR technique, and an amplified fragment was cloned into a eukaryotic expression plasmid system (MX2-mFc, containing a murine IgG2a Fc domain) constructed in-house by employing a conventional cloning technique, wherein the eukaryotic expression plasmid system contained a puromycin screening system, so that a recombinant fusion protein expression plasmid hCD112R ECD mFC was produced. The expression and purification were performed using an expression cell 293E to obtain an hCD 112R mFC recombinant protein.

The amino acid sequence of the extracellular domain of human CD112R (hCD112R ECD) comprises amino acids at positions Met1-Ala160 amino acid under NCBI accession No. NM_024070, and was used for detecting the activity of the antibody of the present invention.

### Example 2. Preparation of Mouse Hybridoma Cells

### 2.1 Immunization of animals

Five BALB/c mice (purchased from Simonsen laboratories of Gilroy, female BALB/c, 8 weeks) were immunized with a hCD112R-Fc (purchased from R&D Systems, Cat No. 9365-PV) recombinant protein as an antigen. After primary immunization (50 µg/mouse), booster immunization (25 µg/mouse) was performed once every week or every 2 weeks for 6 immunizations in total.

### 2.2 Cell fusion

On the fourth day after the last booster immunization, the inguinal lymph nodes, the popliteal lymph nodes and the spleens of the mice were collected and ground in normal saline, and then the suspension rich in lymphocytes was collected and fused with mouse myeloma cell Sp2/0 using the conventional electrotransfection method. The fusion product was cultured in a DMEM complete medium containing 1:50 HAT (hypoxanthine, amethopterin and thymidine) for 5 days to screen for successful fusion cells (hybridoma cells). Then the medium was changed to a DMEM complete medium containing 1:50 HT (hypoxanthine and thymidine), and the culture continued until the end of the screening.

The formula of the DMEM complete medium was as follows: 15% FBS (fetal bovine serum) + 1:50 L-Glutamine + 100 U/mL penicillin-streptomycin + 1:100 OPI (oxaloacetic acid, pyruvic acid and insulin). The incubator conditions were 8% CO₂, 37 °C.

### Example 3. Screening of Mouse Hybridoma Cells and Performance Tests of the Obtained Anti-CD112R Murine Antibodies

Among 9600 different polyclonal hybridoma cell strains, 181 strains were selected by binding ELISA, which secreted antibodies that may specifically bind to hCD112R. Among the 181 binding-positive hybridoma cell strains, 40 strains expressed antibodies that may block the binding of hCD112R to hCD112 expressed on the cell surface in FACS. In a subsequent blocking experiment by ELISA, it was determined that 22 of the 40 polyclonal cell strains described above were capable of blocking the binding of hCD112R to hCD112. Fifteen of these 22 polyclonal hybridoma cell strains that were double positive for FACS and ELISA blocking were selected for subcloning.

Fifteen blocking monoclonal cell strains were obtained by screening subclones. The secreted antibodies therefrom were purified and analyzed. mRNA was extracted from monoclonal hybridoma cells and antibody variable regions were sequenced. Through *in vitro* pharmacodynamic experiments and the combination with the PD-1 monoclonal antibody and the TIGIT monoclonal antibody, 8 candidate monoclonal hybridoma strains capable of promoting T cell activation were identified for the final recombinant expression, with the serial numbers of 2G10.1, 3D2.1, 4E2, 4E5.1, 5B2.1, 5C10.1, 6A10.1 and 7C2.1, respectively, which were abbreviated as 2G10, 3D2, 4E2, 4E5, 5B2, 5C10, 6A10 and 7C2, respectively, in subsequent chimeric antibody construction.

The experimental methods are shown below.

### 3.1 Assay on the blocking of the binding of hCD112R to hCD112 by hybridoma antibodies by ELISA

A 96-well MaxiSorp plate (Thermo Fisher Nunc) was coated with 2 µg/mL hCD112 (murine Fc-tag, prepared by Junmeng) and blocked with 1% BSA. hCD112R at a final concentration of 0.5 µg/mL was co-incubated with a hybridoma culture supernatant on a microplate for 30 min. The mixture was then transferred to a MaxiSorp plate and incubated for 30 min. Horseradish peroxidase-labeled goat anti-human secondary antibody was added for the assay.

The results showed that the hybridoma antibodies of the present invention were capable of effectively blocking the binding of hCD112R to hCD112.

### 3.2 Assay on the affinity of anti-CD112R hybridoma antibodies for hCD112R mFc by Biacore

Biacore T200 was used to assay the binding affinity of the anti-CD112R hybridoma antibodies for the recombinant hCD112R mFc protein. Goat anti-human Fc antibody fragments (Jackson ImmunoResearch) were coupled to the surface of a CM5 chip, and the anti-CD112R hybridoma antibodies were each captured by using the chip, after which recombinant hCD112R mFc proteins at different concentrations were injected into the CM5 chip. As shown in Table 4, 15 anti-CD112R hybridoma antibodies all had higher affinity for recombinant hCD112R mF.

**Table 4: The affinity of the anti-CD112R hybridoma antibodies for recombinant hCD112R mFc**

| Number | Affinity KD (M) | Number | Affinity KD (M) |
|---|---|---|---|
| 1D11.1 | 2.36E-11 | 6A6.1 | 1.74E-11 |
| 2G10.1 | 3.02E-11 | 6A10.1 | 1.03E-11 |
| 3D2.1 | 3.36E-11 | 6C6.1 | 2.05E-11 |
| 4E2 | 2.37E-11 | 6D2.1 | 1.34E-11 |
| 4E5.1 | 9.26E-12 | 7C2.1 | 1.01E-11 |
| 5B1.1 | 1.63E-11 | 8D3.1 | 1.58E-11 |
| 5B2.1 | 2.32E-11 | 8H8.1 | 5.88E-11 |
| 5C10.1 | 8.55E-12 | | |

### 3.3 Assay on the blocking of the binding of hCD112R to hCD112 on the cell surface by hybridoma antibodies by FACS

Cells CHO CD112 overexpressing human CD112 constructed in-house, were co-incubated with a hCD112R mFc protein solution (0.5 µg/mL) prepared in a staining buffer (PBS + 1% FBS), and the anti-CD112R hybridoma antibodies or the control antibodies at different concentrations (initial concentration: 100 µg/mL, 3-fold dilution, 10 concentration gradients) at 4 °C for 30 min. Unbound antibodies were then washed with a staining buffer and the cells were incubated with 1% (v/v) goat anti-mouse IgG Fc fluorescent secondary antibodies (Jackson ImmunoResearch) prepared in a staining buffer at 4 °C for 30 min in the dark. Finally, the cells were collected by using a flow cytometer, and the fluorescent antibodies bound to the cell surface were detected. Raw data were analyzed by using FlowJo to obtain MFI values, and a four-parameter curve fitting was performed by using GraphPad Prism software to obtain IC₅₀ values.

The results are shown in Table 5, which showed that the hybridoma antibodies of the present invention were capable of effectively blocking the binding of hCD1 12R to hCD112.

**Table 5: Blocking of the binding of hCD 1 12R to hCD 112 by the anti-CD112R hybridoma antibodies**

| Number | IC₅₀ (ng/mL) | Number | IC₅₀ (ng/mL) |
|---|---|---|---|
| 1D11.1 | 207.1 | 6A6.1 | 145.1 |
| 2G10.1 | 260.5 | 6A10.1 | 120.1 |
| 3D2.1 | 44.9 | 6C6.1 | 22.9 |
| 4E2 | 4581.0 | 6D2.1 | 272.4 |
| 4E5.1 | 110.5 | 7C2.1 | 266.8 |
| 5B1.1 | 146.8 | 8D3.1 | 147.9 |
| 5C10.1 | 84.3 | | |

### 3.4 Assay on the activity of hybridoma antibodies in a CD112R/CD112 reporter gene assay system

Target cells CHO CD112 (stably expressing human CD112 and CD3scFv) constructed in-house were placed into a 96-well flat-bottom white plate overnight at a cell density of 5 × 10⁴ cells/well. After the medium in the cell wells was pipetted the next day, the anti-CD112R hybridoma antibodies or the control antibodies at different concentrations (initial concentration: 30 µg/mL, 3-fold dilution, 8 concentration gradients in total) were added to the target cells with an experimental buffer (RPMI 1640 (1×) (purchased from Gibco, Cat No. 22400-071) + 2% FBS) and incubated at 37 °C for 30 min. Effector cells Jurkat CD112R (stably expressing human CD112R and luc2P/NFAT-RE) constructed in-house were then added to the cell plate at a cell density of 1 × 10⁵ cells/well and co-incubated at 37 °C for 6 h in an incubator. Finally, a ONE-Glo luciferase assay reagent (Promega) was added to a mixed system of the cells and the antibodies, and a chemiluminescence signal was detected by using a multi-mode microplate reader. A four-parameter regression curve was fitted by using GraphPad Prism software and EC₅₀ values were calculated. The positive control was Ref (anti-CD112R antibody, University of Colorado, obtained by expression and purification according to the sequences in US20170240613).

The results showed that the hybridoma antibodies of the present invention were capable of effectively blocking the interaction of CD112R with CD112 in a CD112R/CD112 reporter gene system and promoting the activation of T cells.

The EC₅₀ values of the hybridoma antibodies 2G10.1, 5C10.1, 3D2.1 and 4E2 were 72.91 ng/mL, 40.36 ng/mL, 49.28 ng/mL and 61.26 ng/mL, respectively, which were significantly superior to that of the control antibody Ref (920.4 ng/mL); the EC₅₀ values of 7C2.1 and 6A10.1 were 37.94 ng/mL and 50.73 ng/mL, respectively, which were significantly superior to that of the control antibody Ref (870.9 ng/mL); the EC₅₀ value of 4E5.1 was 55.19 ng/mL, which was significantly superior to that of the control antibody Ref (866.3 ng/mL).

### 3.5 Assay on the activity of hybridoma antibodies in combination with an anti-TIGIT antibody in a dual reporter gene system.

Target cells CHO CD112 (stably expressing hCD112 and CD3scFv) were placed into a 96-well flat-bottom white plate (Corning, Cat No. 3917) overnight at a cell density of 5 × 10⁴ cells/well, and then the anti-CD112R hybridoma antibody at a fixed concentration (0.3 µg/mL) and the anti-TIGIT antibodies at different concentrations (from 0.5 ng/mL to 30 µg/mL, 3-fold dilution) were added and incubated at 37 °C for 30 min. Effector cells Jurkat PD-1 TIGTI CD112R (stably expressing human PD-1, human TIGIT, human CD112R and luc2P/NFAT-RE) were then added to the target cells at a cell density of 1 × 10⁵ cells/well and co-incubated at 37 °C for 4-6 h in an incubator. Finally, a ONE-Glo luciferase assay reagent (Promega, Cat No. E6120) was added to a mixed system of the cells and the antibodies, and a chemiluminescence signal was detected by using a multi-mode microplate reader.

Anti-TIGIT antibody (JS006): derived from the anti-TIGIT humanized antibody hu20 in PCT application No. PCT/CN2020/101883.

Hybridoma antibodies: 2G10.1, 3D2.1, 4E2, 4E5.1, 5B2.1, 5C10.1, 6A10.1 and 7C2.1.

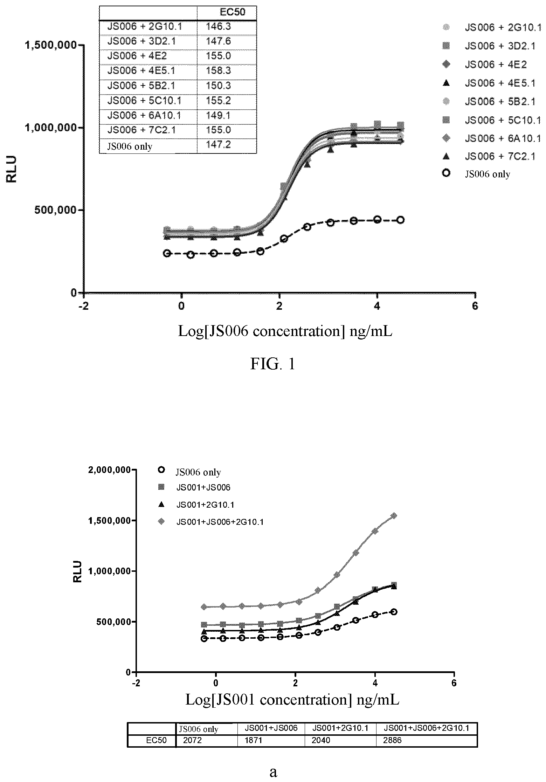

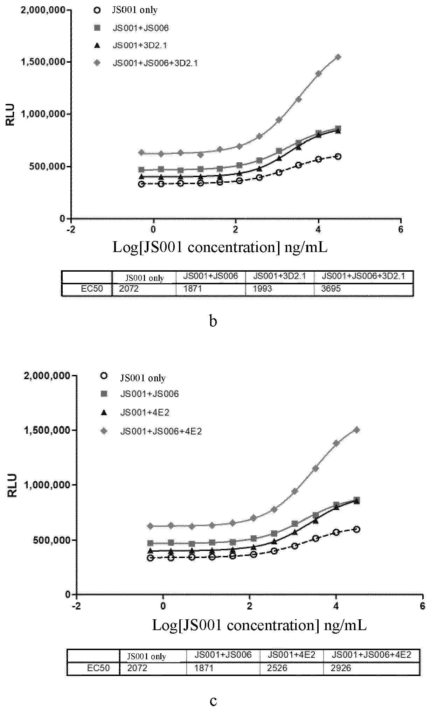

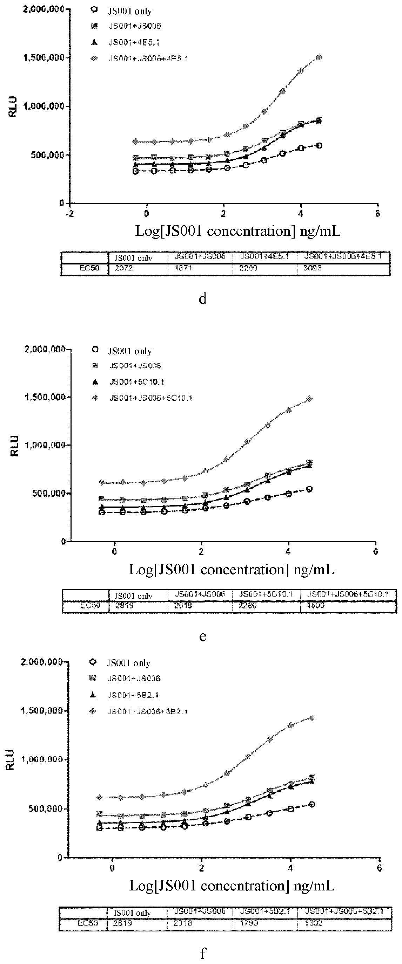

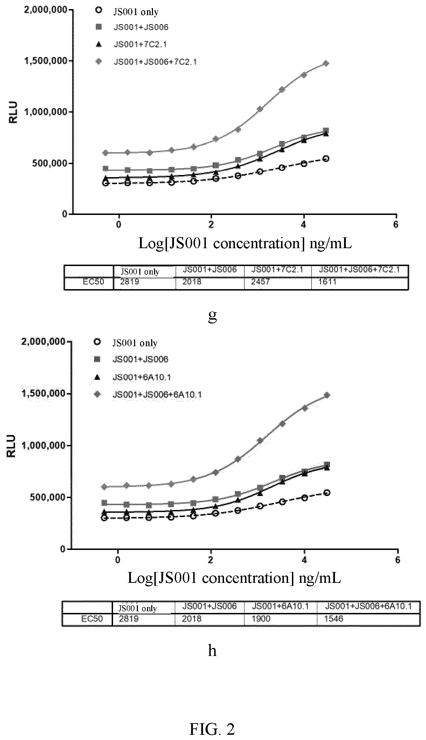

As shown in the dose-dependent curves of FIG. 1, in the dual reporter gene system, after the hybridoma antibodies of the present invention are used in combination with the anti-TIGIT antibody, the luciferase reporter gene NFAT signal of the combination was significantly higher than that of the anti-TIGIT antibody monotherapy group, which indicated that the combination of the anti-CD112R antibodies and the anti-TIGIT antibody had a significant synergistic effect.

### 3.6 Assay on the activity of hybridoma antibodies in combination with an anti-TIGIT antibody and an anti-PD-1 antibody in a triple reporter gene system

Target cells CHO PD-L1 CD112 (stably expressing hPD-L1, hCD112 and CD3scFv) were placed into a 96-well flat-bottom white plate (Corning, Cat No. 3917) overnight at a cell density of 5 × 10⁴ cells/well, and then the anti-PD-1 antibodies at different concentrations (from 0.5 ng/mL to 10 µg/mL, 3-fold dilution) and the anti-TIGIT antibody at a fixed concentration (0.3 µg/mL) and the anti-CD112R hybridoma antibody at a fixed concentration (0.3 µg/mL) were added and incubated at 37 °C for 30 min. Effector cells Jurkat PD-1 TIGTI CD112R (stably expressing human PD-1, human TIGIT, human CD112R and luc2P/NFAT-RE) were then added to the target cells at a cell density of 1 × 10⁵ cells/well and co-incubated at 37 °C for 4-6 h in an incubator. Finally, a ONE-Glo luciferase assay reagent (Promega, Cat No. E6120) was added to a mixed system of the cells and the antibodies, and a chemiluminescence signal was detected by using a multi-mode microplate reader.

Anti-TIGIT antibody (JS006): derived from the anti-TIGIT humanized antibody hu20 in PCT application No. PCT/CN2020/101883.

Anti-PD-1 antibody (JS001): toripalimab derived from Junshi Biosciences.

Hybridoma antibodies: 2G10.1, 3D2.1, 4E2, 4E5.1, 5B2.1, 5C10.1, 6A10.1 and 7C2.1.

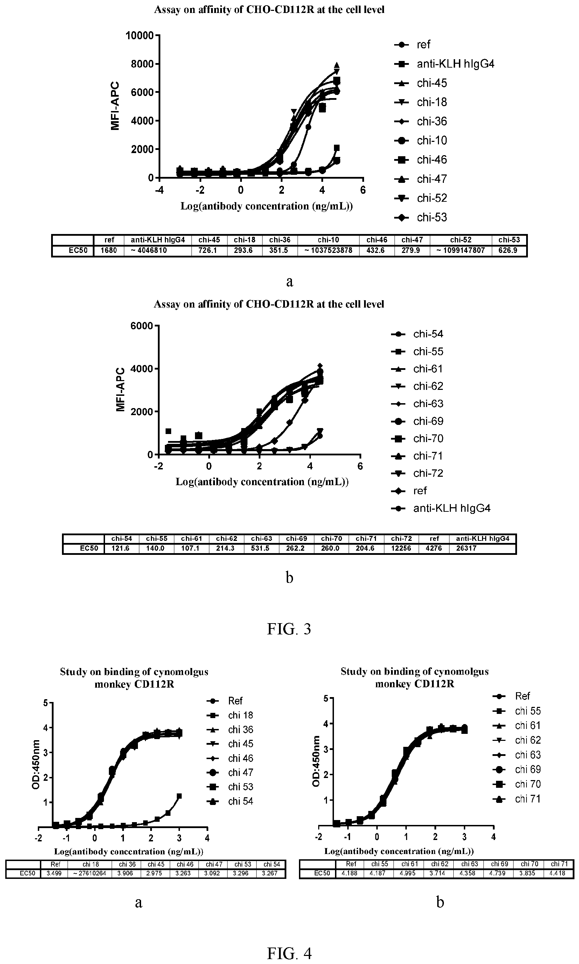

As shown in FIG. 2 (a-h), the assay results showed that the luciferase reporter gene NFAT signal of the combination group of the anti-CD112R hybridoma monoclonal antibody, the anti-TIGIT antibody and the anti-PD-1 antibody was significantly higher than that of the anti-PD-1 antibody monotherapy group and the combination group of the anti-PD-1 antibody and the anti-CD112R hybridoma monoclonal antibody at 0.3 µg/mL, which indicated that the combination of the anti-TIGIT antibody, the anti-PD-1 antibody and the anti-CD112R antibody had a significant synergistic effect.

### Example 4. Sequencing of Variable Regions of Anti-CD112R Murine Antibodies (Shown According to the Kabat or IMGT Scheme)

The DNA coding sequences corresponding to the variable regions of the anti-CD112R murine antibody were determined by using a degenerate primer PCR-based method. Candidate hybridoma cells were cultured and collected by centrifugation at 1000 rpm, and total RNA was extracted with Trizol. A first-strand cDNA was synthesized by using the total RNA as a template, and then the DNA coding sequences corresponding to the variable regions were amplified by PCR by using the first-strand cDNA as a subsequent template. The primer sequences used in the amplification reaction were complementary to the first framework regions of the variable regions and the constant regions of the antibody (Larrick, J.W. et al., 1990, Scand. J. Immunol., 32, 121-128 and Coloma, J.J. et al., (1991) BioTechniques, 11, 152-156). 1 µL of cDNA, 5 µL of 10× PCR buffer, 1 µL (25 pmoL) of each of the upstream and downstream primers, 1 µL of dNTP, 1 µL of 25 mmol PL MgClz, and 39 µL of H₂O were added to a 50 µL reaction system, pre-denatured at 95°C for 10 min, and subjected to temperature cycling for PCR amplification after adding 1 µL of Taq enzyme. The reaction conditions were denaturation at 94 °C for 1 min, annealing at 58 °C for 1 min, and extension at 72 °C for 15 s, for 32 cycles in total, followed by incubation at 72 °C for 10 min. PCR products were recovered and purified. The products of the amplification were sequenced to obtain the amino acid sequences of the heavy chain variable region and the light chain variable region of the anti-human CD112R murine antibody.

The NCBI Ig-Blast (http://www.ncbi.nlm.nih.gov/projects/igblast/) was used to search for consensus sequences in germline and rearranged Ig variable region sequence databases. The amino acid sequences of complementarity-determining regions (CDRs) were identified by sequence annotation and by Internet-based sequence analysis (http://www.imgt.org/IMGT_vquest/vquest and http://www.ncbi.nlm.nih.gov/igblast/), based on Kabat system (Wu, T. T and Kabat, E. A. 1970 J. Exp. Med., 132:211-250) and IMGT system (Lefranc M.-P. et al., 1999 Nucleic Acids Research, 27, 209-212).

The amino acid sequences of the light chain variable region and the heavy chain variable region and CDRs of the selected anti-CD112R murine antibodies were shown in Table 1. It was found by PCR sequencing analysis that the hybridoma cell 4E2 contained two heavy chains containing heavy chain variable regions 4E2-VH1 and 4E2-VH2, respectively.

### Example 5. Construction of Chimeric Anti-CD112R Antibodies

The light chain variable region and the heavy chain variable region of anti-human CD112R murine antibodies 2G10, 4E2HC1, 4E2HC2, 4E5HC, 5B2, 6A10, 7C2, 5C10 and 3D2 were selected to construct chimeric anti-CD112R antibodies, based on the expression amount, activity, type, and the like, of the antibodies expressed by the hybridoma cells.

The coding sequences for the heavy chain constant region Fc and the light chain constant region κ were cloned from human blood cells (from Beijing Blood Institute) and introduced into the pCDNA3.1 plasmid. The coding sequences for the heavy chain variable region and the light chain variable region of the anti-human CD112R murine antibodies described above were synthesized by Genscript. The coding sequences for the heavy chain variable regions and the light chain variable regions of various anti-human CD112R murine antibodies were digested with enzyme Bspq I. Then the digested sequences were introduced, in various combinations as shown in Table 2, into the pCDNA3.1 plasmid into which the coding sequences for the constant regions had been introduced, and correct clones were determined by sequencing. Various chimeric heavy and light chain expression plasmids were mixed and paired, and used to transfect expression cells to obtain 72 chimeric anti-human CD112R antibodies. The numbers and pairing modes of the antibodies are shown in detail in Table 2. All subsequent experimental materials were given by the cells transfected with these plasmids.

### Example 6. Screening of Chimeric Antibodies

### 6.1 The binding of chimeric anti-CD112R antibodies to human CHO-CD112R-expressing cells

CHO-CD112R cells (constructed in-house by Junshi, the amino acid sequence of CD112R in the construction of stable cell lines overexpressing CD112R comprises amino acids at positions Met1-Arg326 under NCBI accession No. Q6DKI7) overexpressing human CD112R were incubated with each of the chimeric anti-CD112R antibodies at different concentrations (initial concentration: 50 µg/mL, 5-fold dilution, 12 concentration gradients in total) at 4 °C for 30 min, and then washed and incubated with a fluorescence-labeled secondary antibody at 4 °C for 30 min in the dark. Finally, the cells were collected by using a flow cytometer (BD Canto II), and the fluorescent antibodies bound to the cell surface were detected. Raw data were analyzed by using FlowJo to obtain MFI values, the antibody dose-dependent binding curves were fitted by using GraphPad (FIG. 3, a and b) and EC₅₀ was calculated (Table 3). The positive control and the negative control were Ref (anti-CD112R antibody, University of Colorado, obtained by expression and purification according to the sequences in US20170240613) and anti-KLH hu-IgG4 antibody, respectively.

As shown in FIG. 3 and Table 6, most of the chimeric anti-CD112R antibodies of the present invention were capable of binding to human CD112R highly expressed on the surface of CHO-CD112R cells with high affinity, and the binding activity of the chimeric anti-CD112R antibodies was significantly better than that of the control antibody Ref.

**Table 6: The affinity of the chimeric anti-CD112R antibodies at the cell level (EC₅₀)**

| Chimeric antibody | EC₅₀(ng/ml) | Relative activity (%) * |
|---|---|---|
| chi-61 | 107.1 | 3993 |
| chi-54 | 121.6 | 3516 |
| chi-55 | 140 | 3054 |
| chi-71 | 204.6 | 2090 |
| chi-62 | 214.3 | 1995 |
| chi-70 | 260 | 1645 |
| chi-69 | 262.2 | 1631 |
| chi-63 | 531.5 | 805 |
| chi-47 | 279.9 | 600 |
| chi-18 | 293.6 | 572 |
| chi-36 | 351.5 | 478 |
| chi-46 | 432.6 | 388 |
| chi-53 | 626.9 | 268 |
| chi-45 | 726.1 | 231 |
| ref | 4276 | 100 |
| chi-72 | 12256 | 35 |
| chi-10 | NA | NA |
| chi-52 | NA | NA |
| anti-KLH hIgG4 | NA | NA |

| | | |
|---|---|---|
| Note: "*" indicated the EC₅₀ ratio of the control antibody Ref to the chimeric anti-CD112R antibody; "NA" indicates not detected. | | |

### 6.2 Binding of chimeric anti-CD112R antibodies to cynomolgus monkey CD112R

A plate was coated with a recombinant cynomolgus monkey CD112R antigen (Acrobiosystems, Cat No. PVG-C5259) at a fixed concentration, and the chimeric anti-CD112R antibodies diluted in a gradient (initial concentration: 1 µg/mL, 2.5-fold gradient dilution, 12 concentration gradients in total) were added to bind to the antigen. Horseradish peroxidase (HRP)-conjugated mouse anti-human IgG4 Fc (Southern Biotech, 9200-05) was subjected to a 5000-fold dilution and used as a detection antibody for detection. The color development was performed with 0.1 mg/mL TMB, and finally stopped with 2 M hydrochloric acid. The plate was read at 420 nm/620 nm. EC₅₀ was obtained by fitting with a four-parameter logistic regression (4PL) model.

The positive control was Ref (anti-CD112R antibody, University of Colorado, obtained by expression and purification according to the sequences in US20170240613).

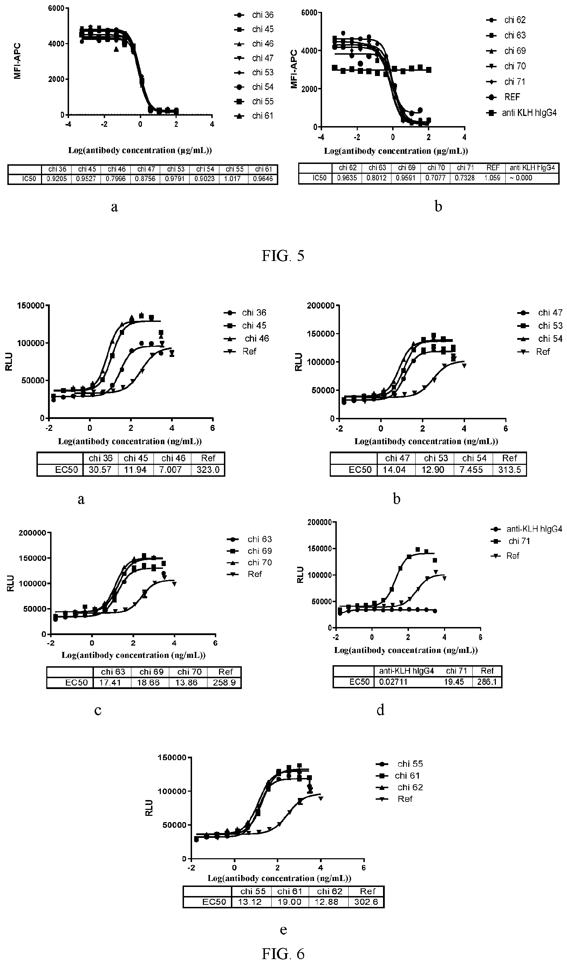

As shown in FIG. 4 (a and b) and Table 7, among the following 14 chimeric antibodies, except for chi 18, which did not bind to the cynomolgus monkey CD112R, the other 13 chimeric anti-CD112R antibodies all bound to the cynomolgus monkey CD112R.

**Table 7: The binding of the chimeric anti-CD112R antibodies to the cynomolgus monkey CD112R (EC₅₀ ng/mL)**

| Chimeric antibody | EC₅₀(ng/ml) | Relative activity (%) * |
|---|---|---|
| chi 18 | NA | NA |
| chi 36 | 3.906 | 89.6 |
| chi 45 | 2.975 | 117.6 |
| chi 46 | 3.263 | 107.2 |
| chi 47 | 3.092 | 113.2 |
| chi 53 | 3.296 | 106.2 |
| chi 54 | 3.267 | 107.1 |
| chi 55 | 4.187 | 100 |
| chi 61 | 4.995 | 83.8 |
| chi 62 | 3.714 | 112.8 |
| chi 63 | 4.358 | 96.1 |
| chi 69 | 4.739 | 88.4 |
| chi 70 | 3.835 | 109.2 |
| chi 71 | 4.418 | 94.8 |

| | | |
|---|---|---|
| Note: "*" indicated the EC₅₀ ratio of the control antibody Ref to the chimeric anti-CD112R antibody. | | |

### 6.3 Blocking of the binding of a CD112R protein to human CHO-CD112-expressing cells by chimeric anti-CD112R antibodies

Cells CHO CD112 overexpressing human CD112 were co-incubated with a hCD112R mFc protein solution (5 µg/mL)prepared in a staining buffer (PBS + 1% FBS), and the chimeric anti-CD112R antibodies or the control antibodies at different concentrations (initial concentration: 100 µg/mL, 3-fold dilution, 10 concentration gradients) at 4 °C for 30 min. Unbound antibodies were then washed with a staining buffer and the cells were incubated with 1% (v/v) goat anti-mouse IgG Fc fluorescent secondary antibodies (Jackson ImmunoResearch) prepared in a staining buffer at 4 °C for 30 min in the dark. Finally, the cells were collected by using a flow cytometer (BD Canto II), and the fluorescent antibodies bound to the cell surface were detected. Raw data were analyzed by using FlowJo to obtain MFI values, and a four-parameter curve fitting was performed by using GraphPad Prism software to obtain IC₅₀ values. The positive control and the negative control were Ref (anti-CD112R antibody, University of Colorado, obtained by expression and purification according to the sequences in US20170240613) and the anti-KLH hu-IgG4 antibody, respectively.

As shown in FIG. 5 (a and b) and Table 8, the following 13 chimeric antibodies were all capable of effectively blocking the binding of the CD112R protein to CHO-CD 112 cells.

**Table 8: The ability of the chimeric anti-CD112R antibodies to block the binding of the CD112R protein to CHO-CD112 cells (IC₅₀)**

| Chimeric antibody | IC₅₀ (ng/ml) | Relative activity (%) * |
|---|---|---|
| chi 70 | 0.7077 | 149.6 |
| chi 71 | 0.7328 | 144.5 |
| chi 46 | 0.7996 | 132.4 |
| chi 63 | 0.8012 | 132.2 |
| chi 47 | 0.8756 | 120.9 |
| chi 54 | 0.9023 | 117.4 |
| chi 36 | 0.9205 | 115.0 |
| chi 45 | 0.9527 | 111.2 |
| chi 69 | 0.9591 | 110.4 |
| chi 62 | 0.9635 | 109.9 |
| chi 61 | 0.9646 | 109.8 |
| chi 53 | 0.9791 | 108.2 |
| chi 55 | 1.017 | 104.1 |
| Ref | 1.059 | 100.0 |

| | | |
|---|---|---|
| Note: "*" indicated the EC₅₀ ratio of the control antibody Ref to the chimeric anti-CD112R antibody. | | |

### 6.4 Assay on the activity of chimeric anti-CD112R antibodies in a CD112R/CD112 reporter gene assay system

Target cells CHO CD112 (constructed in-house by Junshi Biosciences, stably expressing human CD112 and CD3scFv) cell were placed into a 96-well flat-bottom white plate overnight at a cell density of 5 × 10⁴ cells/well. After the medium in the cell wells was pipetted the next day, the chimeric anti-CD112R antibodies or the control antibodies at different concentrations (initial concentration: 3 µg/mL, 3-fold dilution, 10 concentration gradients in total) were added to the target cells with an experimental buffer (RPMI 1640 (1×) (purchased from Gibco, Cat No. 22400-071) + 2% FBS) and incubated at 37 °C for 30 min. Effector cells Jurkat CD112R (stably expressing human CD112R and luc2P/NFAT-RE) constructed in-house were then added to the cell plate at a cell density of 1 × 10⁵ cells/well and co-incubated at 37 °C for 6 h in an incubator. Finally, a ONE-Glo luciferase assay reagent (Promega) was added to a mixed system of the cells and the antibodies, and a chemiluminescence signal was detected by using a multi-mode microplate reader (TECAN M1000 pro). A four-parameter regression curve was fitted by using GraphPad Prism software and EC₅₀ values were calculated. The positive control and the negative control were the anti-CD112R antibody Ref of University of Colorado (obtained by expression and purification according to the sequences in US20170240613) and the anti-KLH hu-IgG4 antibody, respectively.

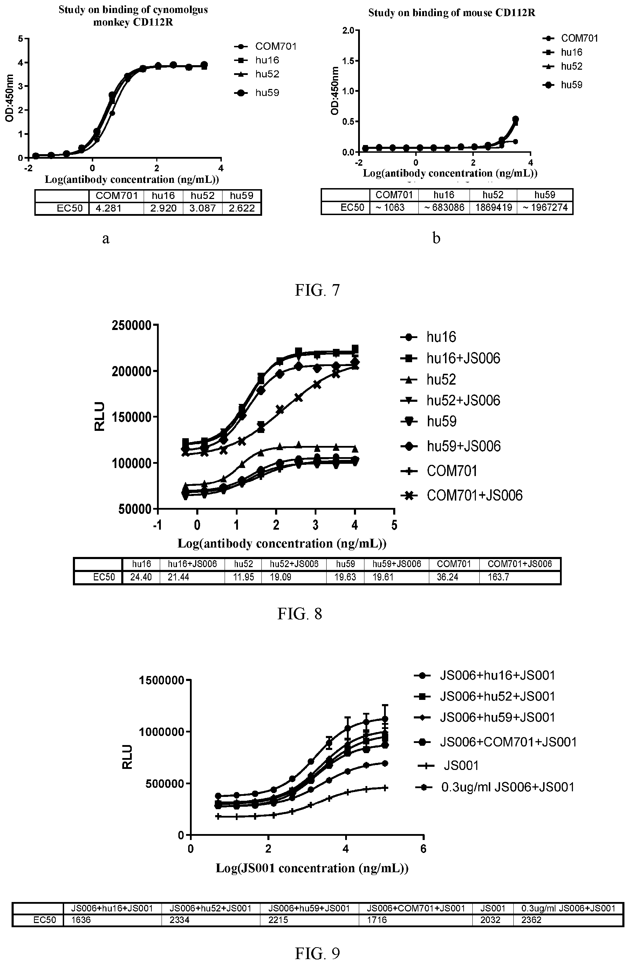

As shown in FIG. 6 (a-e) and Table 9, the following 13 chimeric antibodies were all capable of effectively blocking the interaction of CD112R with CD112 in a CD 112R/CD 112 reporter gene system and promoting the activation of T cells. The relative activity of the 7 chimeric antibodies, chi 46, chi 54, chi 45, chi 62, chi 53, chi 55 and chi 47, was more than 20 times higher than that of Ref.

The heavy chain and the light chain of the 7 chimeric antibodies were selected for separate humanized design and modification.

**Table 9: Data for assay on the activity of the chimeric anti-CD112R antibodies in the CD112R/CD112 reporter gene detection system**

| Chimeric antibody | EC₅₀(ng/ml) | Relative activity (%) * |
|---|---|---|
| chi 46 | 7.01 | 4609.68 |
| chi 54 | 7.46 | 4205.23 |
| chi 45 | 11.94 | 2705.19 |
| chi 62 | 12.88 | 2349.38 |
| chi 53 | 12.9 | 2430.23 |
| chi 55 | 13.12 | 2306.4 |
| chi 47 | 14.04 | 2232.91 |
| chi 70 | 13.86 | 1867.97 |
| chi 63 | 17.41 | 1487.08 |
| chi 69 | 18.66 | 1387.46 |
| chi 61 | 19 | 1592.63 |
| chi 71 | 19.45 | 1470.95 |
| chi 36 | 30.57 | 1056.59 |

| | | |
|---|---|---|
| Note: "*" indicated the EC₅₀ ratio of the control antibody Ref to the chimeric anti-CD112R antibody. | | |

### Example 7. Humanization of Antibody Variable Regions

For humanization of antibody variable regions, human IgG genes homologous to the cDNA sequence of the murine antibody were retrieved in the human immunoglobulin gene database of the NCBI (http://www.ncbi.nlm.nih.gov/igblast/), and the amino acid sequences of the variable region CDRs and their precise boundaries were defined by the Kabat numbering system or the IMGT numbering system. Human IGHV with high homology to the murine antibody was selected as a template for humanization and antibody variable regions were humanized by CDR grafting.

Humanization was performed based on the sequences of the obtained murine antibodies described above. Briefly, the humanization comprises the following steps: A. comparing the gene sequence of the murine antibody with the gene sequence of the human embryonic antibody to find out a sequence with high homology; B. analyzing and inspecting HLA-DR affinity, and selecting a human embryonic framework sequence with low affinity; and C. analyzing the framework amino acid sequences of the variable region and their periphery by using a computer simulation technology and applying molecular docking to investigate their spatial and stereo combination modes. The key amino acid individuals that may interact with human CD112R and maintain the spatial framework in the gene sequence of the murine antibody were analyzed by calculating electrostatic force, Van der Waals' force, hydrophilicity and hydrophobicity, and entropy value, and grafted to the selected human embryonic gene framework. The amino acid positions of the framework regions that must be retained were mapped. After that, the humanized antibody was synthesized. On this basis, various humanized antibody variable regions were obtained, and the designed humanized anti-CD112R antibody variable regions were variously combined to obtain 60 humanized anti-CD112R antibodies. The numbers and amino acid sequences of the antibodies are shown in detail in Table 3.

### Example 8. Screening of Humanized Anti-CD112R Antibodies

### 8.1 Binding of humanized anti-CD112R antibodies to a human CD112R protein

A plate was coated with a hCD112R-ECD-mFC protein (1.5 µg/mL) at a fixed concentration, and the humanized anti-CD112R antibodies diluted in a gradient (initial concentration: 1 µg/mL, 2.5-fold gradient dilution, 12 concentration gradients in total) were added to bind to the antigen. HRP-conjugated goat anti-human antibody IgG (Fc specific) (Sigma, A0170) was subjected to a 5000-fold dilution and used as a detection antibody for detection. The color development was performed with 0.1 mg/mL TMB, and finally stopped with 2 M hydrochloric acid. The plate was read at 420 nm/620 nm. EC₅₀ was obtained by fitting with a four-parameter logistic regression (4PL) model. The positive controls were COM701 (anti-CD112R antibody, Compugen, obtained by expression and purification according to the sequences in US20180280506A1) and the anti-KLH hu-IgG4 antibody.

As shown in Table 10, 60 humanized anti-CD112R antibodies were all capable of binding to the recombinant human CD112R protein with high affinity.

**Table 10: The binding of the humanized anti-CD112R antibodies to the human CD112R protein EC₅₀**

| Sample name | EC₅₀(ng/ml) | Relative activity (%) * | Sample name | EC₅₀(ng/ml) | Relative activity (%) * |
|---|---|---|---|---|---|
| Hu-01 | 2.76 | 154.5 | hu-19 | 4.109 | 139.0 |
| Hu-02 | 3.033 | 140.6 | hu-20 | 5.183 | 128.4 |
| Hu-03 | 3.554 | 119.9 | hu-21 | 2.904 | 229.2 |
| Hu-04 | 2.951 | 144.5 | hu-22 | 4.253 | 156.5 |
| Hu-05 | 2.751 | 155.0 | hu-23 | 4.361 | 152.6 |
| Hu-06 | 2.707 | 157.5 | hu-24 | 4.288 | 155.2 |
| Hu-07 | 4.32 | 91.0 | hu-25 | 4.027 | 165.3 |
| Hu-08 | 3.091 | 127.2 | hu-26 | 3.513 | 189.5 |
| Hu-09 | 3.452 | 113.9 | hu-27 | 4.42 | 137.3 |
| Hu-10 | 2.571 | 152.9 | hu-28 | 4.787 | 126.7 |
| Hu-11 | 2.747 | 143.1 | hu-29 | 4.128 | 147.0 |
| Hu-12 | 2.885 | 136.3 | hu-30 | 3.961 | 153.2 |
| hu-13 | 4.331 | 131.9 | hu-31 | 3.386 | 179.2 |
| hu-14 | 4.43 | 129.0 | hu-32 | 4.043 | 150.1 |
| hu-15 | 4.514 | 126.6 | hu-33 | 3.476 | 174.5 |
| hu-16 | 4.259 | 126.1 | hu-34 | 3.681 | 162.1 |
| hu-17 | 4.184 | 136.5 | hu-35 | 4.394 | 135.8 |
| hu-18 | 4.664 | 122.5 | hu-36 | 4.201 | 142.0 |
| hu-37 | 4.832 | 177.6 | hu-49 | 4.948 | 176.3 |
| hu-38 | 5.621 | 152.7 | hu-50 | 4.588 | 190.1 |
| hu-39 | 5.654 | 151.8 | hu-51 | 6.634 | 138.0 |
| hu-40 | 5.528 | 155.3 | hu-52 | 5.679 | 161.2 |
| hu-41 | 5.147 | 166.8 | hu-53 | 5.415 | 169.0 |
| hu-42 | 5.123 | 167.5 | hu-54 | 6.308 | 145.1 |
| hu-43 | 4.589 | 187.0 | hu-55 | 5.531 | 165.5 |
| hu-44 | 6.248 | 139.6 | hu-56 | 4.752 | 192.6 |
| hu-45 | 6.437 | 135.5 | hu-57 | 5.35 | 171.1 |
| hu-46 | 4.964 | 175.7 | hu-58 | 4.876 | 172.1 |
| hu-47 | 6.722 | 129.8 | hu-59 | 4.959 | 169.2 |
| hu-48 | 5.74 | 152.0 | hu-60 | 6.024 | 139.3 |

| | | | | | |
|---|---|---|---|---|---|
| Note: "*" indicated the EC₅₀ ratio of the control antibody COM701 to the humanized anti-CD112R antibody; Hu and hu in the numbers of the humanized anti-CD112R antibodies had the same meaning and may be used interchangeably, for example, Hu-01, Hu01, hu-01, hu01, hu-1, and hu1 all indicated the same humanized antibody. | | | | | |

### 8.2 Blocking of the binding of a CD112R protein to CHO-CD112-expressing cells by humanized anti-CD112 antibodies

Cells CHO CD112 overexpressing human CD112 were co-incubated with a hCD112R mFc protein solution (5 µg/mL) prepared in a staining buffer (PBS + 1% FBS), and the humanized anti-CD112R antibodies or the control antibodies at different concentrations (initial concentration: 100 µg/mL, 3-fold dilution, 10 concentration gradients) at 4 °C for 30 min. Unbound antibodies were then washed with a staining buffer and the cells were incubated with 1% (v/v) goat anti-mouse IgG Fc fluorescent secondary antibodies (Jackson ImmunoResearch) prepared in a staining buffer at 4 °C for 30 min in the dark. Finally, the cells were collected by using a flow cytometer (BD Canto II), and the fluorescent antibodies bound to the cell surface were detected. Raw data were analyzed by using FlowJo to obtain MFI values, and a four-parameter curve fitting was performed by using GraphPad Prism software to obtain IC₅₀ values. The positive control and the negative control were COM701 (anti-CD112R antibody, Compugen, obtained by expression and purification according to the sequences in US20180280506A1) and the anti-KLH hu-IgG4 antibody, respectively.

As shown in Table 11, the following 60 chimeric antibodies were all capable of effectively blocking the binding of the CD112R protein to CHO-CD112-expressing cells.

**Table 11: The ability of the humanized anti-CD112R antibodies to block the binding of the CD112R protein to CHO-CD112 cells (IC₅₀)**

| Sample | IC50 (ng/ml) | Relative activity (%) * | Sample | IC50 (ng/ml) | Relative activity (%) * |
|---|---|---|---|---|---|
| hu-1 | 4314 | 92.20 | hu-37 | 3166 | 76.00 |
| hu-2 | 4282 | 92.90 | hu-3 8 | 3052 | 78.90 |
| hu-3 | 4235 | 93.90 | hu-3 9 | 3247 | 74.10 |
| hu-4 | 4223 | 94.20 | hu-40 | 2719 | 88.50 |
| hu-5 | 4408 | 90.20 | hu-41 | 2905 | 82.90 |
| hu-6 | 4464 | 89.10 | hu-42 | 2985 | 80.60 |
| hu-7 | 5058 | 78.60 | hu-43 | 2583 | 93.20 |
| hu-8 | 4621 | 86.10 | hu-44 | 3553 | 67.70 |
| hu-9 | 4078 | 97.50 | hu-45 | 2850 | 84.50 |
| hu-10 | 4165 | 95.50 | hu-46 | 2288 | 105.20 |
| hu-11 | 4340 | 91.60 | hu-47 | 2789 | 86.30 |
| hu-12 | 4180 | 95.10 | hu-48 | 3108 | 77.40 |
| hu-17 | 2120 | 169 | hu-49 | 2469 | 97.50 |
| hu-13 | 2185 | 164 | hu-50 | 2675 | 90.00 |
| hu-14 | 2212 | 162 | hu-51 | 3479 | 69.20 |
| hu-20 | 2251 | 159 | hu-52 | 2521 | 95.50 |
| hu-28 | 2272 | 157 | hu-53 | 3181 | 75.70 |
| hu-27 | 2306 | 155 | hu-54 | 3249 | 74.10 |
| hu-30 | 2371 | 151 | hu-55 | 2807 | 85.70 |
| hu-34 | 2397 | 149 | hu-56 | 3391 | 71.00 |
| hu-36 | 2441 | 146 | hu-57 | 3636 | 66.20 |
| hu-21 | 2443 | 146 | hu-58 | 3251 | 74.00 |
| hu-26 | 2472 | 145 | hu-59 | 3167 | 76.00 |
| hu-19 | 2478 | 144 | hu-60 | 3486 | 69.00 |
| hu-33 | 2481 | 144 | hu-18 | 3390 | 71.00 |
| hu-16 | 2497 | 143 | hu-24 | 2890 | 124 |
| hu-18 | 2508 | 143 | hu-15 | 3286 | 109 |
| hu-31 | 2515 | 142 | hu-35 | 3400 | 105 |
| hu-23 | 2529 | 141 | hu-29 | 3675 | 97 |
| hu-22 | 2667 | 134 | hu-25 | 3702 | 97 |
| hu-32 | 2818 | 127 | chi-54 | 3874 | 92 |

| | | | | | |
|---|---|---|---|---|---|
| Note: "*" indicated the IC₅₀ ratio of the control antibody COM701 to the humanized anti-CD112R antibody. | | | | | |

### 8.3 Assay on the activity of humanized anti-CD112 antibodies in a CD 112R/CD 112 reporter gene assay system

Target cells CHO CD112 (stably expressing hCD112 and CD3scFv) cell were placed into a 96-well flat-bottom white plate overnight at a cell density of 5 × 10⁴ cells/well. After the medium in the cell wells was pipetted the next day, the humanized anti-CD112R antibodies or the control antibodies at different concentrations (initial concentration: 3 µg/mL, 3-fold dilution, 10 concentration gradients in total) were added to the target cells with an experimental buffer (RPMI 1640 (1×) + 2% FBS) and incubated at 37 °C for 30 min. Effector cells Jurkat CD112R (stably expressing human CD112R and luc2P/NFAT-RE) were then added to the cell plate at a cell density of 1 × 10⁵ cells/well and co-incubated at 37 °C for 6 h in an incubator. Finally, a ONE-Glo luciferase assay reagent (Promega) was added to a mixed system of the cells and the antibodies, and a chemiluminescence signal was detected by using a multi-mode microplate reader (TECAN M1000 pro). A four-parameter regression curve was fitted by using GraphPad Prism software and EC₅₀ values were calculated. The positive control was COM701 (anti-CD112R antibody, Compugen, obtained by expression and purification according to the sequences in US20180280506A1).

As shown in Table 12, among the following 60 humanized antibodies, except for hu-57, the other 59 humanized anti-CD112R antibodies were all capable of effectively blocking the interaction of CD112R with CD112 in a CD112R/CD112 reporter gene system and promoting the activation of T cells. The relative activity of the 6 humanized antibodies, hu-52, hu-51, hu-53, hu-59, hu-54 and hu-55, was more than 3 times higher than that of Ref (CGEN). The relative activity of the 6 humanized antibodies, hu-16, hu-39, hu-45, hu-58, hu-56 and hu-60, was 2.5-3 times higher than that of Ref (CGEN).

**Table 12: Data for assay on the activity of the humanized anti-CD112R antibodies in the CD112R/CD112 reporter gene assay system**

| Sample | EC₅₀ (ng/ml) | Relative activity (%) * | Sample | EC₅₀ (ng/ml) | Relative activity (%) * |
|---|---|---|---|---|---|
| hu-10 | 37.72 | 199 | hu-22 | 26.73 | 190.7 |
| hu-9 | 38.45 | 195.3 | hu-21 | 28.51 | 178.8 |
| hu-3 | 37.98 | 188.9 | hu-20 | 37.95 | 134.7 |
| hu-4 | 38.47 | 186.5 | hu-19 | 40.37 | 126.6 |
| hu-11 | 52.89 | 148.1 | hu-28 | 30.61 | 232.3 |
| hu-5 | 46.09 | 147.5 | hu-29 | 27.16 | 230.2 |
| hu-7 | 143.6 | 48.6 | hu-27 | 32.77 | 217 |
| hu-12 | 54.25 | 144.4 | hu-23 | 37.4 | 216.8 |
| hu-6 | 52.92 | 128.5 | hu-30 | 33.96 | 184.1 |
| hu-8 | 63.33 | 110.1 | hu-35 | 30.99 | 183.7 |
| hu-2 | 57.66 | 109.8 | hu-26 | 35.32 | 183.4 |
| hu-1 | 68.83 | 92 | hu-24 | 48.55 | 167 |
| hu-16 | 19.58 | 253 | hu-36 | 35.62 | 159.8 |
| hu-17 | 22.77 | 241.1 | hu-34 | 33.57 | 159.6 |
| hu-14 | 22.46 | 214.9 | hu-33 | 34.08 | 157.2 |
| hu-15 | 23.66 | 209.3 | hu-25 | 41.24 | 157.1 |
| hu-18 | 26.3 | 208.7 | hu-32 | 47.05 | 119.1 |
| hu-13 | 25.18 | 191.7 | hu-31 | 50.74 | 110.4 |
| hu-37 | 35.54 | 181.8 | hu-52 | 18.02 | 414.7 |
| hu-38 | 33.66 | 192 | hu-51 | 22.51 | 331.9 |
| hu-39 | 21.69 | 253.6 | hu-53 | 23.03 | 313.5 |
| hu-40 | 24.23 | 227 | hu-59 | 18.79 | 312.3 |
| hu-41 | 26.67 | 242.9 | hu-55 | 22.27 | 301.9 |
| hu-42 | 35.48 | 182.6 | hu-54 | 24.07 | 300 |
| hu-43 | 42.57 | 158.5 | hu-58 | 21.61 | 277 |
| hu-44 | 38.66 | 174.5 | hu-56 | 24.33 | 276.3 |
| hu-45 | 25.6 | 261.4 | hu-60 | 22.46 | 261.3 |
| hu-46 | 26.91 | 248.6 | hu-50 | 26.79 | 245.1 |
| hu-47 | 38.98 | 190.5 | hu-49 | 30.72 | 213.7 |
| hu-48 | 42.06 | 176.5 | hu-57 | 1326 | 4.5 |

| | | | | | |
|---|---|---|---|---|---|
| Note: "*" indicated the EC₅₀ ratio of the control antibody COM701 to the humanized anti-CD112R antibody. | | | | | |

### 8.4 Binding activity of humanized anti-CD112R antibodies to cynomolgus monkey and mouse CD112R

Plates were coated with a recombinant cynomolgus monkey CD112R antigen (purchased from Acrobiosystems, Cat No. PVG-C5259) and a mouse CD112R antigen (purchased from Acrobiosystems, Cat No. PVG-M5257) at fixed concentrations, respectively, and the humanized anti-CD112R antibodies diluted in a gradient (initial concentration: 1 µg/mL, 2.5-fold gradient dilution, 12 concentration gradients in total) were added to bind to the antigens. HRP-conjugated mouse anti-human IgG4 Fc (Southern Biotech, 9200-05) was subjected to a 5000-fold dilution and used as detection antibody for detection. The color development was performed with 0.1 mg/mL TMB, and finally stopped with 2 M hydrochloric acid. The plates were read at 420 nm/620 nm. EC₅₀ was obtained by fitting with a four-parameter logistic regression (4PL) model.

As shown in FIG. 7 (a and b), hu16, hu52 and hu59 all bound to cynomolgus monkey CD112R with high affinity, and their EC₅₀ were 2.92 ng/mL, 3.087 ng/mL and 2.622 ng/mL, respectively, but none of them had binding activity to mouse CD112R.

### 8.5 Assay on the activity of the humanized anti-CD112R antibodies in combination with the anti-TIGIT antibody in a dual reporter gene system

Target cells CHO CD112 cell (stably expressing hCD112 and CD3scFv) were placed into a 96-well white flat-bottom plate (Corning, Cat No. 3917) overnight at a cell density of 5 × 10⁴ cells/well, and then the humanized anti-CD112R antibodies at different concentrations (from 0.15 ng/mL to 3 µg/mL, 3-fold dilution) and the anti-TIGIT antibody at a fixed concentration (0.3 µg/mL) were added and incubated at 37 °C for 30 min. Effector cells Jurkat PD-1 TIGTI CD112R (stably expressing human PD-1, human TIGIT, human CD112R and luc2P/NFAT-RE) were then added to the target cells at a cell density of 1 × 10⁵ cells/well and co-incubated at 37 °C for 4-6 h in an incubator. Finally, a ONE-Glo luciferase assay reagent (Promega, Cat No. E6120) was added to a mixed system of the cells and the antibodies, and a chemiluminescence signal was detected by using a multi-mode microplate reader (TECAN M1000 pro).

Anti-TIGIT antibody (JS006): derived from the anti-TIGIT humanized antibody hu20 in PCT application No. PCT/CN2020/101883.

The positive control was COM701 (anti-CD112R antibody, Compugen, obtained by expression and purification according to the sequences in US20180280506A1).

As shown in the dose-dependent curves of FIG. 8, in the dual reporter gene system, after the anti-CD112R antibodies hu16, hu52 and hu59 are used in combination with the anti-TIGIT antibody, the luciferase reporter gene NFAT signal of the combination was significantly higher than that of the anti-CD112R antibody monotherapy group, which indicated that the combination of the anti-CD112R antibodies and the anti-TIGIT antibody had a significant synergistic effect, and the efficacy of the combination group of the three humanized anti-CD112R antibodies and the anti-TIGIT antibody is significantly better than that of the COM701 combination group.

### 8.6 Assay on the activity of humanized anti-CD112R antibodies in combination with an anti-TIGIT antibody and an anti-PD-1 antibody in a triple reporter gene system

Target cells CHO PD-L1 CD112 (stably expressing hPD-L1, hCD112 and CD3scFv) were placed into a 96-well flat-bottom white plate overnight at a cell density of 5 × 10⁴ cells/well (Corning, Cat No. 3917), and then the anti-PD-1 antibodies at different concentrations (from 0.5 ng/mL to 10 µg/mL, 3-fold dilution) and the anti-TIGIT antibody at a fixed concentration (0.3 µg/mL) and the anti-CD112R antibody at a fixed concentration (0.3 µg/mL) were added and incubated at 37 °C for 30 min. Effector cells Jurkat PD-1 TIGTI CD112R (stably expressing human PD-1, human TIGIT, human CD112R and luc2P/NFAT-RE) were then added to the target cells at a cell density of 1 × 10⁵ cells/well and co-incubated at 37 °C for 4-6 h in an incubator. Finally, a ONE-Glo luciferase assay reagent (Promega, Cat No. E6120) was added to a mixed system of the cells and the antibodies, and a chemiluminescence signal was detected by using a multi-mode microplate reader (TECAN M1000 pro).

Anti-TIGIT antibody (JS006): derived from the humanized anti-TIGIT antibody hu20 in PCT application No. PCT/CN2020/101883.

Anti-PD-1 antibody (JS001): toripalimab derived from Junshi Biosciences.

The positive control was COM701 (anti-CD112R antibody, Compugen, obtained by expression and purification according to the sequences in US20180280506A1).

As shown in the dose-dependent curves of FIG. 9, in the triple reporter gene system, the luciferase reporter gene NFAT signal of the combination group of the anti-CD112R antibodies hu16, hu52 and hu59, the anti-TIGIT antibody and the anti-PD-1 antibody was significantly higher than that of the anti-PD-1 antibody monotherapy group and the combination group of the anti-PD-1 antibody and the anti-TIGIT antibody at 0.3 µg/mL, which indicated that the combination of the anti-TIGIT antibody, the anti-PD-1 antibody and the anti-CD112R antibodies had a significant synergistic effect.

### Example 9. Pharmacodynamic Evaluation of Anti-CD112R Antibodies, Anti-TIGIT Antibody and Combinations Thereof on Subcutaneous Xenograft Tumor Model of Human Melanoma A375 Mixed with PBMCs

Experimental animals: 48 NCG mice, female, 6-8 weeks old, weighing about 18-22 g. Purchased from Jiangsu Gempharmatech Co., Ltd.

Anti-TIGIT antibody (JS006): derived from the anti-TIGIT humanized antibody hu20 in PCT application No. PCT/CN2020/101883.

The anti-CD112R antibody is the humanized antibody hu52 described herein.

A375 cells were cultured in a DMEM medium containing 10% fetal bovine serum (FBS). The A375 cells in the logarithmic phase were collected and HBSS (Hank's balanced salt solution) was resuspended to an appropriate concentration for subcutaneous tumor inoculation in NCG mice. The A375 cells used in co-culture experiments were treated with Mitomycin C for 2 h and then washed three times with PBS. Frozen PBMC was taken out, thawed and counted. The obtained PBMCs were added to the A375 cells treated with Mitomycin C. The PBMCs and the A375 were co-cultured for 5 days. The culture medium was a RPMI 1640 culture medium containing IL-2 and 10% FBS. After the PBMC were co-cultured with the A375 for 5 days, the PBMC and the freshly digested A375 cells were collected. 4×10⁵ PBMCs and 4×10⁶ A375 cells were inoculated subcutaneously on the right side of the NCG mice at 0.2 mL/mouse (containing 50% Matrigel). The day of inoculation was day 0, and the mice were randomly divided, according to the body weight, into groups for drug administration. Detailed administration regimens, administration doses and routes of administration are shown in Table 13.

**Table 13: Dosing regimen**

| **Group** | **Administration group** | **N** | **Dose** | **Dosing regimen** | **Route of administration** |
|---|---|---|---|---|---|
| 1 | Vehicle control | 8 | -- | Q3D | i.p. |
| 2 | JS006 | 8 | 30 mg/kg | Q3D | i.p. |
| 3 | hu52 | 8 | 15 mg/kg | Q3D | i.p. |
| 4 | hu52 | 8 | 30 mg/kg | Q3D | i.p. |
| 5 | JS006+ hu52 | 8 | (30+15) mg/kg | Q3D | i.p. |
| 6 | JS006+ hu52 | 8 | (30+30) mg/kg | Q3D | i.p. |

| | | | | | |
|---|---|---|---|---|---|
| Note: N: the number of animals used; i.p.: intraperitoneal injection; Q3D: once every three days. Administration volume: the administration volume (0.1 mL/10g) was adjusted according to the body weight of tumor-bearing mice. | | | | | |

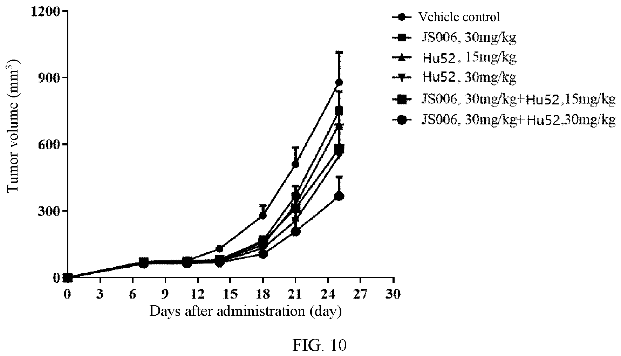

After the start of the experiment, tumor volume and body weight of the mice were measured twice a week. The experimental results are shown in Table 14 and FIG. 10.

**Table 14: Mean tumor volume of each group of mice (Mean ± SEM)**

| **Group** | **Treatment group** | **Tumor volume (mm³)** | | **TGI % (Day 25)** | **T/C % (Day 25)** | **P value** |
|---|---|---|---|---|---|---|
| | | **Day 0** | **Day 25** | | | |
| 1 | Vehicle control | 0±0 | 879.65±133.85 | -- | -- | -- |
| 2 | JS006,30mg/kg | 0±0 | 748.81±88.96 | 14.87 | 85.13 | 0.383 |
| 3 | hu52,15mg/kg | 0±0 | 690.15±75.86 | 21.54 | 78.46 | 0.209 |
| 4 | hu52,30mg/kg | 0±0 | 548.24+123.52* | 37.68 | 62.32 | 0.031 |
| 5 | JS006,30mg/kg+ hu52,15mg/kg | 0±0 | 580.58±108.66 | 34.00 | 66.00 | 0.050 |
| 6 | JS006,30mg/kg+ hu52,30mg/kg | 0±0 | 366.84±86.34** | 58.30 | 41.70 | 0.001 |

| | | | | | | |
|---|---|---|---|---|---|---|
| Note: p < 0.05 indicated a significant difference; T/C (%) indicated a relative tumor proliferation rate, i.e., the percentage value of the tumor volume of the administration group relative to that of the control group at a certain time point, wherein T and C indicated the tumor volumes (TVs) of the administration group and the control group, respectively, at a certain specific time point; and tumor growth inhibition (TGI) rate (%) = (1 - T/C) × 100%. | | | | | | |

At the end of the experiment (day 25 after the administration), compared to the control group, the anti-CD112R antibody hu52 at a dose level of 30 mg/kg significantly inhibited tumor growth, with a tumor growth inhibition rate of 37.68% (p < 0.05); the combination of the anti-CD112R antibody hu52 at 30 mg/kg and JS006 at 30 mg/kg showed a significant synergistic anti-tumor effect, with a tumor growth inhibition rate of 58.30% (p < 0.01).

## Claims

1. An anti-CD112R antibody or an antigen-binding fragment thereof, wherein the antibody or the antigen-binding fragment thereof comprises HCDR1, HCDR2, HCDR3, LCDR1, LCDR2, and LCDR3 as follows:
HCDR1: SYHMS;
HCDR2: AIX₁X₂X₃X₄X₅X₆TYYX₇DTVKG, wherein X₁ is N or S, X₂ is S or R, X₃ is N or D, X₄ is D, G or V, X₅ is I or D, X₆ is N or S, and X₇ is L or P;
HCDR3: HEDYX₈GFAMDX₉, wherein X₈ is Y or F, and X₉ is Y or S;
LCDR1: KASQSVX₁₀NDVA, wherein X₁₀ is T or S;
LCDR2: YVSX₁₁X₁₂X₁₃T, wherein X₁₁ is N or H, X₁₂ is H or R, and X₁₃ is Y or F;
LCDR3: X₁₄QAYRSPWT, wherein X₁₄ is Q or H;
preferably, the antibody or the antigen-binding fragment thereof comprises: HCDR1 having an amino acid sequence set forth in SEQ ID NO: 13; HCDR2 having an amino acid sequence set forth in SEQ ID NO: 14, 41, 50, or 68; HCDR3 having an amino acid sequence set forth in SEQ ID NO: 15 or 42; LCDR1 having an amino acid sequence set forth in SEQ ID NO: 43 or 52; LCDR2 having an amino acid sequence set forth in SEQ ID NO: 44, 53, or 71; and LCDR3 having an amino acid sequence set forth in SEQ ID NO: 54 or 72.

2. An anti-CD112R antibody or an antigen-binding fragment thereof, wherein the anti-CD112R antibody or the antigen-binding fragment thereof comprises a heavy chain variable region and/or a light chain variable region, wherein:
the heavy chain variable region comprises:
(I) HCDR1, HCDR2, and HCDR3 having amino acid sequences set forth in SEQ ID NO: 1, SEQ ID NO: 2, and SEQ ID NO: 3, respectively; or HCDR1, HCDR2, and HCDR3 having 1, 2, or 3 amino acid alterations as compared with the amino acid sequences set forth in SEQ ID NO: 1, SEQ ID NO: 2, and SEQ ID NO: 3, respectively; or
(II) HCDR1, HCDR2, and HCDR3 having amino acid sequences set forth in SEQ ID NO: 7, SEQ ID NO: 8, and SEQ ID NO: 9, respectively; or HCDR1, HCDR2, and HCDR3 having 1, 2, or 3 amino acid alterations as compared with the amino acid sequences set forth in SEQ ID NO: 7, SEQ ID NO: 8, and SEQ ID NO: 9, respectively; or
(III) HCDR1, HCDR2, and HCDR3 having amino acid sequences set forth in SEQ ID NO: 13, SEQ ID NO: 14, and SEQ ID NO: 15, respectively; or HCDR1, HCDR2, and HCDR3 having 1, 2, or 3 amino acid alterations as compared with the amino acid sequences set forth in SEQ ID NO: 13, SEQ ID NO: 14, and SEQ ID NO: 15, respectively; or
(IV) HCDR1, HCDR2, and HCDR3 having amino acid sequences set forth in SEQ ID NO: 16, SEQ ID NO: 17, and SEQ ID NO: 18, respectively; or HCDR1, HCDR2, and HCDR3 having 1, 2, or 3 amino acid alterations as compared with the amino acid sequences set forth in SEQ ID NO: 16, SEQ ID NO: 17, and SEQ ID NO: 18, respectively; or
(V) HCDR1, HCDR2, and HCDR3 having amino acid sequences set forth in SEQ ID NO: 22, SEQ ID NO: 23, and SEQ ID NO: 24, respectively; or HCDR1, HCDR2, and HCDR3 having 1, 2, or 3 amino acid alterations as compared with the amino acid sequences set forth in SEQ ID NO: 22, SEQ ID NO: 23, and SEQ ID NO: 24, respectively; or
(VI) HCDR1, HCDR2, and HCDR3 having amino acid sequences set forth in SEQ ID NO: 28, SEQ ID NO: 29, and SEQ ID NO: 30, respectively; or HCDR1, HCDR2, and HCDR3 having 1, 2, or 3 amino acid alterations as compared with the amino acid sequences set forth in SEQ ID NO: 28, SEQ ID NO: 29, and SEQ ID NO: 30, respectively; or
(VII) HCDR1, HCDR2, and HCDR3 having amino acid sequences set forth in SEQ ID NO: 34, SEQ ID NO: 35, and SEQ ID NO: 36, respectively; or HCDR1, HCDR2, and HCDR3 having 1, 2, or 3 amino acid alterations as compared with the amino acid sequences set forth in SEQ ID NO: 34, SEQ ID NO: 35, and SEQ ID NO: 36, respectively; or
(VIII) HCDR1, HCDR2, and HCDR3 having amino acid sequences set forth in SEQ ID NO: 40, SEQ ID NO: 41, and SEQ ID NO: 42, respectively; or HCDR1, HCDR2, and HCDR3 having 1, 2, or 3 amino acid alterations as compared with the amino acid sequences set forth in SEQ ID NO: 40, SEQ ID NO: 41, and SEQ ID NO: 42, respectively; or
(IX) HCDR1, HCDR2, and HCDR3 having amino acid sequences set forth in SEQ ID NO: 49, SEQ ID NO: 50, and SEQ ID NO: 51, respectively; or HCDR1, HCDR2, and HCDR3 having 1, 2, or 3 amino acid alterations as compared with the amino acid sequences set forth in SEQ ID NO: 49, SEQ ID NO: 50, and SEQ ID NO: 51, respectively; or
(X) HCDR1, HCDR2, and HCDR3 having amino acid sequences set forth in SEQ ID NO: 55, SEQ ID NO: 56, and SEQ ID NO: 57, respectively; or HCDR1, HCDR2, and HCDR3 having 1, 2, or 3 amino acid alterations as compared with the amino acid sequences set forth in SEQ ID NO: 55, SEQ ID NO: 56, and SEQ ID NO: 57, respectively; or
(XI) HCDR1, HCDR2, and HCDR3 having amino acid sequences set forth in SEQ ID NO: 61, SEQ ID NO: 62, and SEQ ID NO: 63, respectively; or HCDR1, HCDR2, and HCDR3 having 1, 2, or 3 amino acid alterations as compared with the amino acid sequences set forth in SEQ ID NO: 61, SEQ ID NO: 62, and SEQ ID NO: 63, respectively; or
(XII) HCDR1, HCDR2, and HCDR3 having amino acid sequences set forth in SEQ ID NO: 67, SEQ ID NO: 68, and SEQ ID NO: 69, respectively; or HCDR1, HCDR2, and HCDR3 having 1, 2, or 3 amino acid alterations as compared with the amino acid sequences set forth in SEQ ID NO: 67, SEQ ID NO: 68, and SEQ ID NO: 69, respectively; or
(XIII) HCDR1, HCDR2, and HCDR3 having amino acid sequences set forth in SEQ ID NO: 73, SEQ ID NO: 74, and SEQ ID NO: 75, respectively; or HCDR1, HCDR2, and HCDR3 having 1, 2, or 3 amino acid alterations as compared with the amino acid sequences set forth in SEQ ID NO: 73, SEQ ID NO: 74, and SEQ ID NO: 75, respectively; or
(XIV) HCDR1, HCDR2, and HCDR3 having amino acid sequences set forth in SEQ ID NO: 79, SEQ ID NO: 80, and SEQ ID NO: 81, respectively; or HCDR1, HCDR2, and HCDR3 having 1, 2, or 3 amino acid alterations as compared with the amino acid sequences set forth in SEQ ID NO: 79, SEQ ID NO: 80, and SEQ ID NO: 81, respectively; or
(XV) HCDR1, HCDR2, and HCDR3 having amino acid sequences set forth in SEQ ID NO: 143, SEQ ID NO: 144, and SEQ ID NO: 145, respectively; or HCDR1, HCDR2, and HCDR3 having 1, 2, or 3 amino acid alterations as compared with the amino acid sequences set forth in SEQ ID NO: 143, SEQ ID NO: 144, and SEQ ID NO: 145, respectively;
the light chain variable region comprises:
(I) LCDR1, LCDR2, and LCDR3 having amino acid sequences set forth in SEQ ID NO: 4, SEQ ID NO: 5, and SEQ ID NO: 6, respectively; or LCDR1, LCDR2, and LCDR3 having 1, 2, or 3 amino acid alterations as compared with the amino acid sequences set forth in SEQ ID NO: 4, SEQ ID NO: 5, and SEQ ID NO: 6, respectively; or
(II) LCDR1, LCDR2, and LCDR3 having amino acid sequences set forth in SEQ ID NO: 10, SEQ ID NO: 11, and SEQ ID NO: 12, respectively; or LCDR1, LCDR2, and LCDR3 having 1, 2, or 3 amino acid alterations as compared with the amino acid sequences set forth in SEQ ID NO: 10, SEQ ID NO: 11, and SEQ ID NO: 12, respectively; or
(III) LCDR1, LCDR2, and LCDR3 having amino acid sequences set forth in SEQ ID NO: 19, SEQ ID NO: 20, and SEQ ID NO: 21, respectively; or LCDR1, LCDR2, and LCDR3 having 1, 2, or 3 amino acid alterations as compared with the amino acid sequences set forth in SEQ ID NO: 19, SEQ ID NO: 20, and SEQ ID NO: 21, respectively; or
(IV) LCDR1, LCDR2, and LCDR3 having amino acid sequences set forth in SEQ ID NO: 25, SEQ ID NO: 26, and SEQ ID NO: 27, respectively; or LCDR1, LCDR2, and LCDR3 having 1, 2, or 3 amino acid alterations as compared with the amino acid sequences set forth in SEQ ID NO: 25, SEQ ID NO: 26, and SEQ ID NO: 27, respectively; or
(V) LCDR1, LCDR2, and LCDR3 having amino acid sequences set forth in SEQ ID NO: 31, SEQ ID NO: 32, and SEQ ID NO: 33, respectively; or LCDR1, LCDR2, and LCDR3 having 1, 2, or 3 amino acid alterations as compared with the amino acid sequences set forth in SEQ ID NO: 31, SEQ ID NO: 32, and SEQ ID NO: 33, respectively; or
(VI) LCDR1, LCDR2, and LCDR3 having amino acid sequences set forth in SEQ ID NO: 37, SEQ ID NO: 38, and SEQ ID NO: 39, respectively; or LCDR1, LCDR2, and LCDR3 having 1, 2, or 3 amino acid alterations as compared with the amino acid sequences set forth in SEQ ID NO: 37, SEQ ID NO: 38, and SEQ ID NO: 39, respectively; or
(VII) LCDR1, LCDR2, and LCDR3 having amino acid sequences set forth in SEQ ID NO: 43, SEQ ID NO: 44, and SEQ ID NO: 45, respectively; or LCDR1, LCDR2, and LCDR3 having 1, 2, or 3 amino acid alterations as compared with the amino acid sequences set forth in SEQ ID NO: 43, SEQ ID NO: 44, and SEQ ID NO: 45, respectively; or
(VIII) LCDR1, LCDR2, and LCDR3 having amino acid sequences set forth in SEQ ID NO: 46, SEQ ID NO: 47, and SEQ ID NO: 48, respectively; or LCDR1, LCDR2, and LCDR3 having 1, 2, or 3 amino acid alterations as compared with the amino acid sequences set forth in SEQ ID NO: 46, SEQ ID NO: 47, and SEQ ID NO: 48, respectively; or
(IX) LCDR1, LCDR2, and LCDR3 having amino acid sequences set forth in SEQ ID NO: 52, SEQ ID NO: 53, and SEQ ID NO: 54, respectively; or LCDR1, LCDR2, and LCDR3 having 1, 2, or 3 amino acid alterations as compared with the amino acid sequences set forth in SEQ ID NO: 52, SEQ ID NO: 53, and SEQ ID NO: 54, respectively; or
(X) LCDR1, LCDR2, and LCDR3 having amino acid sequences set forth in SEQ ID NO: 58, SEQ ID NO: 59, and SEQ ID NO: 60, respectively; or LCDR1, LCDR2, and LCDR3 having 1, 2, or 3 amino acid alterations as compared with the amino acid sequences set forth in SEQ ID NO: 58, SEQ ID NO: 59, and SEQ ID NO: 60, respectively; or
(XI) LCDR1, LCDR2, and LCDR3 having amino acid sequences set forth in SEQ ID NO: 64, SEQ ID NO: 65, and SEQ ID NO: 66, respectively; or LCDR1, LCDR2, and LCDR3 having 1, 2, or 3 amino acid alterations as compared with the amino acid sequences set forth in SEQ ID NO: 64, SEQ ID NO: 65, and SEQ ID NO: 66, respectively; or
(XII) LCDR1, LCDR2, and LCDR3 having amino acid sequences set forth in SEQ ID NO: 70, SEQ ID NO: 71, and SEQ ID NO: 72, respectively; or LCDR1, LCDR2, and LCDR3 having 1, 2, or 3 amino acid alterations as compared with the amino acid sequences set forth in SEQ ID NO: 70, SEQ ID NO: 71, and SEQ ID NO: 72, respectively; or
(XIII) LCDR1, LCDR2, and LCDR3 having amino acid sequences set forth in SEQ ID NO: 76, SEQ ID NO: 77, and SEQ ID NO: 78, respectively; or LCDR1, LCDR2, and LCDR3 having 1, 2, or 3 amino acid alterations as compared with the amino acid sequences set forth in SEQ ID NO: 76, SEQ ID NO: 77, and SEQ ID NO: 78, respectively; or
(XIV) LCDR1, LCDR2, and LCDR3 having amino acid sequences set forth in SEQ ID NO: 82, SEQ ID NO: 83, and SEQ ID NO: 84, respectively; or LCDR1, LCDR2, and LCDR3 having 1, 2, or 3 amino acid alterations as compared with the amino acid sequences set forth in SEQ ID NO: 82, SEQ ID NO: 83, and SEQ ID NO: 84, respectively;
preferably, the antibody or the antigen-binding fragment thereof comprises:
(I) a heavy chain variable region, comprising HCDR1, HCDR2, and HCDR3 having amino acid sequences set forth in SEQ ID NO: 1, SEQ ID NO: 2, and SEQ ID NO: 3, respectively; and a light chain variable region, comprising LCDR1, LCDR2, and LCDR3 having amino acid sequences set forth in SEQ ID NO: 4, SEQ ID NO: 5, and SEQ ID NO: 6, respectively; or
(II) a heavy chain variable region, comprising HCDR1, HCDR2, and HCDR3 having amino acid sequences set forth in SEQ ID NO: 7, SEQ ID NO: 8, and SEQ ID NO: 9, respectively; and a light chain variable region, comprising LCDR1, LCDR2, and LCDR3 having amino acid sequences set forth in SEQ ID NO: 10, SEQ ID NO: 11, and SEQ ID NO: 12, respectively; or
(III) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 4, SEQ ID NO: 5, and SEQ ID NO: 6, respectively; or
(IV) a heavy chain variable region, comprising HCDR1, HCDR2, and HCDR3 having amino acid sequences set forth in SEQ ID NO: 16, SEQ ID NO: 17, and SEQ ID NO: 18, respectively; and a light chain variable region, comprising LCDR1, LCDR2, and LCDR3 having amino acid sequences set forth in SEQ ID NO: 19, SEQ ID NO: 20, and SEQ ID NO: 21, respectively; or
(V) a heavy chain variable region, comprising HCDR1, HCDR2, and HCDR3 having amino acid sequences set forth in SEQ ID NO: 22, SEQ ID NO: 23, and SEQ ID NO: 24, respectively; and a light chain variable region, comprising LCDR1, LCDR2, and LCDR3 having amino acid sequences set forth in SEQ ID NO: 25, SEQ ID NO: 26, and SEQ ID NO: 27, respectively; or
(VI) a heavy chain variable region, comprising HCDR1, HCDR2, and HCDR3 having amino acid sequences set forth in SEQ ID NO: 28, SEQ ID NO: 29, and SEQ ID NO: 30, respectively; and a light chain variable region, comprising LCDR1, LCDR2, and LCDR3 having amino acid sequences set forth in SEQ ID NO: 31, SEQ ID NO: 32, and SEQ ID NO: 33, respectively; or
(VII) a heavy chain variable region, comprising HCDR1, HCDR2, and HCDR3 having amino acid sequences set forth in SEQ ID NO: 34, SEQ ID NO: 35, and SEQ ID NO: 36, respectively; and a light chain variable region, comprising LCDR1, LCDR2, and LCDR3 having amino acid sequences set forth in SEQ ID NO: 37, SEQ ID NO: 38, and SEQ ID NO: 39, respectively; or
(VIII) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(IX) a heavy chain variable region, comprising HCDR1, HCDR2, and HCDR3 having amino acid sequences set forth in SEQ ID NO: 28, SEQ ID NO: 29, and SEQ ID NO: 30, respectively; and a light chain variable region, comprising LCDR1, LCDR2, and LCDR3 having amino acid sequences set forth in SEQ ID NO: 46, SEQ ID NO: 47, and SEQ ID NO: 48, respectively; or
(X) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XI) a heavy chain variable region, comprising HCDR1, HCDR2, and HCDR3 having amino acid sequences set forth in SEQ ID NO: 55, SEQ ID NO: 56, and SEQ ID NO: 57, respectively; and a light chain variable region, comprising LCDR1, LCDR2, and LCDR3 having amino acid sequences set forth in SEQ ID NO: 58, SEQ ID NO: 59, and SEQ ID NO: 60, respectively; or
(XII) a heavy chain variable region, comprising HCDR1, HCDR2, and HCDR3 having amino acid sequences set forth in SEQ ID NO: 61, SEQ ID NO: 62, and SEQ ID NO: 63, respectively; and a light chain variable region, comprising LCDR1, LCDR2, and LCDR3 having amino acid sequences set forth in SEQ ID NO: 64, SEQ ID NO: 65, and SEQ ID NO: 66, respectively; or
(XIII) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XIV) a heavy chain variable region, comprising HCDR1, HCDR2, and HCDR3 having amino acid sequences set forth in SEQ ID NO: 73, SEQ ID NO: 74, and SEQ ID NO: 75, respectively; and a light chain variable region, comprising LCDR1, LCDR2, and LCDR3 having amino acid sequences set forth in SEQ ID NO: 76, SEQ ID NO: 77, and SEQ ID NO: 78, respectively; or
(XV) a heavy chain variable region, comprising HCDR1, HCDR2, and HCDR3 having amino acid sequences set forth in SEQ ID NO: 79, SEQ ID NO: 80, and SEQ ID NO: 81, respectively; and a light chain variable region, comprising LCDR1, LCDR2, and LCDR3 having amino acid sequences set forth in SEQ ID NO: 82, SEQ ID NO: 83, and SEQ ID NO: 84, respectively; or
(XVI) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XVII) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XVIII) a heavy chain variable region, comprising HCDR1, HCDR2, and HCDR3 having amino acid sequences set forth in SEQ ID NO: 13, SEQ ID NO: 14, and SEQ ID NO: 15, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XIX) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XX) a heavy chain variable region, comprising HCDR1, HCDR2, and HCDR3 having amino acid sequences set forth in SEQ ID NO: 40, SEQ ID NO: 41, and SEQ ID NO: 42, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XXI) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XXII) a heavy chain variable region, comprising HCDR1, HCDR2, and HCDR3 having amino acid sequences set forth in SEQ ID NO: 49, SEQ ID NO: 50, and SEQ ID NO: 51, respectively; and a light chain variable region, comprising LCDR1, LCDR2, and LCDR3 having amino acid sequences set forth in SEQ ID NO: 70, SEQ ID NO: 71, and SEQ ID NO: 72, respectively; or
(XXIII) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 43, SEQ ID NO: 44, and SEQ ID NO: 45, respectively; or
(XXIV) a heavy chain variable region, comprising HCDR1, HCDR2, and HCDR3 having amino acid sequences set forth in SEQ ID NO: 67, SEQ ID NO: 68, and SEQ ID NO: 69, respectively; and a light chain variable region, comprising LCDR1, LCDR2, and LCDR3 having amino acid sequences set forth in SEQ ID NO: 52, SEQ ID NO: 53, and SEQ ID NO: 54, respectively; or
(XXV) a heavy chain variable region, comprising HCDR1, HCDR2, and HCDR3 having amino acid sequences set forth in SEQ ID NO: 143, SEQ ID NO: 144, and SEQ ID NO: 145, respectively; and a light chain variable region, comprising LCDR1, LCDR2, and LCDR3 having amino acid sequences set forth in SEQ ID NO: 19, SEQ ID NO: 20, and SEQ ID NO: 21, respectively;
further preferably, the antibody or the antigen-binding fragment thereof comprises a heavy chain variable region and a light chain variable region, wherein:
(I) the heavy chain variable region comprises an amino acid sequence set forth in SEQ ID NO: 85, or comprises an amino acid sequence having at least 95%, 96%, 97%, 98%, or 99% sequence identity to the amino acid sequence set forth in SEQ ID NO: 85; and the light chain variable region comprises an amino acid sequence set forth in SEQ ID NO: 86, or comprises an amino acid sequence having at least 95%, 96%, 97%, 98%, or 99% sequence identity to the amino acid sequence set forth in SEQ ID NO: 86; or
(II) the heavy chain variable region comprises an amino acid sequence set forth in SEQ ID NO: 87, or comprises an amino acid sequence having at least 95%, 96%, 97%, 98%, or 99% sequence identity to the amino acid sequence set forth in SEQ ID NO: 87; and the light chain variable region comprises an amino acid sequence set forth in SEQ ID NO: 88, or comprises an amino acid sequence having at least 95%, 96%, 97%, 98%, or 99% sequence identity to the amino acid sequence set forth in SEQ ID NO: 88; or
(III) the heavy chain variable region comprises an amino acid sequence set forth in SEQ ID NO: 89, or comprises an amino acid sequence having at least 95%, 96%, 97%, 98%, or 99% sequence identity to the amino acid sequence set forth in SEQ ID NO: 89; and the light chain variable region comprises an amino acid sequence set forth in SEQ ID NO: 90, or comprises an amino acid sequence having at least 95%, 96%, 97%, 98%, or 99% sequence identity to the amino acid sequence set forth in SEQ ID NO: 90; or
(IV) the heavy chain variable region comprises an amino acid sequence set forth in SEQ ID NO: 91, or comprises an amino acid sequence having at least 95%, 96%, 97%, 98%, or 99% sequence identity to the amino acid sequence set forth in SEQ ID NO: 91; and the light chain variable region comprises an amino acid sequence set forth in SEQ ID NO: 92, or comprises an amino acid sequence having at least 95%, 96%, 97%, 98%, or 99% sequence identity to the amino acid sequence set forth in SEQ ID NO: 92; or
(V) the heavy chain variable region comprises an amino acid sequence set forth in SEQ ID NO: 93, or comprises an amino acid sequence having at least 95%, 96%, 97%, 98%, or 99% sequence identity to the amino acid sequence set forth in SEQ ID NO: 93; and the light chain variable region comprises an amino acid sequence set forth in SEQ ID NO: 94, or comprises an amino acid sequence having at least 95%, 96%, 97%, 98%, or 99% sequence identity to the amino acid sequence set forth in SEQ ID NO: 94; or
(VI) the heavy chain variable region comprises an amino acid sequence set forth in SEQ ID NO: 95, or comprises an amino acid sequence having at least 95%, 96%, 97%, 98%, or 99% sequence identity to the amino acid sequence set forth in SEQ ID NO: 95; and the light chain variable region comprises an amino acid sequence set forth in SEQ ID NO: 96, or comprises an amino acid sequence having at least 95%, 96%, 97%, 98%, or 99% sequence identity to the amino acid sequence set forth in SEQ ID NO: 96; or
(VII) the heavy chain variable region comprises an amino acid sequence set forth in SEQ ID NO: 97, or comprises an amino acid sequence having at least 95%, 96%, 97%, 98%, or 99% sequence identity to the amino acid sequence set forth in SEQ ID NO: 97; and the light chain variable region comprises an amino acid sequence set forth in SEQ ID NO: 98, or comprises an amino acid sequence having at least 95%, 96%, 97%, 98%, or 99% sequence identity to the amino acid sequence set forth in SEQ ID NO: 98; or
(VIII) the heavy chain variable region comprises an amino acid sequence set forth in SEQ ID NO: 99, or comprises an amino acid sequence having at least 95%, 96%, 97%, 98%, or 99% sequence identity to the amino acid sequence set forth in SEQ ID NO: 99; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(IX) the heavy chain variable region comprises an amino acid sequence set forth in SEQ ID NO: 101 or comprises an amino acid sequence having at least 95%, 96%, 97%, 98%, or 99% sequence identity to the amino acid sequence set forth in SEQ ID NO: 101; and the light chain variable region comprises an amino acid sequence set forth in SEQ ID NO: 102, or comprises an amino acid sequence having at least 95%, 96%, 97%, 98%, or 99% sequence identity to the amino acid sequence set forth in SEQ ID NO: 102; or
(X) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 104, or comprises an amino acid sequence having at least 95%, 96%, 97%, 98%, or 99% sequence identity to the amino acid sequence set forth in SEQ ID NO: 104; or
(XI) the heavy chain variable region comprises an amino acid sequence set forth in SEQ ID NO: 105, or comprises an amino acid sequence having at least 95%, 96%, 97%, 98%, or 99% sequence identity to the amino acid sequence set forth in SEQ ID NO: 105; and the light chain variable region comprises an amino acid sequence set forth in SEQ ID NO: 106, or comprises an amino acid sequence having at least 95%, 96%, 97%, 98%, or 99% sequence identity to the amino acid sequence set forth in SEQ ID NO: 106; or
(XII) the heavy chain variable region comprises an amino acid sequence set forth in SEQ ID NO: 107 or comprises an amino acid sequence having at least 95%, 96%, 97%, 98%, or 99% sequence identity to the amino acid sequence set forth in SEQ ID NO: 107; and the light chain variable region comprises an amino acid sequence set forth in SEQ ID NO: 108, or comprises an amino acid sequence having at least 95%, 96%, 97%, 98%, or 99% sequence identity to the amino acid sequence set forth in SEQ ID NO: 108; or
(XIII) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XIV) the heavy chain variable region comprises an amino acid sequence set forth in SEQ ID NO: 111, or comprises an amino acid sequence having at least 95%, 96%, 97%, 98%, or 99% sequence identity to the amino acid sequence set forth in SEQ ID NO: 111; and the light chain variable region comprises an amino acid sequence set forth in SEQ ID NO: 112, or comprises an amino acid sequence having at least 95%, 96%, 97%, 98%, or 99% sequence identity to the amino acid sequence set forth in SEQ ID NO: 112; or
(XV) the heavy chain variable region comprises an amino acid sequence set forth in SEQ ID NO: 113 or comprises an amino acid sequence having at least 95%, 96%, 97%, 98%, or 99% sequence identity to the amino acid sequence set forth in SEQ ID NO: 113; and the light chain variable region comprises an amino acid sequence set forth in SEQ ID NO: 114, or comprises an amino acid sequence having at least 95%, 96%, 97%, 98%, or 99% sequence identity to the amino acid sequence set forth in SEQ ID NO: 114; or
(XVI) the heavy chain variable region comprises an amino acid sequence set forth in SEQ ID NO: 99, or comprises an amino acid sequence having at least 95%, 96%, 97%, 98%, or 99% sequence identity to the amino acid sequence set forth in SEQ ID NO: 99; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XVII) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 110, or comprises an amino acid sequence having at least 95%, 96%, 97%, 98%, or 99% sequence identity to the amino acid sequence set forth in SEQ ID NO: 110; or
(XVIII) the heavy chain variable region comprises an amino acid sequence set forth in SEQ ID NO: 103, or comprises an amino acid sequence having at least 95%, 96%, 97%, 98%, or 99% sequence identity to the amino acid sequence set forth in SEQ ID NO: 103; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(XIX) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 100, or comprises an amino acid sequence having at least 95%, 96%, 97%, 98%, or 99% sequence identity to the amino acid sequence set forth in SEQ ID NO: 100; or
(XX) the heavy chain variable region comprises an amino acid sequence set forth in SEQ ID NO: 109, or comprises an amino acid sequence having at least 95%, 96%, 97%, 98%, or 99% sequence identity to the amino acid sequence set forth in SEQ ID NO: 109; and the light chain variable region comprises an amino acid sequence set forth in SEQ ID NO: 104, or comprises an amino acid sequence having at least 95%, 96%, 97%, 98%, or 99% sequence identity to the amino acid sequence set forth in SEQ ID NO: 104; or
(XXI) the heavy chain variable region comprises an amino acid sequence set forth in SEQ ID NO: 142 or comprises an amino acid sequence having at least 95%, 96%, 97%, 98%, or 99% sequence identity to the amino acid sequence set forth in SEQ ID NO: 142; and the light chain variable region comprises an amino acid sequence set forth in SEQ ID NO: 92, or comprises an amino acid sequence having at least 95%, 96%, 97%, 98%, or 99% sequence identity to the amino acid sequence set forth in SEQ ID NO: 92.

3. The antibody or the antigen-binding fragment thereof according to claim 1 or 2, wherein the antibody or the antigen-binding fragment thereof comprises:
(I) a heavy chain variable region, comprising an amino acid sequence set forth in any one of SEQ ID NOs: 115, 116, 117, 118, 119, 120, 121, 124, 125, 128, 129, and 130; and a light chain variable region, comprising an amino acid sequence set forth in any one of SEQ ID NOs: 122, 123, 126, 127, 131, and 132; or
(II) a heavy chain variable region, comprising an amino acid sequence having at least 95%, 96%, 97%, 98%, or 99% sequence identity to the amino acid sequence set forth in any one of SEQ ID NOs: 115, 116, 117, 118, 119, 120, 121, 124, 125, 128, 129, and 130; and a light chain variable region, comprising an amino acid sequence having at least 95%, 96%, 97%, 98%, or 99% sequence identity to the amino acid sequence set forth in any one of SEQ ID NOs: 122, 123, 126, 127, 131, and 132; or
(III) a heavy chain variable region having an amino acid sequence set forth in SEQ ID NO: 118 and a light chain variable region having an amino acid sequence set forth in SEQ ID NO: 127; or a heavy chain variable region having an amino acid sequence set forth in SEQ ID NO: 124 or 125 and a light chain variable region having an amino acid sequence set forth in SEQ ID NO: 126; or a heavy chain variable region having an amino acid sequence set forth in SEQ ID NO: 128 and a light chain variable region having an amino acid sequence set forth in SEQ ID NO: 126, 127, 131, or 132; or
a heavy chain variable region having an amino acid sequence set forth in SEQ ID NO: 129 and a light chain variable region having an amino acid sequence set forth in SEQ ID NO: 122, 123, 127, 131, or 132; or
(IV) a heavy chain variable region having an amino acid sequence set forth in SEQ ID NO: 118 and a light chain variable region having an amino acid sequence set forth in SEQ ID NO: 127, or a heavy chain variable region having an amino acid sequence set forth in SEQ ID NO: 128 and a light chain variable region having an amino acid sequence set forth in SEQ ID NO: 127, or a heavy chain variable region having an amino acid sequence set forth in SEQ ID NO: 129 and a light chain variable region having an amino acid sequence set forth in SEQ ID NO: 131;
preferably, the antibody comprises:
(I) a heavy chain comprising an amino acid sequence set forth in any one of SEQ ID NOs: 134, 135, 137, 138, 140, and 141 and a light chain comprising an amino acid sequence set forth in any one of SEQ ID NOs: 133, 136, and 139; or
(II) a heavy chain comprising an amino acid sequence having at least 95%, 96%, 97%, 98%, or 99% sequence identity to the amino acid sequence set forth in any one of SEQ ID NOs: 134, 135, 137, 138, 140, and 141, and a light chain comprising an amino acid sequence having at least 95%, 96%, 97%, 98%, or 99% sequence identity to the amino acid sequence set forth in any one of SEQ ID NOs: 133, 136, and 139; or
(III) a heavy chain having an amino acid sequence set forth in SEQ ID NO: 134 or 135 and a light chain having an amino acid sequence set forth in SEQ ID NO: 133, or a heavy chain having an amino acid sequence set forth in SEQ ID NO: 137 or 138 and a light chain having an amino acid sequence set forth in SEQ ID NO: 136, or a heavy chain having an amino acid sequence set forth in SEQ ID NO: 140 or 141 and a light chain having an amino acid sequence set forth in SEQ ID NO: 139.

4. The antibody or the antigen-binding fragment thereof according to any one of claims 1-3, wherein the antibody is selected from a murine antibody, a chimeric antibody, a humanized antibody, and a fully human antibody, and the antigen-binding fragment is selected from Fab, Fab', F(ab')₂, Fv, scFv, and sdAb.

5. The antibody or the antigen-binding fragment thereof according to any one of claims 1-4, wherein the antibody or the antigen-binding fragment thereof is of any IgG subtype, such as IgG1, IgG2, IgG3 or IgG4, preferably IgG1 subtype or IgG4 subtype.

6. An isolated anti-CD112R antibody or an antigen-binding fragment thereof, having at least one of the following properties:
(I) binding to an epitope of human CD112R protein which is identical to, or completely or partially overlaps with an epitope to which the antibody or the antigen-binding fragment thereof according to any one of claims 1-5 binds; and
(II) competing for binding to an epitope of human CD112R protein with the antibody or the antigen-binding fragment thereof according to any one of claims 1-5.

7. A polynucleotide molecule, encoding the anti-CD112R antibody or the antigen-binding fragment thereof according to any one of claims 1-5 or the isolated antibody or the antigen-binding fragment thereof according to claim 6.

8. An expression vector, comprising the polynucleotide molecule according to claim 7, wherein preferably, the vector is a eukaryotic expression vector.

9. A host cell, comprising the polynucleotide molecule according to claim 7 or the expression vector according to claim 8, wherein preferably, the host cell is a eukaryotic cell; more preferably, the host cell is a mammalian cell.

10. A method for preparing the anti-CD112R antibody or the antigen-binding fragment thereof according to any one of claims 1-5 or the isolated antibody or the antigen-binding fragment thereof according to claim 6, comprising expressing the antibody or the antigen-binding fragment thereof in the host cell according to claim 9 under conditions suitable for the expression of the antibody or the antigen-binding fragment thereof, and isolating the expressed antibody or antigen-binding fragment thereof from the host cell.

11. A multispecific antibody, comprising a light chain variable region and a heavy chain variable region of the antibody or the antigen-binding fragment thereof according to any one of claims 1-5 or the isolated antibody or the antigen-binding fragment thereof according to claim 6.

12. A single-chain antibody, comprising a light chain variable region and a heavy chain variable region of the antibody or the antigen-binding fragment thereof according to any one of claims 1-5 or the isolated antibody or the antigen-binding fragment thereof according to claim 6.

13. An immunoconjugate, comprising the antibody or the antigen-binding fragment thereof according to any one of claims 1-5 or the isolated antibody or the antigen-binding fragment thereof according to claim 6 conjugated to a therapeutic or diagnostic agent.

14. A pharmaceutical composition, comprising the anti-CD112R antibody or the antigen-binding fragment thereof according to any one of claims 1-5, the isolated antibody or the antigen-binding fragment thereof according to claim 6, the polynucleotide molecule according to claim 7, the expression vector according to claim 8, or the host cell according to claim 9, and a pharmaceutically acceptable carrier or excipient.

15. A drug combination, comprising the antibody or the antigen-binding fragment thereof according to any one of claims 1-5, the isolated antibody or the antigen-binding fragment thereof according to claim 6, or the pharmaceutical composition according to claim 14, and one or more additional therapeutic agents; wherein preferably, the therapeutic agent is selected from one or more of an anti-PD-1 antibody, a PD-L1 antibody, and an anti-TIGIT antibody; more preferably, the therapeutic agent is an anti-TIGIT antibody, or a combination of an anti-TIGIT antibody and an anti-PD-1 antibody.

16. Use of the antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the pharmaceutical composition according to claim 14, or the drug combination according to claim 15 in preparing a medicament for treating and/or preventing a CD112R-mediated disease or disorder, wherein preferably, the disease or disorder is a cancer; more preferably, the cancer is selected from melanoma, renal cancer, prostate cancer, breast cancer, colon cancer, lung cancer, bone cancer, pancreatic cancer, skin cancer, head and neck cancer, uterine cancer, ovarian cancer, and rectal cancer.

17. A kit, comprising the antibody or the antigen-binding fragment thereof according to any one of claims 1-5, the isolated antibody or the antigen-binding fragment thereof according to claim 6, the polynucleotide molecule according to claim 7, the expression vector according to claim 8, the host cell according to claim 9, the pharmaceutical composition according to claim 14, or the drug combination according to claim 15.

18. A method for detecting the presence of CD112R in a sample by using the antibody or the antigen-binding fragment thereof according to any one of claims 1-5, a detection composition comprising the antibody or the antigen-binding fragment thereof, or the isolated antibody or the antigen-binding fragment thereof according to claim 6, comprising steps of contacting the antibody or the antigen-binding fragment thereof or the detection composition comprising the antibody or the antigen-binding fragment thereof with the sample.
